# EUROPÄISCHE PATENTANMELDUNG

(11) **EP 2 292 640 A2**
(43) Veröffentlichungstag der Anmeldung: **09.03.2011**
(21) Anmeldenummer: 10013066.5
(22) Anmeldetag: 13.09.2004
(51) Int. Cl.: C07K 5/078, C07K 5/065, A61K 38/05, A61P 9/00, A61P 7/02

(54) **Basisch-substituierte Benzylaminanaloga als Inhibitoren des Gerinnungsfaktors Xa, ihre Herstellung und Verwendung**

(30) Priorität: 11.09.2003 DE 10342108
(62) Teilanmeldung aus: 04765144.3
(71) Anmelder: The Medicines Company (Leipzig) GmbH, 04103 Leipzig (DE)
(72) Erfinder: Die Erfindernennung liegt noch nicht vor
(74) Vertreter: Bösl, Raphael Konrad

(57) **Zusammenfassung**

Neue basisch-substiutierte Benzlaminanaloga der allgemeinen Formel (I) als Inhibitoren des Gerinnungsfaktors Xa, ihre Herstellung und Verwendung zur Therapie und Prophylaxe von kardiovaskulären Erkrankungen und thromboembolischen Ereignissen.

## Beschreibung

Die Erfindung betrifft neue basisch-substituierte Benzylaminanaloga als Inhibitoren des Gerinnungsfaktors Xa, ihre Herstellung und Verwendung zur Therapie und Prophylaxe von kardiovaskulären Erkrankungen und thromboembolischen Ereignissen.

Die gegenwärtig klinisch eingesetzten Antikoagulantien vom Hepauin-Typ bzw. die Vitamin-K-Antagonisten werden nicht allen Anforderungen an ein "ideales" Antithrombotikum gerecht Deshalb wird mit kleinmolekularen Hemmstoffen der Gerinnungsenzyme, speziell von Thrombin und Faktor Xa (F Xa), nach Alternativen gesucht. Ein besonderer Vorteil von F Xa-Hemmstoffen im Vergleich zu Thrombin-Hemmstoffen könnte die geringere Blutungsneigung sein, die sich bei verschiedenen Tierversuchen gezeigt hat. So wurde bei antithrombotisch effektiven Dosen die Blutungszeit nur minimal beeinflußt (J.M. Herbert et al., J. Pharmacol. Exp. Ther. 276, 1030-1038, 1996; K. Sato et al., Br. J. Pharmacol. 123, 92-96, 1998).

Die ersten nichtpeptidischen Verbindungen mit hoher Affinität für F Xa waren symmetrische Bis-benzamidine (Kᵢ = 13 nM für die wirksamste Verbindung BABCH) (J. Stürzebecher et al., Thromb. Res. 54, 245-252, 1998). Auch das Naphthamidin-Derivat DX-9065a besitzt zwei basische Gruppen und hemmt F Xa selektiv mit einem Kᵢ = 24 nM (T. Hara et al., Thromb. Haemost. 71, 314-319, 1994). Der mit DX-9065a strukturell verwandte Inhibitor YM-60828 (K. Sato et al. Eur. J. Pharmacol. 339, 141-146, 1997) ist noch wirksamer (Kᵢ = 1.3 nM). Inzwischen wurde eine ganze Reihe weiterer bis-basischer Verbindungen beschrieben, bei denen z. B. zwei Benzamidin-Reste über einen Oxazolin-Ring (Kᵢ = 18 nM) (M.L. Quan et al., Bioorg. Med. Chem. Lett. 7, 2813-2818, 1997) bzw. eine Carboxymethylalkyl-Kette (Kᵢ = 34 nM) verknüpft sind (T.P. Maduskuie et al., J. Med. Chem. 41, 53-62, 1998). Nachteil der bis-basischen Verbindungen ist insbesondere die geringe Bioverfügbarkeit nach oraler Gabe.

Auch Hemmstoffe für F Xa, die nur eine basische Gruppe enthalten, wurden beschrieben. N-substituierte Amidino-phenoxypyridine (Kᵢ = 0,11 nM für BX-807834) wurden auf der Basis von BABCH entwickelt (R. Mohan et al., Bioorg. Med. Chem. Lett. 8, 1877-1882, 1998; G.B. Phillips et al. J. Med. Chem. 41, 3557-3562, 1998). Amide des Nα-Adamantyloxycarbonyl-3-amidinophenylalanins (Kᵢ = 74 nM für die wirksamste Verbindung) sind selektive Hemmstoffe des F Xa (S. Sperl et al., Biol. Chem. 381, 321-329, 2000), während Nα-arylsulfonyl-aminoacylierte Ester des 3-Amidinophenylalanins eine geringe Hemmwirkung (Kᵢ = 840 nM für TAPAM) besitzen (J. Stürzebecher et al., Thromb. Res. 54, 245-252, 1998). Die WO 96/10022 offenbart Hemmstoffe, die überhaupt keine starke Ladung mehr besitzen (Kᵢ = 3,0 nM für die wirksamste Verbindung). Eine weitere Serie von wirksamen Faktor Xa-Hemmstoffen ohne basische Substituenten wurden kürzlich von Choi-Sledeski et al. (J. Med. Chem. 46, 681-684, 2003) beschrieben.

Bisher wurden nur wenige Peptide als Hemmstoffe für F Xa beschrieben, die sich von der Substrat-Sequenz Ile-Glu-Gly-Arg ableiten. Die von Kettner und Shaw (Thromb. Res. 22, 645-652, 1981) beschriebenen Chlormethylketone hemmen F Xa irreversibel und sind nicht für in vivo-Anwendungen geeignet. Dagegen sind die Peptide SEL 2489 (Kᵢ = 25 nM) und SEL 2711 (Kᵢ = 3 nM) außerordentlich wirksam (J. A. Ostrem et al., Biochemistry 37, 1053-1059, 1998). Auch einige Peptidyl-Arginin-Aldehyde und Peptidyl-Arginyl-Ketone wurden beschrieben, die neben Argininal oder einem Arginyl-Ketonderivat, wie z.B. Arginyl-Ketothiazol in P3-Position ein D-Arginin bzw. eine unnatürliche basische Aminosäure, wie z.B. 4-Amidinophenylalanin, 3- oder 4-Amidinopiperidinylalanin und 4-Guanidinophenylalanin in P3 besitzen (Z. H. Jonathan, Bioorg. Med. Lett. 9, 3459-3464, 1999 und Übersichtsarbeit: Zhu und Scarborough Current Opinion in Cardiovascular, Pulmonary & Renal Investigational Drugs 1999, 1, 63-88).) In der Anmeldung WO 01/96366 sind Hemmstoffe offenbart, die sich von acyliertem Amidinobenzylamin ableiten und neben einer natürlichen Aminosäure in P2 einen D-Ser-Ether oder ein vergleichbares Derivat einer unnatürlichen Aminosäure enthalten. Verbindungen diese Typs hemmen sowohl F Xa (Kᵢ = 30 nM für die wirksamste Verbindung) als auch die Gerinnung von menschlichem Blutplasma sehr wirksam. Allerdings haben Verbindungen diese Typs nur unzureichende pharmakokinetische Eigenschaften für eine Anwendung in vivo; sie werden nach oraler Gabe kaum resorbiert und im Versuchstier nach i.v.-Gabe sehr schnell aus der Zirkulation eliminiert.

In dem U.S. Patent Nr. US 5,914,319 wurden Thrombinhemmstoffe beschrieben, die in P3-Position ein d-Homophenylalanin oder d-Homocyclohexylalanin besitzen und auch eine schwache Faktor Xa-Inhibierung mit Hemmkonsten im mikromaolaren Bereich zeigen (für Faktor Xa: Kₐₛₛ < 5,5 x 10⁶ l/mol, entspricht ca. Kᵢ > 0,18 µM). Diese Inhibitoren besitzen jedoch zwingend eine Iminosäure in P2-Position, also Analoga des Prolins oder N(Alkyl)Glycin-Derivate. Auch ist die Thrombinaffinität deutlich erhöht und das. Selektivitätsverhältnis (Kᵢ für Thrombin/Kᵢ für F Xa) ist für die angegebenen Verbindungen < 0,08.

Der Erfindung liegt daher die Aufgabe zu Grunde, einen für therapeutische Anwendungen geeigneten Wirkstoff anzugeben, der den Gerinnungsfaktor Xa mit hoher Aktivität und Spezifität hemmt und der vorzugsweise nach i.v.-, s.c.- oder oraler Gabe möglichst lange im Körper zirkuliert.

Überraschend wurde gefunden, dass acyliertes Amidinobenzylamin gemäß der im Patentanspruch 1 angeführten allgemeinen Formel I, 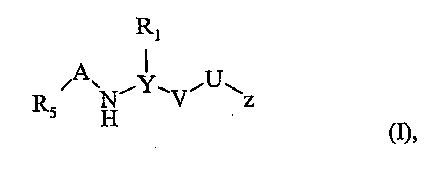 wobei
A p₂-P₁ ist, mit 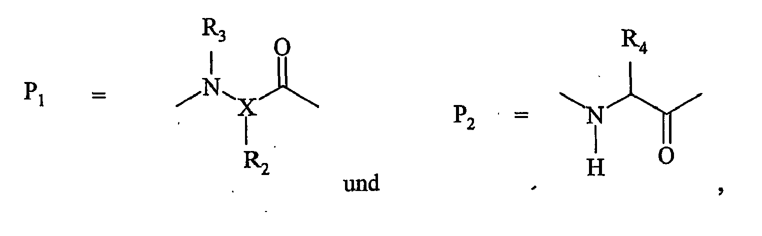 insbesondere Verbindungen des 4-Amidinobenzylamins, sowohl Faktor Xa sehr wirksam inaktivieren als auch langsam aus der Zirkulation eliminiert werden, wenn neben der Amidinofunktion weitere geladene oder polare Gruppen eingeführt werden, wobei es sich vor allem herausgestellt hat, dass D-Homophenylalanin, D-Homotyrosin bzw. D-Homo-4-pyridylalanin und dessen Derivate an der Position P₂ gemäß der allgemeinen Formel I besonders wirksam sind. Durch die Verwendung von ausgewählten α-Aminosäuren in P2-Position konnte auch die Selektivität als Faktor Xa-Hemmstoffe entscheidend erhöht werden; was besonders überraschend war.

Zur Klarstellung wird darauf hingewiesen, dass die Benennung der Reste P₂ und P₁ in dem Struktursegment A der allgemeinen Formel I sich nicht auf die sonst üblicherweise verwendete Nomenklatur der Aminosäurereste in Peptidsubstraten von Serinproteasen und davon abgeleiteten Inhibitoren bezieht, wie sie von Schechter und Berger eingeführt wurde (Schechter und Berger, Biochem. Biophys. Res. Comm. 27, 157-162 (1967)). In allen Teilen der Erfindung, d.h. sowohl in der Beschreibung als auch in den Ansprüchen gelten die folgenden Definitionen:

Der Buchstabe P in Zusammenhang mit einer Zahl von 1 bis 3 in normaler Schrift, d.h. P1, P2 oder P3, wird für Aminosäurereste und deren Derivate entsprechend der Nomenklatur von Schechter und Berger verwendet. Dagegen steht der Buchstabe P in Zusammenhang mit einer tiefgestellten 1 oder 2, d.h. P₁ oder P₂, für Aminosäurereste und deren Derivate als Bestandteile der Struktur A in Formel I der vorliegenden Erfindung. Dabei entspricht substituierte oder unsubstituierte natürliche oder unatürliche Aminosäure P₁ in der Struktur A P2 nach Schechter und Berger und die in der D-Konfiguration vorliegende substituierte oder unsubstituierte natürliche oder unatürliche Aminosäure P₂ in der Struktur A entspricht P3 nach Schechter und Berger.

Ein Gegenstand der vorliegenden Erfindung ist daher eine Verbindung der allgemeinen Formel I 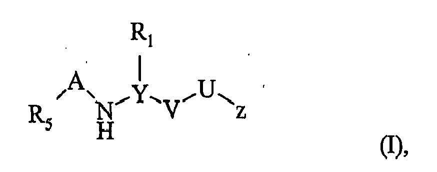 wobei
A p₂-P₁ ist, mit 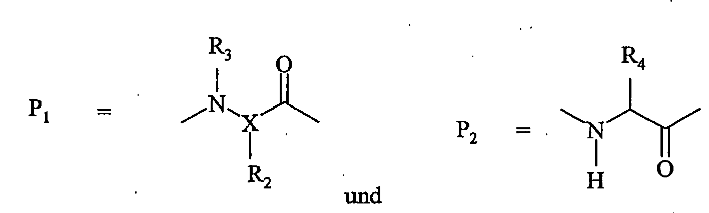 ist;
R₁ ein H oder -(CH₂)ₐCOOR₆ mit a = 0, 1, 2, 3, 4 oder 5, vorzugsweise mit a= 0, 1 oder 2, ist, wobei R₆ ein verzweigter oder unverzweigter Alkylrest mit vorzugsweise 1 bis 6 C-Atomen, insbesondere 1 bis 3 C-Atomen, vor allem Ethyl, insbesondere ist R₁ ein H;
R₂ ein H, -CH₂-OR₇ oder -CH₂-OCOOR₇ ist, wobei R₇ ein H oder ein verzweigter oder unverzweigter Alkylrest mit 1-5, insbesondere 1-3 C-Atomen, ist, oder R₂ ist ein -CH₂-CH₂-COOR_{7*}, wobei R_{7*} ein H oder ein verzweigter oder unverzweigter Alkylrest mit 1-5 C-Atomen, vorzugsweise Ethyl, ist;
R₃ ein H ist;
R₄ -(CH₂)_{f}-R₈ mit f = 0 oder 2, vorzugsweise mit f = 2, -CH₂NHR₈, -(CH₂)₂NHR₈ oder -CH=CH-R₈ ist, wobei R₈ ein einfach oder mehrfach substituierter oder unsubstituierter Cycloalkyl-, Aryl- oder Heteroarylrest ist, wobei der Cycloalkyl-, Aryl- oder Heteroarylrest vorzugsweise 5 bis 14, insbesondere 5 bis 6 C-Atome im Ring und im Falle des Heteroarylrestes vorzugsweise 1 bis 3 N als Heteroatome besitzt, oder wenn R₄ gleich -(CH₂)_{f}-R₈ mit R₈ gleich ein Hydroxycycloalkylrest mit 4 bis 14, insbesondere 6 bis 10, vor allem 6 C-Atomen ist, dann ist f = 1, und wobei P₂ in der Struktur A der allgemeinen Formel I in der D- oder L-Konfiguration, vorzugsweise in der D-Konfiguration vorliegt;
R₅ -(CH₂)ᵢ-COOR₉ mit i = 1, 2 oder 3, vorzugsweise mit i = 1, und R₉ gleich ein verzweigter oder unverzweigter Alkylrest mit 1-5 C-Atomen, vorzugsweise Ethyl, ist, oder R₅ ist -SO₂R_{9*}, -SO₂-NE-R_{9*}, wobei R_{9*} ein H, ein verzweigtes oder unverzweigtes Alkyl mit 1-10, vorzugsweise 1 bis 6, insbesondere 1 bis 4, vor allem 1 bis 2 C-Atomen, ein einfach oder mehrfach substituierter oder unsubstituierter Aryl-, Heteroaryl-, Aralkyl-, vorzugsweise Benzyl-, Heteroaralkylrest oder ein Cyclohexylalkylrest, vorzugsweise ein Cyclohexylmethylrest ist, wobei der Substituent eine -OH, -O-COOR₇, -CH₂-OCOOR₇, mit R₇ wie oben definiert, -NH₂, NO₂, -COOR₁₀, -CH₂-COOR₁₀-Gruppe oder ein Cl-, F- oder Br-Atom sein kann und wobei R₁₀ ein H oder ein Alkylrest mit 1 bis 6, insbesondere mit 1 bis 4 C-Atomen, vor allem Ethyl, ist;
U ein Phenyl- oder Cyclohexylrest ist; ein aromatischer oder nichtaromatischer heterocyclischer Rest mit 1-10, vorzugsweise 6 Ringatomen mit mindestens einem N, S oder O als Heteroatom, insbesondere Pyridin, Piperidin oder Pyrimidin, ist, oder ein Thienylrest ist;
V (CH₂)ₙ mit n = 0 oder 1, vorzugsweise 0, ist;
X N oder CH, vorzugsweise CH, ist;
Y N oder CH, vorzugsweise CH, ist;
Z in 2, 3- oder 4-Position, vorzugsweise in 4-Position, vorkommt und eine Aminomethyl-, eine Guanidinofunktion oder eine Amidinogruppe 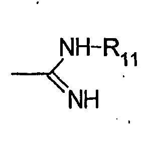 ist, wobei R₁₁ H, OH, NH₂, -COR₁₂ oder -COOR₁₂ ist, wobei R₁₂ ein verzweigter oder unverzweigter Alkylrest mit 1 bis 8, vorzugsweise 1 bis 6 C-Atomen oder ein einfach oder mehrfach substituierter oder unsubstituierter Aryl- oder Heteroaryl-, Aralkyl- oder Heteroaralkylrest ist, wobei der Alkylrest vorzugsweise 1 bis 16, insbesondere 1 bis 8, vor allem 1 bis 4 und besonders bevorzugt 1 bis 2 C-Atome und der Aryl- oder Heteroarylrest vorzugsweise 4 bis 14, insbesondere 6 bis 10, vor allem 6 C-Atome und vorzugsweise 1 bis 3 N als Heteroatome besitzt;
oder eine Verbindung der allgemeinen Formel I in Form eines Prodrugs oder in Form ihres Salzes.

Weitere besonders geeignete Verbindungen sind Verbindungen nach der allgemeinen Formel I, wobei U an 1, 2 oder 3 Positionen vorzugsweise mit einem Halogen, insbesondere Fluor oder Chlor, oder einem Methyl-, Ethyl-, Propyl-, Methoxy-, Ethoxy-, oder Propoxyrest substituiert ist.

Ebenso besonders geeignete Verbindungen sind Verbindungen nach der allgemeinen Formel I, wobei mindestens eine Carboxylgruppe als Ester, bevorzugt als Ethylester, geschützt vorliegt, die im Sinne eines Prodrugs erst nach Aufnahme im Körper in eine Carboxylgruppe umgewandelt wird.

Ganz allgemein ist ein Prodrug ein pharmazeutisch inaktives Derivat der entsprechenden pharmazeutisch wirksamen Substanz, das nach oraler Gabe spontan oder enzymatisch unter Freisetzung der pharmazeutisch wirksamen Substanz biotransformiert wird.

Folglich versteht man als Prodrug beispielsweise Verbindungen der allgemeinen Formel I, bei denen zusätzlich oder ausschließlich ein oder mehrere Carboxylgruppen in Form ihrer Alkylester mit einem verzweigten oder unverzweigten Alkyl mit 1-5 C-Atomen, vorzugsweise Ethyl, und/oder bei denen ein oder mehrere Hydroxylgruppen als Carbonate, bei denen der endständige Rest gleich R₇, wie oben definiert, vorliegen können. Ein Prodrug im Sinne der vorliegenden Erfindung ist beispielsweise auch ein Amidino- oder Guanidinobenzylaminderivat gemäß der allgemeinen Formel I, bei dem der Amidino- bzw. Guanidinobenzylaminrest als Hydroxyamidin bzw. Hydroxyguanidin oder als Alkyloxycarbonylderivat mit vorzugsweise einem verzweigten oder unverzweigten Alkylrest mit 1-5 C-Atomen, vorzugsweise Ethyl, vorliegt.

Weitere besonders geeignete Verbindung sind Verbindungen, bei denen das Strukturelement 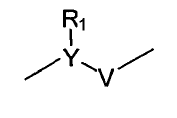 der Formel I eine -CH₂- oder -NH-Gruppe, vorzugsweise eine -CH₂-Gruppe bedeutet.

Besonders bevorzugt sind auch Verbindungen, bei denen
R₁ ein H ist;
R₂ ein H, -CH₂-CH₂-COOH, -CH₂-CH₂-COOCH₂CH₃ oder -CH₂OH ist;
R₃ ein H ist;
R₄ ein -(CH₂)₂-R₈, -CH₂NHR₈, -(CH₂)₂NER₈ oder ein -CH₂-4-Hydroxycyclohexyl-Rest ist, wobei R₈ ein einfach oder mehrfach substituierter oder unsubstituierter Cycloalkyl-, Aryl- oder Heteroarylrest ist, wobei der Cycloalkyl-, Aryl- oder Heteroarylrest 5 oder 6 C-Atome und im Falle eines Heteroarylrestes 1 oder 2 N als Heteroatome besitzt, vorzugsweise ist R₈ ein Phenyl-, Hydroxyphenyl-, Pyridyl- oder Aminopyridylrest;
R₅ ein Methylsulfonyl, Ethylsulfonyl, n-Propylsulfonyl, iso-Propylsulfonyl, Butylsulfonyl, Benzylsulfonyl, n-Butylsulfonyl, Aminobenzylsulfonyl, Hydroxybenzylsulfonyl, Chlorobenzylsulfonyl, Fluorobenzylsulfonyl, Carboxybenzylsulfonyl, Ethyloxycarbonylbenzylsulfonyl, Carboxymethylbenzylsulfonyl, Ethyloxycarbonylmethylbenzylsulfonyl, Pyridylmethylsulfonyl, N-(oxid)-pyridylmethylsulfonyl, -CH₂-COOH oder ein -CH₂COOCH₂CH₃ Rest ist;
U ein Phenylrest ist;
V (CH₂)ₙ mit n = 0 ist;
X CH ist;
Y CH ist;
Z in 4-Position vorkommt und eine Amidinogruppe 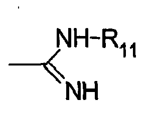
ist,
wobei R₁₁ H, OH oder -COOR₁₂ mit R₁₂ ein verzweigter oder unverzweigter Alkylrest mit 2, 4 oder 6 C-Atomen ist.

Andere besonders geeignete Verbindungen sind Verbindungen, bei denen R₄ ein -CH₂-CH₂-R₈ Rest ist, wobei R₈ ein Aryl- oder Heteroarylrest mit 4-6 Ringatomen ist, der 1 oder 2 Heteroatome, vorzugsweise N, besitzt und der mit einer oder mehreren -NH₂ und/oder - OH Gruppen substituiert sein kann, vorzugsweise ist P₂ in der Struktur A der allgemeinen Formel I von einem Homophenylalanin, Homotyrosin, Indanylglycin oder 4-Pyridylhomoalanin abgeleitet, und die P₂- Aminosäure liegt insbesondere in der D-Konfiguration vor.

Sofern nichts anderes definiert ist, bedeutet der Begriff "Substituent" bzw. "substituiert" gemäß der vorliegenden Erfindung vorzugsweise -OH, -NH₂, -NO₂, -COOH, - COOCH₂CH₃ oder ein Halogen, wobei der Begriff "Halogen" im allgemeinen Fluor, Chlor oder Brom, insbesondere Fluor oder Chlor, bedeutet.

Als Alkylrest wird im allgemeinen, sofern nicht anders definiert, ein Rest mit vorzugsweise 1-5 C-Atomen, insbesondere Ethyl, und als Cycloalkyl-, Aryl-, Aralkylrest wird im allgemeinen, sofern nicht anders definiert, ein Rest mit vorzugsweise 4 bis 14, insbesondere 6 bis 10, vor allem 6 C-Atomen als Ringatome bezeichnet. Der Begriff "Hetero" bedeutet im allgemeinen, sofern nicht anders definiert, vorzugsweise N, S oder O, insbesondere N, wobei bei einem Heteroarylrest mindestens ein C-Atom des Ringes durch ein Heteroatom ersetzt ist, vorzugsweise sind 1, 2 oder 3 C-Atome des Ringes insbesondere durch N ersetzt.

Im Detail sind besonders bevorzugte Verbindungen gemäß der vorliegenden Erfindung Verbindungen gemäß den Ansprüchen bzw. Verbindungen 11 bis 20 und 22 bis 65 der Tabelle 1.

Unter den einzelnen besonders bevorzugten Verbindungen gehören jedoch auch Verbindungen, bei denen in den genannten Strukturen der Glycinrest mit dem Strukturelement 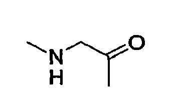 jeweils ersetzt ist durch einen Serinrest mit dem Strukturelement 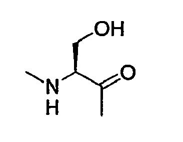 oder durch einen Glutaminsäurerest mit dem Strukturelement 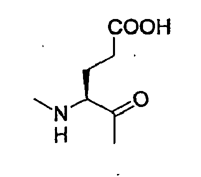 oder durch einen Glutamin-γ-ethylester mit dem Strukturelement 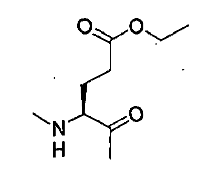 Beispielsweise sind dies folgende Strukturen mit einem Serinrest:

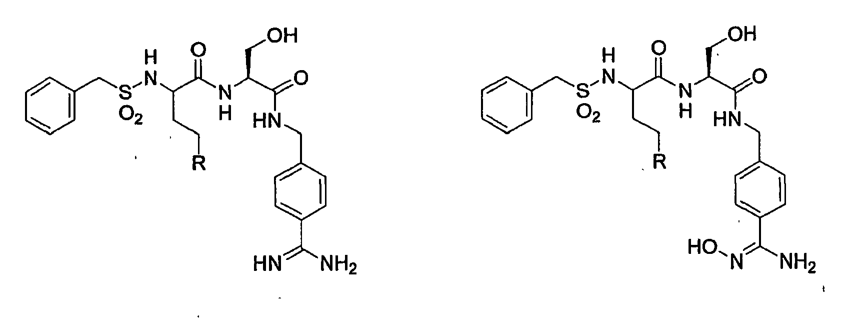

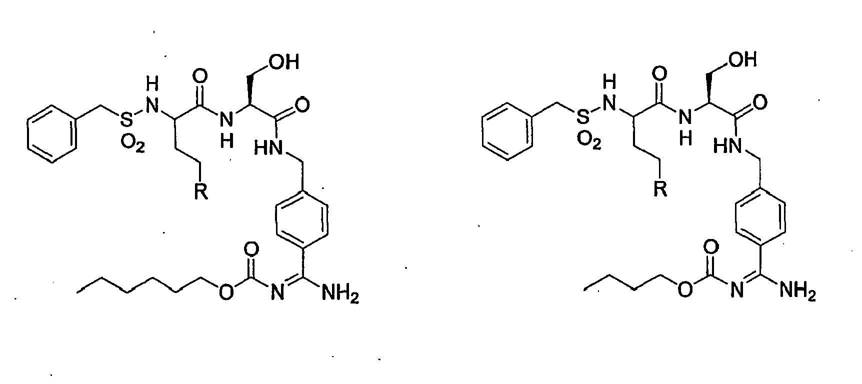

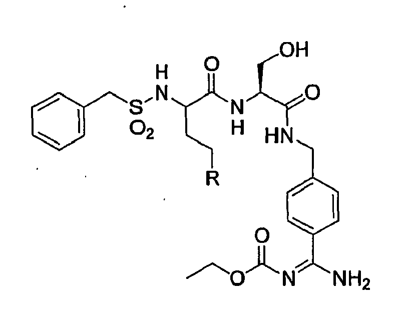

Weitere geeignete Verbindungen der vorliegenden Erfindung:

Ein weiterer Gegenstand der vorliegenden Erfindung sind Verbindungen wie vorstehend genannt, wobei Z eine Aminogruppe ist. Vorzugsweise ist in solchen Verbindungen U ein Phenylrest, ein Cyclohexylrest oder ein N-Atom-Heteroarylrest, vorzugsweise ein Pyridylrest.

Ein weiterer Gegenstand der vorliegenden Erfindung sind Verbindungen wie vorstehend genannt mit der Ausnahme, dass R₂-(CH₂)ₐCONHR_{7*} oder -(CH₂)ₐCONHR_{7**} mit a = 1, 2, oder 3 ist, wobei R_{7**} ein Arylrest, vorzugsweise ein Phenylrest oder ein Aralkyl, vorzugsweise ein Benzylrest ist oder ein Heteroarylrest mit ein bis zwei N-, S- oder O-Heteroatomen, vorzugsweise N-Heteroatomen ist.

Ein weiterer Gegenstand der vorliegenden Erfindung sind Verbindungen wie vorstehend genannt, wobei R₂ -(CH₂)ₐCONHR_{7*} oder -(CH₂)ₐCONHR_{7**} mit a = 1, 2, oder 3 ist und wobei R_{7**} substituiert ist mit mindestens einem Halogen, einer Methyl-, einer Ethyl-, einer Amino-, einer Hydroxy-, einer Nitro-, einer -COOH, einer -CH₂COOH, oder einer - CH₂NH₂- Gruppe.

Ein weiterer Gegenstand der vorliegenden Erfindung sind Verbindungen wie vorstehend genannt, wobei R₂ ein -(CH₂)ₙ-NH₂ ist, mit n= 1, 2, 3, 4 oder 5, vorzugsweise 1 oder 4, ist.

Ein weiterer Gegenstand der vorliegenden Erfindung sind Verbindungen wie vorstehend genannt, wobei R₅ R₉ = H ist.

Ein weiterer Gegenstand der vorliegenden Erfindung sind Verbindungen wie vorstehend genannt, wobei R₄ eine -CH₂-SR₈ oder -CH₂CH₂-SR₈ Gruppe ist. Beispiele derartiger Verbindungen sind insbesondere solche, in denen R₅ eine -SO₂R_{9*} oder eine -SO₂CH₂R_{9*} Gruppe ist oder in denen R₅ eine -SO₂R_{9*} oder eine -SO₂CH₂R_{9*} Gruppe ist und v = (CH₂)ₙ mit n = 0 ist oder in denen R₅ eine -SO₂R_{9*} oder eine -SO₂CH₂R_{9*} Gruppe und v = (CH₂)ₙ mit n = 0 und mit U = ein Phenylrest, ein Cyclohexylrest, ein N-Heteroarylvorzugsweise Pyridylrest ist.

Ebenso sind Verbindungen Gegenstand der Erfindung, in denen R₅ eine -SO₂R_{9*} oder eine -SO₂CH₂R_{9*} Gruppe oder in denen R₅ eine -SO₂R_{9*} oder eine -SO₂CH₂R_{9*} Gruppe und v = (CH₂)ₙ mit n = 0 ist oder in denen R₅ eine -SO₂R_{9*} oder eine -SO₂CH₂R_{9*} Gruppe und v = (CH₂)ₙ mit n = 0 und U = ein Phenylrest, ein Cyclohexylrest, ein N-Heteroarylvorzugsweise ein Pyridylrest ist und in denen R_{9*} ein Phenylrest, ein Cyclohexylrest, ein Pyridylrest oder ein Pyridyl-N-oxidrest ist.

Ebenso sind Verbindungen Gegenstand der Erfindung in denen R₅ eine -SO₂R_{9*} oder eine -SO₂CH₂R₉- Gruppe ist oder in denen R₅ eine -SO₂R_{9*} oder -SO₂CH₂R_{9*} Gruppe und v = (CH₂)ₙ mit n = 0 ist oder in denen R₅ eine -SO₂R_{9*} oder -SO₂CH₂R_{9*} Gruppe und v = (CH₂)ₙ mit n = 0 und U = ein Phenylrest, ein Cyclohexylrest, N-Heteroaryl- vorzugsweise ein Pyridylrest ist und in denen R_{9*} ein substituierter Phenyl- oder Cyclohexyl- oder Pyridyl- oder Pyridyl-N-oxidrest ist, wobei der Substituent eine -OH, -O-COOR₇, - CH₂OCOOR₇, mit R₇ wie oben definiert, NH₂-, NO₂-, -COOR₁₀, -CH₂COOR₁₀- Gruppe oder ein Cl- oder F- oder Br-Atom sein kann.

Ebenso sind Verbindungen Gegenstand der Erfindung in denen R₅ eine -SO₂R_{9*} oder -SO₂CH₂R_{9*} Gruppe ist oder in denen R₅ eine -SO₂R_{9*} oder -SO₂CH₂R_{9*} Gruppe und v = (CH₂)ₙ mit n = 0 ist oder in denen R₅ eine -SO₂R_{9*} oder -SO₂CH₂R_{9*} Gruppe und v = (CH₂)ₙ mit n = 0 und U ein Phenlyrest, ein Cyclohexylrest oder ein N-Heteroaraylvorzugsweise ein Pyridylrest ist und in denen R_{9*} ein substituierter Phenyl- oder Cyclohexyl- oder Pyridyl- oder Pyridyl-N-oxidrest ist, wobei der Substituent eine -OH, - O-COOR₇, -CH₂OCOOR₇, mit R₇ wie oben definiert, NH₂-, NO₂-, -COOR₁₀, - CH₂COOR₁₀- Gruppe oder ein Cl- oder F- oder Br-Atom sein kann und in denen R₁ - (CH₂)ₐCONHR₆, oder -(CH₂)ₐCONHR_{6*} mit a = 0, 1, 2, 3, 4 oder 5, vorzugsweise 0, 1 oder 2 ist, wobei R_{6*} ein Arylrest, vorzugsweise ein Phenylrest ist.

Ebenso sind Verbindungen Gegenstand der Erfindung in denen R₅ eine -SO₂R_{9*} oder eine -SO₂CH₂R_{*} Gruppe ist oder in denen R₅ eine -SO₂R_{9*} oder -SO₂CH₂R_{9*} Gruppe und v = (CH₂)ₙ mit n = 0 ist oder in denen R₅ eine -SO₂R_{9*} oder eine -SO₂CH₂R_{9*} Gruppe und v = (CH₂)ₙ mit n = 0 und U ein Phenlyrest, ein Cyclohexylrest oder ein N-Heteroarylvorzugsweise ein Pyridylrest ist und in denen R_{9*} ein substituierter Phenyl- oder Cyclohexyl- oder Pyridyl- oder Pyridyl-N-oxidrest ist, wobei der Substituent eine -OH, - O-COOR₇, -CH₂OCOOR₇, mit R₇ wie oben definiert, eine NH₂-, NO₂-, -COOR₁₀, - CH₂COOR₁₀- Gruppe oder ein Cl- oder F- oder Br-Atom sein kann und in denen R₂ -CH₂-CH₂-CONHR_{7*} oder -CH₂CH₂CONHR_{7**} oder -CH₂CH₂COOR_{7**} ist wobei R_{7**} ein Arylrest, - vorzugsweise ein Benzyl- oder Phenylrest ist.

Ebenso sind Verbindungen Gegenstand der Erfindung in denen R₅ eine -SO₂R_{9*} oder eine -SO₂CH₂R_{9*} Gruppe ist oder in denen R₅ eine -SO₂R_{9*} oder eine -SO₂CH₂R_{9*} Gruppe und v = (CH₂)ₙ mit n = 0 oder in denen R5 eine -SO₂R_{9*} oder -SO₂CH₂R_{9*} Gruppe und v = (CH₂)ₙ mit n = 0 und U = ein Phenlyrest, ein Cyclohexylrest oder ein Pyridylrest ist und in denen R_{9*} ein substituierter Phenyl- oder Cyclohexyl- oder Pyridylrest ist, wobei der Substituent eine -OH, -O-CO0R₇, -CH₂OCOOR₇, mit R₇ wie oben definiert, eine NH₂-, NO₂-, -COOR₁₀, -CH₂COOR₁₀- Gruppe oder ein Cl- oder F- oder Br-Atom sein kann und in denen R₄ eine -CH₂-SR₈ oder -CH₂CH₂SR₈ -Gruppe ist.

Ebenso sind Verbindungen Gegenstand der Erfindung wie vorstehend genannt, wobei R₈ ein Pyridyl-N-oxid- Rest ist.

Außerdem sind Verbindungen Gegenstand der Erfindung, in denen P₁ ein Prolylrest oder ein Azetidincarbonsäurerest ist.

Ebenso sind Verbindungen Gegenstand der Erfindung in denen P₂ ein 4-N-oxid-Pyridylhomoalaninrest ist. Auch sind Verbindungen Gegenstand der Erfindung in denen P₂ ein Lysyl- oder ein a,β-Diaminopropionsäurerest ist.

Ebenso sind die Verbindungen eine oder mehr oder alle der Verbindungen 22 bis 65 der Tabelle 1 Gegenstand der Erfindung.

Neben der Inaktivierung von Faktor Xa werden die zusätzlich geladenen 4-Amidinobenzylamin-Derivate gemäß der vorliegenden Erfindung wie bereits erwähnt auf vorteilhafte und überraschende Weise sehr langsam eliminiert, so dass die erfindungsgemäßen Verbindungen eine neue Gruppe von hochaktiven F Xa-Hemmstoffen darstellen.

Die Verbindungen liegen in der Regel als Salze, vorzugsweise mit Mineralsäuren oder geeigneten organischen Säuren, vorzugsweise mit Salzsäure, Schwefelsäure, Essigsäure, Ameisensäure, Methylsulfonsäure, Bernsteinsäure, Äpfelsäure oder Trifluoressigsäure, vor allem in Form ihrer Hydrochloride, Sulfate oder Acetate.

Die Verbindungen der allgemeinen Formel I können in prinzipiell bekannter Weise, wie nachfolgend beschrieben, beispielsweise wie folgt hergestellt werden, wobei im allgemeinen sequentiell die entsprechenden Aminosäuren an ein an der Amidinogruppe geschütztes Amidinobenzylamin angekoppelt werden, wobei die N-terminale Aminosäure entweder den R₅-Rest bereits trägt oder dieser anschließend daran gebunden wird:

Aus dem kommerziell erhältlichen 4-Cyanobenzylamin (Showa Denko, Japan) wird das Boc-geschützte 4-Acetyloxamidinobenzylamin nach dem Fachmann bekannten Verfahren gewonnen. Nach Abspaltung der Boc-Schutzgruppe erfolgt die Ankopplung der weiteren Aminosäuren und der Schutzgruppe R₅ mittels Standardkopplungsmethoden mit Boc als N-terminaler Schutzgruppe. Die zweite Aminosäure kann auch direkt als N-aryl- bzw. N-aralkyl-sulfonyl-geschützte Aminosäure gekoppelt werden. Die Peptidanaloga werden sequentiell, beginnend vom Acetyloxamidinobenzylamin, aufgebaut. Die meisten Zwischenprodukte kristallisieren gut und lassen sich damit einfach reinigen. Die Endreinigung der Hemmstoffe erfolgt auf der letzten Stufe vorzugsweise über präparative, reversed-phase HPLC.

Ein weiterer Gegenstand der Erfindung ist daher ein Verfahren zur Herstellung einer Verbindung nach der allgemeinen Formel I, wobei sequentiell die entsprechenden Aminosäuren an ein an der Amidinogruppe geschütztes Amidinobenzylamin, beispielsweise an ein 4-Acetyloxamidinobenzylamin oder an ein 4-(Benzyloxycarbonylamidino)benzylamin angekoppelt werden, wobei die N-terminale Aminosäure entweder den R₅-Rest bereits trägt oder dieser anschließend daran gebunden wird.

Ein anderer Gegenstand der Erfindung ist auch ein Arzneimittel enthaltend eine erfindungsgemäße Verbindung sowie weitere pharmazeutisch geeignete Hilfs- und/oder Zusatzstoffe. Geeignete Hilfs- und/oder Zusatzstoffe, die z.B. der Stabilisierung und/oder Konservierung des Arzneimittels dienen, sind dem Fachmann allgemein geläufig (z.B. Sucker H. et al., (1991) Pharmazeutische Technologie, 2. Auflage, Georg Thieme Verlag, Stuttgart). Hierzu zählen beispielsweise physiologische Kochsalzlösungen, Ringer-Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Das Arzneimittel könnte beispielsweise in parenteraler Anwendungsform, insbesondere in intraartieller, intravenöser, intramuskulärer oder subcutaner Form, in enteraler Anwendungsform, insbesondere zur oralen oder rektalen Anwendung, oder in topischer Anwendungsform, insbesondere als Dermatikum, angewendet werden. Bevorzugt sind intravenöse oder subkutane Anwendungen.

In einer Ausführungsform der Erfindung wird das Arzneimittel beispielsweise in Form einer Tablette, eines Dragées, einer Kapsel, eines Pellets, Suppositoriums, einer Lösung, insbesondere einer Injektions- oder Infusionslösung, von Augen-, Nasen und Ohrentropfen, eines Safts, einer Kapsel, einer Emulsion oder Suspension, eines Globuli, Styli, Aerosols, Puders, einer Paste, Creme oder Salbe eingesetzt.

Die erfindungsgemäßen Hemmstoffe von Faktor Xa oder die genannten Arzneimittel werden bevorzugt zur Therapie oder Prophylaxe einer kardiovaskulären Erkrankung oder eines thromboembolischen Ereignisses, insbesondere in oraler, subkutaner, intravenöser oder transdermaler Form verwendet.

Die Erfindung soll nachstehend anhand von mehreren Ausführungsbeispielen näher erläutert werden, ohne sie darauf zu beschränken.

### Methoden

Analytische HPLC: Shimadzu LC-10A System, Säule: Phenomenex-Luna C₁₈, 5 µm (250 x 4 mm) Lösungsmittel A: 0,1 % TFA in Wasser, B: 0,1 % TFA in ACN, Gradient: 10 % B bis 70 % B in 60 min, 1 ml/min Fluß, Detektion bei 220 oder 215 nm.

Präparative HPLC: Shimadzu LC-8A System, Säule: Phenomenex-Luna C₁₈, 5 µm (250 x 30 mm) Lösungsmittel A: 0,1 % TFA in Wasser, B: 0,1 % TFA in ACN, Gradient: 5 % B bis 50 % B in 120 min, 10 ml/min Fluß, Detektion bei 220 nm.

Massenspektroskopie: Die Massenspektren wurden auf einem ESI-MS LCQ der Firma Finnigan (Bremen, Deutschland), gemessen.

**Verwendete Abkürzungen**

| Ac | Acetyl |
|---|---|
| AcOxam | N-(Acetyloxy)amidin |
| Amb | Amidomethylbenzen |
| 4-Amba | 4-Amidinobenzylamid |
| Boc | tert.-Butyloxycarbonyl |
| Bzl | Benzyl |
| Bzls | Benzylsulfonyl |
| dCha | d-βCyclohexylalanin |
| CKIBE | Chlorkohlensäureisobutylester |
| DIEA | Diisopropylethylamin |
| DCM | Dichlormethan |
| DMF | N,N-Dimethylformamid |
| i.V. | im Vakuum |
| MS | Massenspektroskopie |
| NMM | N-Methylmorpholin |
| PyBOP | Benzotriazol-1-yl-N-oxy-tris(pyrrolidino)phosphoniumhexafluorophosphat |
| TEA | Triethylamin |
| TFA | Trifluoressigsäure |
| THF | Tetrahydrofuran |
| TMS-Cl | Trimethylsilylchlorid |
| tBu | tert.-Butyl |

### Beispiel 1

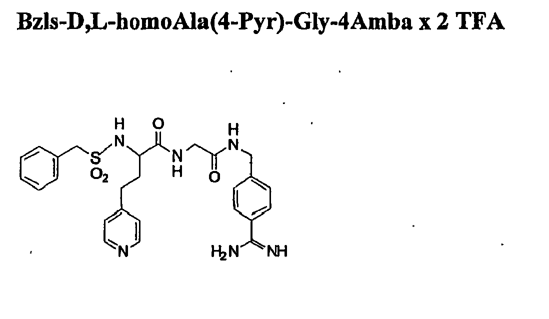

### 1a) H-Gly-4-(Acetyloxamidino)Benzylamid x HCl (H-Gly-Amb(4AcOxam))

2 g (5,49 mmol) Boc-Gly-4-(Acetyloxamidino)Benzylamid (hergestellt wie in WO 01/96286 A2 beschrieben) wurden mit 30 ml 1 N HCl in Eisessig versetzt. Der Ansatz wurde gelegentlich umgeschüttelt. Nach 45 min wurde das Lösungsmittel etwas eingeengt und das Produkt durch Zugabe von Diethylether gefällt, auf einer Fritte abgesaugt, mit Ether gewaschen und im Vakuum getrocknet.
Ausbeute: 1,55 g (5,15 mmol), weißer Feststoff

### 1b) Boc-D,L-homoAla(4-Pyr)-Gly-Amb(4AcOxan)

250 mg (0,89 mmol) Boc-D,L-homoAla(4-Pyr)-OH [RSP Amino Acids DBA, Shirley MA, USA] und 308 mg (1,02 mmol) Produkt 1a wurden in 20 ml DMF gelöst und bei 0 °C mit 531 mg (1,02 mmol) PyBop und 533 µl (3,06 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, 1x mit NaCl- gesättigtem Wasser, 2x mit gesättigter NaHCO₃-Lösung und 2x mit NaClgesättigtem Wasser gewaschen und über Na₂SO₄ getrocknet. Das Lösungsmittel wurde im Vakuum entfernt (gelbliches Öl).
Ausbeute: ca. 600 mg (Rohprodukt), HPLC: 27,89% B

### 1c) H-D.L-homoAla(4-Pyr)-Gly-Amb(4AcOxam) x HCl

600 mg Rohprodukt 1b wurden mit 10 ml 1N HCl in Eisessig versetzt. Der Ansatz wurde gelegentlich umgeschüttelt. Nach 1 h wurde das Lösungsmittel etwas eingeengt und das Produkt durch Zugabe von Diethylether gefällt, auf einer Fritte abgesaugt, mit Ether gewaschen und im Vakuum getrocknet.
Ausbeute: 320 mg (0,69 mmol) hellgelber Feststoff, HPLC: 16,83% B

### 1 d) Bzls-D.L-homoAla(4-Pyr)-Gly-Amb(4AcOxam)

75 mg (0,16 mmol) Rohprodukt 1c und 37 mg (0,19 mmol) Phenylmethansulfonylchlorid (Bzls-Cl) [Fluka] wurden in 10 ml DMF gelöst und bei 0 °C mit 68 µl (0,39 mmol) DIEA versetzt. Der Ansatz wurde 20 min bei 0°C und weitere 2 h bei Raumtemperatur gerührt. Anschließend wurde das Lösungsmittel im Vakuum entfernt, der Rückstand in Essigester aufgenommen, 1x mit NaCl- gesättigtem Wasser, 2x mit gesättigter NaHCO₃-Lösung und 2x mit NaCl- gesättigtem Wasser gewaschen und über Na₂SO₄ getrocknet. Das Lösungsmittel wurde im Vakuum entfernt (helles Öl).
Ausbeute: ca. 280 mg (Rohprodukt), HPLC: 29,27% B

### 1e) Bzls-D,L-homoAla(4-Pyr)-Gly-4Amba

Das Rohprodukt 1d wurde in 50ml 90% Essigsäure gelöst und mit 20 mg Katalysator (10% Pd/C) versetzt. Der Ansatz wurde bei Raumtemperatur und Normaldruck für 5 h mit Wasserstoff hydriert. Anschließend wurde der Katalysator abfiltriert und das Lösungsmittel im Vakuum eingeengt. Der verbleibende Rückstand wurde im Vakuum getrocknet und mit präparativer reversed- phase HPLC gereinigt und lyophilisiert.
Ausbeute: 34,6 mg (0,054 mmol) lyophilisiertes Pulver, HPLC: 22,97% B

MS: berechnet 522,20 (monoisotopic), gefunden 523,4 [M+M]⁺

### Beispiel 2

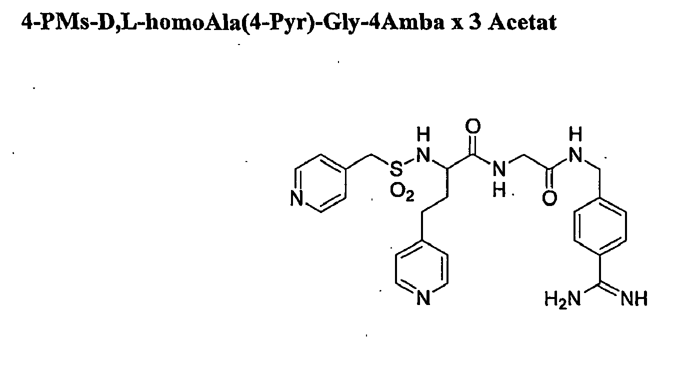

### 2a) 4-PMs-D,L-homoA1a(4-Pyr)-Gly-Amb(4AcOxam)

50 mg (0,11 mmol) Produkt 1c wurden in 10 ml DCM suspendiert und mit 34 µl (0,28 mmol) Chlortrimethylsilan (= TMS-Cl) [Merck] und 69 µl (0,4 mmol) DIEA versetzt und der Ansatz 15 min bei Raumtemperatur gerührt. Anschließend wurden 41 mg (0,12 mmol) 4-Pyridylmethylsulfonylchlorid x Triflat (= 4-PMs-Cl) [Array Biopharma, Boulder, CO, USA] und weitere 20 µl (0,11 mmol) DIEA zugegeben und weiter über Nacht bei Raumtemperatur gerührt. Dann wurde das Lösungsmittel im Vakuum entfernt. Der Rückstand wurde ohne weitere Reinigung direkt für den nächsten Syntheseschritt eingesetzt.

### 2b) 4-PMs-D,L-homoAla(4-Pyr)-Gly-4Amba

Das Rohprodukt 2a wurde in 50ml 90% Essigsäure gelöst und mit 20mg Katalysator (10% Pd/C) versetzt. Der Ansatz wurde bei Raumtemperatur und Normaldruck über Nacht mit Wasserstoff hydriert. Anschließend wurde der Katalysator abfiltriert und das Lösungsmittel im Vakuum eingeengt. Der verbleibende Rückstand wurde in 5 ml Wasser gelöst und auf eine Ionenaustauschsäule (Fractogel- EMD COO- Säule, Dimension 16 x 125 mm, equilibriert mit Wasser) aufgegeben. Die Säule wurde mit 85 ml Wasser gewaschen und anschließend das Produkt mit einem Ammoniumacetat- Gradienten eluiert. Die Produkt-enthaltenden Fraktionen (HPLC-Kontrolle) wurden vereinigt und lyophilisiert.
Ausbeute: 20 mg (0,034 mmol) lyophilisiertes Pulver, HPLC: 13,14% B

MS: berechnet 523,20 (monoisotopic), gefunden 524,3 [M+H]⁺

### Beispiel 3

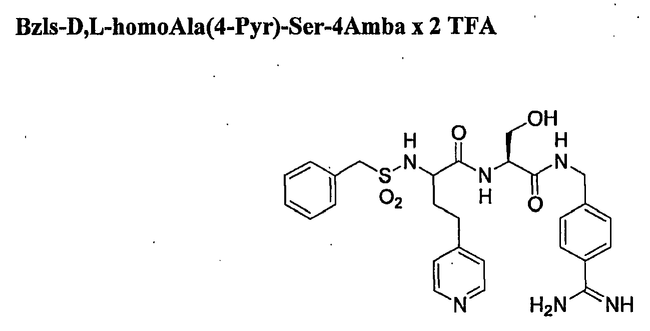

### 3a) Boc-4-Cyanobenzylamid

100 g (0,593 mol) 4-Cyanobenzylamin x HCl wurden in 1,21 Dioxan und 600 ml 2 N NaOH gelöst. 142,3 g (0,652 mol) di(tert.-Butyl)Pyrocarbonate wurden bei 0 °C über 10 min in zwei Portionen zugegeben. Der pH-Wert wurde durch Zugabe von 2 N NaOH auf 9-10 eingestellt und der Ansatz für weitere 4 h gerührt. Das Lösungsmittel wurde im Vakuum entfernt, der Rückstand mit Essigester aufgenommen, jeweils 3x mit 5% KHSO₄ und NaCl- gesättigtem Wasser gewaschen und anschließend über Na₂SO₄ getrocknet. Das Lösungsmittel wurde im Vakuum entfernt (weißer Feststoff).
Ausbeute: 132,6 g (0,57 mol) weißer Feststoff, HPLC: 51,6% B

### 3b) Boc-4-Acetyloxamidinobenzylamid

130 g (0,56 mol) Produkt 3a, 58,4 g (0,84 mol) Hydroxylamin x HCl und 146 ml DIEA wurden in 1,51 Methanol gelöst. Der Ansatz wurde 6 h unter Rückfluß gekocht und anschließend über Nacht bei Raumtemperatur gerührt. Das Lösungsmittel wurde im Vakuum entfernt, der ölige Rückstand in 1,51 Essigsäure gelöst, mit 160 ml (1,68 mol) Acetanhydrid versetzt und 30 min gerührt. Das Lösungsmittel wurde im Vakuum entfernt, der Rückstand mit Essigester aufgenommen und 3x mit NaCl- gesättigtem Wasser gewaschen und anschließend über Na₂SO₄ getrocknet. Das Lösungsmittel wurde im Vakuum weitestgehend entfernt und das Produkt aus Essigester kristallisiert.
Ausbeute: 110,6 g (0,36 mol) kristalliner Feststoff, HPLC: 39,76% B

### 3c) H-4-Aceiyloxamidinobenzylamin x HCl

50 g (163 mmol) Produkt 3b wurden in 11 Essigsäure gelöst und es wurden 800 ml 1N HCl in Eisessig zugegeben. Der Ansatz wurde geschüttelt und nach einigen Minuten begann das Produkt auszufallen. Nach 75 min wurde das Produkt abgesaugt, mit Diethylether gewaschen und im Vakuum getrocknet.
Ausbeute: 36 g (147,7 mmol) weißer Feststoff, HPLC: 18,97% B

### 3d) Boc-Ser-4-Acetyloxamidinobenzylamid

25 g (122 mmol) Boc-Ser-OH wurden in 750 ml DMF gelöst und auf -15°C gekühlt. Es wurden 13,42 ml (122 mmol) N-Methylmorpholin und 15,86 ml (122 ml) Chlorkohlensäureisobutylester zugegeben und 10 min gerührt. Dann wurden 29,74 g (122 mmol) Produkt 3c und 13,42 ml (122 mmol) N-Methylmorpholin hinzugefügt und der Ansatz für 1 h bei -15°C und über Nacht bei Raumtemperatur gerührt. Anschließend wurde das DMF im Vakuum entfernt, der Rückstand in 21 Essigester gelöst und jeweils 2x mit 300 ml gesättigter NaHCO₃- Lösung und 300 ml NaCl- gesättigtem Wasser gewaschen, über Na₂SO₄ getrocknet und das Lösungsmittel im Vakuum entfernt (Öl).
Ausbeute: 43 g Rohprukt Öl, HPLC: 29,87% B

### 3e) H-Ser-4-Acetyloxamidinobenzylamid x TFA

40 g des öligen Rohproduktes 3d wurden mit 200 ml Trifluoressigsäure versetzt und 1 h gerührt. Das Produkt wurde durch Zugabe von Diethylether gefällt, abgesaugt, mit Diethylether gewaschen und im Vakuum getrocknet.
Ausbeute: 27 g (66 mmol) weißer Feststoff, HPLC: 20,22% B

### 3f) Boc-D.L-homoAla(4-Pyr)-Ser-Amb(4AcOxam)

100 mg (0,36 mmol) Boc-D,L-homoAla(4-Pyr)-OH [RSP Amino Acids DBA, Shirley MA, USA] und 161 mg (0,4 mmol) Rohprodukt 3e wurden in 15 ml DMF gelöst und bei 0 °C mit 206 mg (0,4 mmol) PyBop und 207 µl (1,2 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, 1x mit NaCl- gesättigtem Wasser, 2x mit gesättigter NaHCO₃-Lösung und 2x mit NaClgesättigtem Wasser gewaschen und über Na₂SO₄ getrocknet. Das Lösungsmittel wurde im Vakuum entfernt (helles Öl).
Ausbeute: ca. 300 mg (Rohprodukt), HPLC: 26,8% B und 27,4% B (Doppelpeak, Racemat)

### 3g) H-D,L-homoAla(4-Pyr)-Ser-Amb(4AcOxam) x TFA

300 mg Rohprodukt aus 3f wurden in 5 ml 50% TFA in Dichlormethan versetzt. Der Ansatz wurde gelegentlich umgeschüttelt. Nach 45 min wurde das Lösungsmittel eingeengt, der Rückstand in Methanol angelöst und das Produkt durch Zugabe von Diethylether gefällt, auf einer Fritte abgesaugt, mit Ether gewaschen und im Vakuum getrocknet.
Ausbeute: 186 mg (0,33 mmol) weißer Feststoff, HPLC: 21,6% B und 22,7% B (Doppelpeak, Racemat)

### 3h) Bzls-D,L-homoAla(4-Pyr)-Ser-Amb(4AcOxam

75 mg (0,13 mmol) Produkt 3g und 38 mg (0,2 mmol) Phenylmethansulfonylchlorid (= Bzls-Cl) [Fluka] wurden in 10 ml DMF gelöst und bei 0 °C mit 68 µl (0,39 mmol) DIEA versetzt. Der Ansatz wurde 20 min bei 0°C und über Nacht bei Raumtemperatur gerührt. Das Lösungsmittel wurde im Vakuum entfernt (Öl).
Ausbeute: ca. 120 mg (Rohprodukt), HPLC: 28,1% B und 28,6% B (Doppelpeak)

### 3i) Bzls-D,L-homoAla(4-Pyr)-Ser-4Amba x 2 TFA

Das Rohprodukt aus 3h wurde in 50ml 90% Essigsäure gelöst und mit 20 mg Katalysator (10% Pd/C) versetzt. Der Ansatz wurde bei Raumtemperatur und Normaldruck über Nacht mit Wasserstoff hydriert. Anschließend wurde der Katalysator abfiltriert und das Lösungsmittel im Vakuum eingeengt. Der verbleibende Rückstand wurde im Vakuum getrocknet und mit präparativer reversed- phase HPLC gereinigt und lyophilisiert. Dadurch konnten die Diastereomere getrennt werden.

HPLC: 22,01% B (Verbindung 3a) und 22,6% B (Verbindung 3b).

MS: berechnet 552,22 (monoisotopic), gefunden 553,5 [M+H]⁺

### Beispiel 4

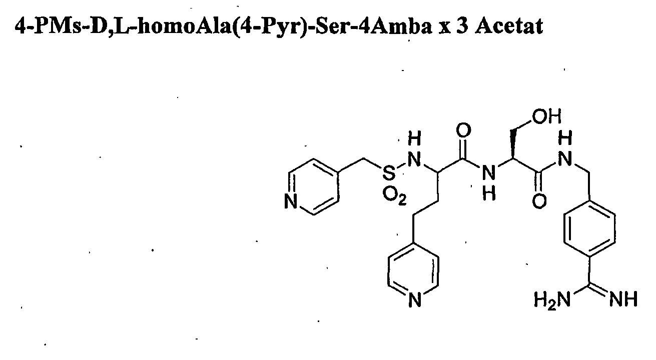

### 4a) 4-PMs-D,L-homoAla(4-Pyr)-Ser-Amb(4AcOxam)

55 mg (0,1 mmol) Produkt 3g wurden in 10 ml DCM suspendiert und mit 31 µl (0,25 mmol) Chlortrimethylsilan (= TMS-Cl) [Merck] und 61 µl (0,36 mmol) DIEA versetzt und der Ansatz 15 min bei Raumtemperatur gerührt. Anschließend wurden 36 mg (0,105 mmol) 4-Pyridylmethylsulfonylchlorid x Triflat (= 4-PMs-Cl) [Array Biopharma, Boulder, CO, USA] und weitere 17,5 µl (0,1 mmol) DIEA zugegeben und weiter über Nacht bei Raumtemperatur gerührt. Dann wurde das Lösungsmittel im Vakuum entfernt. Der Rückstand wurde ohne weitere Reinigung direkt für den nächsten Syntheseschritt eingesetzt.

### 4b) 4-PMs-D,L-homoAla(4-Pyr)-Ser-4Amba x 3 Acetat

Das Rohprodukt aus 4a wurde in 50mol 90% Essigsäure gelöst und mit 20mg Katalysator (10% Pd/C) versetzt. Der Ansatz wurde bei Raumtemperatur und Normaldruck über Nacht mit Wasserstoff hydriert. Anschließend wurde der Katalysator abfiltriert und das Lösungsmittel im Vakuum eingeengt. Der verbleibende Rückstand wurde in 5 ml Wasser gelöst und auf eine Ionenaustauschsäule (Fractogel- EMD COO- Säule, Dimension 16 x 125 mm, equilibriert mit Wasser) aufgegeben. Die Säule wurde mit 85 ml Wasser gewaschen und anschließend das Produkt mit einem Ammoniumacetat- Gradienten eluiert. Die Produkt-enthaltenden Fraktionen wurden vereinigt und lyophilisiert.
Ausbeute: 17,2 mg (0,028 mmol) lyophilisiertes Pulver, HPLC: 12,1 undd 12,3% B (Doppelpeak, Racemat)

MS: berechnet 553,21 (monoisotopic), gefunden 554,5 [M+H]⁺

### Beispiel 5

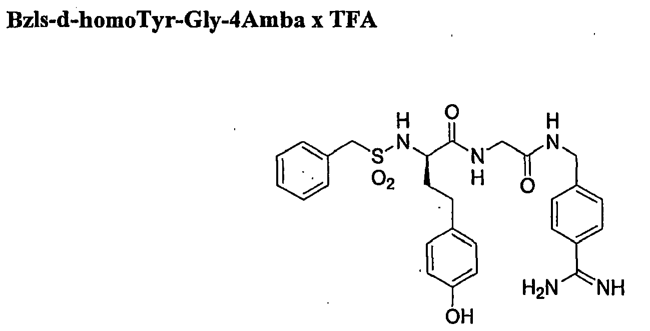

### 5a) Bzls-d-homoTyr-OH

300 mg (1,09 mmol) H-d-homoTyr-OH x HBr [Chem-Impex International, Wood Dale, IL, USA] wurden in 20 ml DCM suspendiert und mit 425 µl (3,37 mmol) Chlortrimethylsilan (= TMS-Cl) [Merck] und 586 µl (3,37 mmol) DIEA versetzt und der Ansatz 1 h bei 60 °C unter Rückfluß gerührt, dann wieder auf Raumtemperatur abgekühlt. Anschließend wurden 229 mg (1,2 mmol) Phenylmethansulfonylchlorid (= Bzls-Cl) [Fluka] und weitere 190 µl (1,09 mmol) DIEA zugegeben und für 2 h bei Raumtemperatur gerührt. Das Lösungsmittel wurde im Vakuum entfernt, der Rückstand in Essigester aufgenommen, 1x mit 5% KHSO₄- Lösung und 2x mit NaCl- gesättigtem Wasser gewaschen und über Na₂SO₄ getrocknet. Nach Entfernen des Lösungsmittels wurde das Produkt aus Essigester kristallisiert.
Ausbeute: 353 mg (1,01 mmol) hellgelber Feststoff, HPLC: 40,9% B

### 5b) Bzls=d-homoTyr-Gly-Amb(4AcOxam)

50 mg (0,14 mmol) Produkt 5a und 43 mg (0,14 mmol) H-Gly-Amb(4AcOxam) (= Produkt 1a) wurden in 15 ml DMF gelöst und bei 0 °C mit 74,4 mg (0,14 mmol) PyBop und 74,6 µl (0,43 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, 1x mit 5% KHSO₄- Lösung und 2x mit NaClgesättigtem Wasser gewaschen und über Na₂SO₄ getrocknet. Als Rückstand blieb ein helles Öl.
Ausbeute: ca. 200 mg (Rohprodukt), HPLC: 39,84% B

### 5c) Bzls-d-homoTyr-Gly-4Amba

Das Rohprodukt aus 5b wurde in 50ml 90% Essigsäure gelöst und mit 20 mg Katalysator (10% Pd/C) versetzt. Der Ansatz wurde bei Raumtemperatur und Normaldruck für 6 h mit Wasserstoff hydriert. Anschließend wurde der Katalysator abfiltriert und das Lösungsmittel im Vakuum eingeengt. Der verbleibende Rückstand wurde im Vakuum getrocknet und ohne weitere Vorreinigung mit präparativer reversed- phase HPLC gereinigt und lyophilisiert.
Ausbeute: 37,5 mg (0,058 mmol) lyophilisiertes Pulver, HPLC: 32,37% B

MS: berechnet 537,20 (monoisotopic), gefunden 538,4 [M+H]⁺

### Beispiel 6

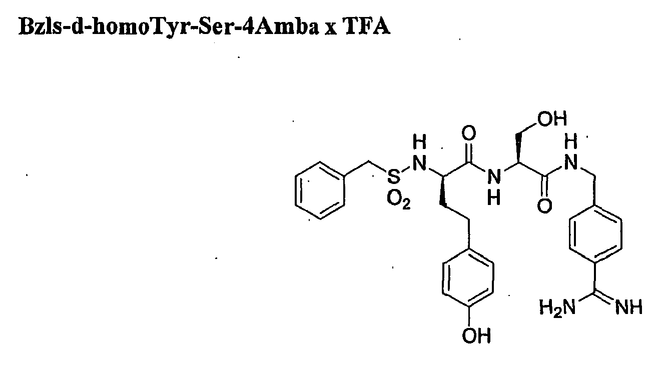

### 6a) Bzls-d-homoTyr-Ser-Amb(4AcOxam)

50 mg (0,14 mmol) Produkt 5a und 58,4 mg (0,14 mmol) H-Ser-Amb(4AcOxam) (= Produkt 3e) wurden in 15 ml DMF gelöst und bei 0 °C mit 74,4 mg (0,14 mmol) PyBop und 74,6 µl (0,43 mmol) DIEA versetzt. Der Ansatz wurde 20 min bei 0°C und über Nacht bei Raumtemperatur gerührt. Anschließend wurde das Lösungsmittel im Vakuum entfernt, der Rückstand in Essigester aufgenommen, 1x mit 5% KHSO₄- Lösung und 2x mit NaClgesättigtem Wasser gewaschen und über Na₂SO₄ getrocknet (helles Öl).
Ausbeute: ca. 165 mg (Rohprodukt), HPLC: 38,49% B

### 6b) Bzls-d-homoTyr-Ser-4Amba x TFA

Das Rohprodukt aus 6a wurde in 50ml 90% Essigsäure gelöst und mit 20 mg Katalysator (10% Pd/C) versetzt. Der Ansatz wurde bei Raumtemperatur und Normaldruck für 6 h mit Wasserstoff hydriert. Anschließend wurde der Katalysator abfiltriert und das Lösungsmittel im Vakuum eingeengt. Der verbleibende Rückstand wurde im Vakuum getrocknet und ohne weitere Vorreinigung mit präparativer reversed- phase HPLC gereinigt und lyophilisiert.
Ausbeute: 38 mg (0,056 mmol) lyophilisiertes Pulver, HPLC: 31,74% B

MS: berechnet 567,22 (monoisotopic), gefunden 568,5 [M+H]⁺

### Beispiel 7

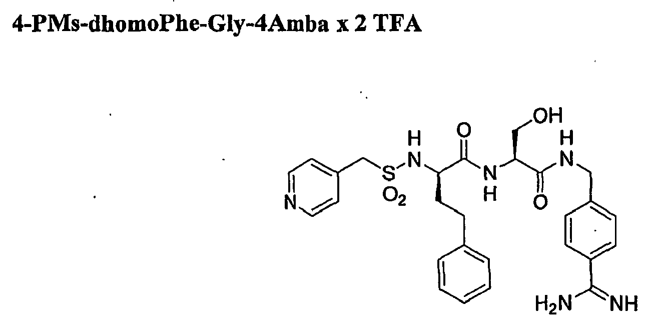

### 7a) Boc-d-homoPhe-Gly-Amb(4AcOxam)

732 mg (2,62 mmol) Boc-d-homoPhe-OH [Bachem] und 788 mg (2,62 mmol) H-Gly-Amb(4AcOxam) (=Produkt 1a) wurden in 50 ml DMF gelöst und bei 0 °C mit 1,36 g (2,62 mmol) PyBop und 1,37 ml (7,86 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, jeweils 2x mit 5% KHSO₄, gesättigter NaHCO₃-Lösung und NaCl- gesättigtem Wasser gewaschen und anschließend über Na₂SO₄ getrocknet. Das Lösungsmittel wurde im Vakuum entfernt (hellbraunes Öl).
Ausbeute: ca. 1,8 g (Rohprodukt), HPLC: 47,87% B

### 7b) H-d-homoPhe-Gly-Amb(4AcOxam) x HCl

600 mg Rohprodukt aus 7a wurden mit 15 ml 1 N HCl in Eisessig versetzt. Der Ansatz wurde gelegentlich umgeschüttelt. Nach 1 h wurde das Lösungsmittel etwas eingeengt und das Produkt durch Zugabe von Diethylether gefällt, auf einer Fritte abgesaugt, mit Ether gewaschen und im Vakuum getrocknet.
Ausbeute: 1,02 g (2,2 mmol) hellgelber Feststoff, HPLC: 28,11% B

### 7c) 4-PMs-dhomoPhe-Gly-Amb(4AcOxam)

50 mg (0,11 mmol) Produkt 7b wurden in 10 ml DCM suspendiert und mit 20,5 µl (0,16 mmol) Chlortrimethylsilan (= TMS-Cl) [Merck] und 49 µl (0,28 mmol) DIEA versetzt und der Ansatz 15 min bei Raumtemperatur gerührt. Anschließend wurden 41 mg (0,12 mmol) 4-Pyridylmethylsulfonylchlorid x Triflat (= 4-PMs-Cl) [Array Biopharma, Boulder, CO, USA] und weitere 20 µl (0,11 mmol) DIEA zugegeben und weiter für 2 h bei Raumtemperatur gerührt. Dann wurde das Lösungsmittel im Vakuum entfernt. Der Rückstand wurde ohne weitere Reinigung direkt für den nächsten Syntheseschritt eingesetzt

### 7d) 4-PMs-dhomoPhe-Gly-4Amba x 2 TFA

Das Rohprodukt aus 7c wurde in 50ml 90% Essigsäure gelöst und mit 20mg Katalysator (10% Pd/C) versetzt. Der Ansatz wurde bei Raumtemperatur und Normaldruck über Nacht mit Wasserstoff hydriert. Anschließend wurde der Katalysator abfiltriert und das Lösungsmittel im Vakuum eingeengt. Der verbleibende Rückstand wurde im Vakuum getrocknet und ohne weitere Vorreinigung mit präparativer reversed- phase HPLC gereinigt und lyophilisiert.
Ausbeute: 30 mg (0,047 mmol) lyophilisiertes Pulver, HPLC: 26,01% B

MS: berechnet 522,20 (monoisotopic), gefunden 523,3 [M+H]⁺

### Beispiel 8

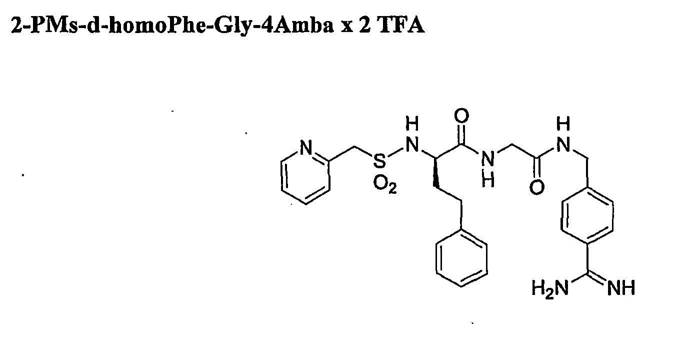

### 8a) 2-PMs-dhomoPhe-OH

75 mg (0,42 mmol) H-d-homoPhe-OH [Bachem] wurden in 10 ml DCM suspendiert und mit 116 µl (0,92 mmol) Chlortrimethylsilan (= TMS-Cl) [Merck] und 160 µl (0,92 mmol) DIEA versetzt und der Ansatz 1 h bei 60 °C unter Rückfluß gerührt, dann wieder auf Raumtemperatur abgekühlt. Anschließend wurden 150 mg (0,44 mmol) 2-Pyridylmethylsulfonylchlorid x Triflat (= 2-PMs-Cl) [Array Biopharma, Boulder, CO, USA] und weitere 77 µl (0,44 mmol) DIEA zugegeben und weiter über Nacht bei Raumtemperatur gerührt. Dann wurde das Lösungsmittel entfernt. Der Rückstand wurde ohne weitere Reinigung direkt für den nächsten Syntheseschritt eingesetzt.
Ausbeute: ca. 300 mg Rohprodukt, HPLC: 32,86% B

### 8b) 2-PMs-dhomoPhe-Gly-Amb(4AcOxam)

150 mg (ca. 0,2 mmol) Rohprodukt 8a und 60,2 mg (0,2mmol) H-Gly-Amb(4AcOxam (=Produkt 1a) wurden in 10 ml DMF gelöst und bei 0 °C mit 104 mg (0,2 mmol) PyBop und 104,5 µl (0,6 mmol) DIEA versetzt. Der Ansatz wurde 20 min bei 0°C und weitere 2h bei Raumtemperatur gerührt. Anschließend wurde das Lösungsmittel im Vakuum entfernt, der Rückstand in Essigester aufgenommen, 2x mit gesättigter NaHCO₃-Lösung und 2x mit NaCl- gesättigtem Wasser gewaschen und über Na₂SO₄ getrocknet. Das Lösungsmittel wurde im Vakuum entfernt (hellbraunes Öl).

HPLC: 35,28% B

### 8c) 2-PMs-dhomoPhe-Glv-4Amba

Das Rohprodukt aus 8b wurde in 50ml 90% Essigsäure gelöst und mit 20 mg Katalysator (10% Pd/C) versetzt. Der Ansatz wurde bei Raumtemperatur und Normaldruck für 5 h mit Wasserstoff hydriert. Anschließend wurde der Katalysator abfiltriert und das Lösungsmittel im Vakuum eingeengt. Der verbleibende Rückstand wurde im Vakuum getrocknet und ohne weitere Vorreinigung mit präparativer reversed- phase HPLC gereinigt und das Produkt lyophilisiert.
Ausbeute: 69 mg (0,11 mmol) lyophilisiertes Pulver, HPLC: 31,18% B

MS: berechnet 522,20 (monoisotopic), gefunden 523,4 [M+H]⁺

### Beispiel 9

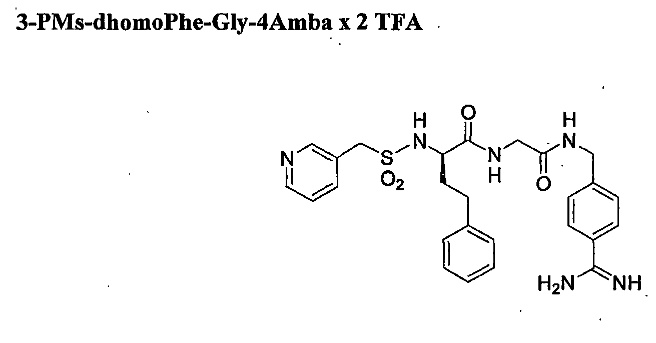

### 9) 3-PMs-dhomoPhe-Gly-4Amba

Beispiel 9 wurde in Analogie zu Beispiel 8 synthetisiert, nur dass 3-Pyridylmethylsulfonylchlorid x Triflat (= 3-PMs-Cl) [Array Biopharma, Boulder, CO, USA] verwendet wurde. Das Endprodukt wurde mit präparativer reversed- phase HPLC gereinigt und lyophilisiert.
Ausbeute: 62 mg (0,097 mmol) lyophilisiertes Pulver, HPLC: 29,08% B
MS: berechnet 522,20 (monoisotopic), gefunden 523,4 [M+H]⁺

**Tabelle 1: Bestimmung der Hemmkonstanten für Faktor Xa und Thrombin. Zusätzlich ist das Selektivitätsverhältnis SV angegeben (SV = K_{i Thrombin}/K_{i Faktor Xa}).**

| Nr. | Struktur | Kᵢ (µM) | | SV |
|---|---|---|---|---|
| | | Faktor Xa | Thrombin | |
| 10 | 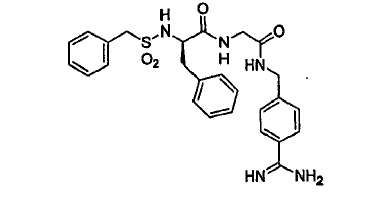 | 0,026 | 0,068 | 2,6 |
| 11 | 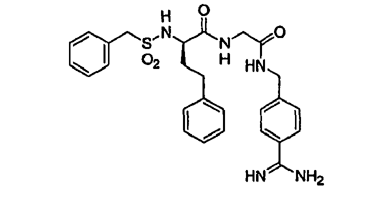 | 0,0065 | 0,047 | 7,2 |
| 12 | 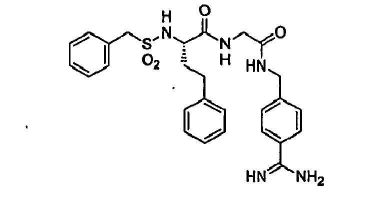 | 0,36 | 11 | 31 |
| 13 | 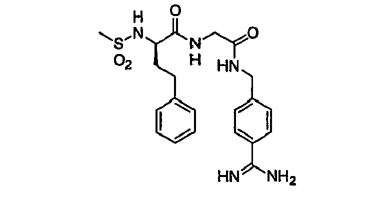 | 1, 1 | 1,3 | 1,2 |
| 8 | 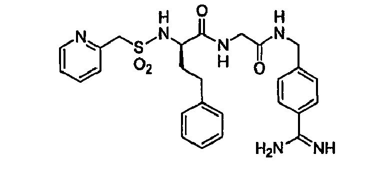 | 0,051 | 4,9 | 96 |
| 9 | 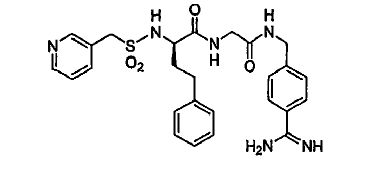 | 0,062 | 5,9 | 95 |
| 14 | 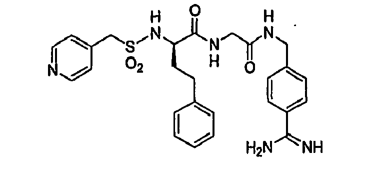 | 0,08 | 3,6 | 45 |
| 15 | 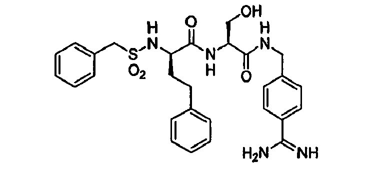 | 0,04 | 0,6 | 15 |
| 7 | 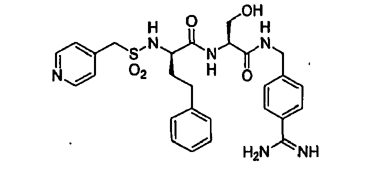 | 0,46 | 3,3 | 7,2 |
| 16 | 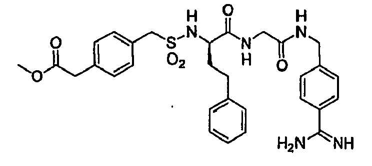 | 0,038 | 1,8 | 47 |
| 17 | 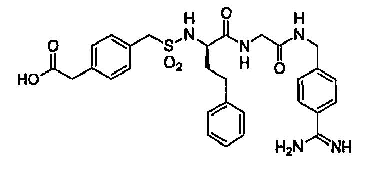 | 0,054 | 14 | 259 |
| 18 | 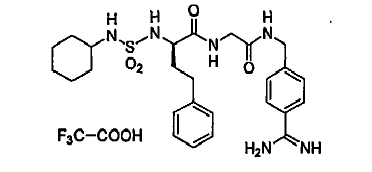 | 0,11 | 5,4 | 49 |
| 19 | 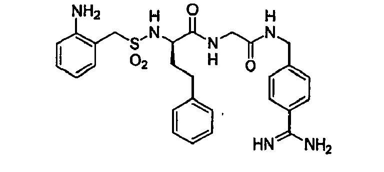 | 0,0067 | 0,92 | 137 |
| 20 | 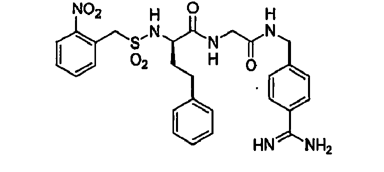 | 0,026 | 1,2 | 46 |
| 5 | 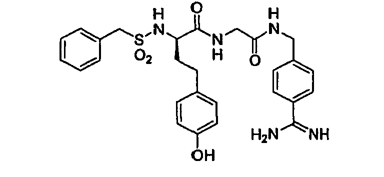 | 0,0027 | 1,5 | 556 |
| 6 | 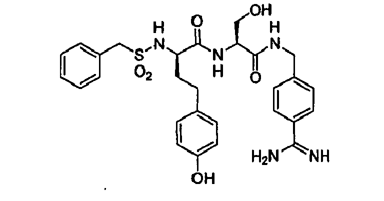 | 0,019 | 1,4 | 74 |
| 1 | 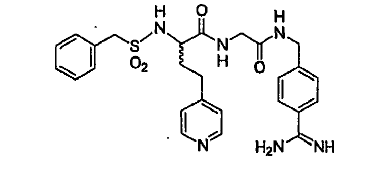 | 0,0029 | 2 | 690 |
| 2 | 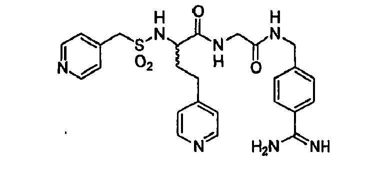 | 0,013 | 3,2 | 246 |
| 3a | 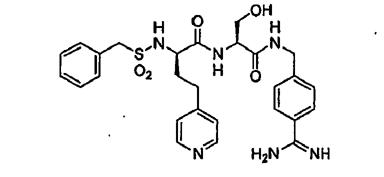 | 0,0094 | 0,91 | 97 |
| 3b | 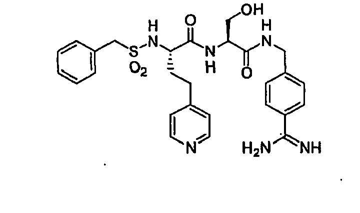 | 0,095 | 4,6 | 48,4 |
| 4 | 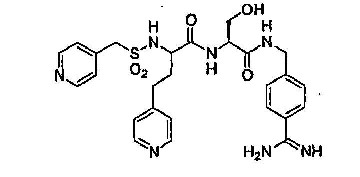 | 0,097 | 6,3 | 65 |
| 21 | 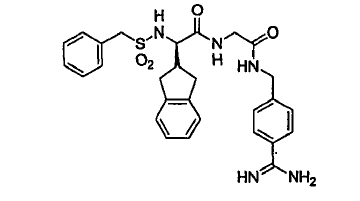 | 0,029 | 0,15 | 5,2 |
| | | | | |
| 22 | 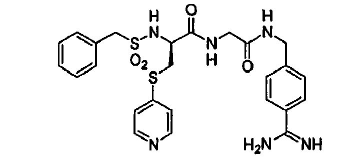 | 0,0027 | 0,7 | 259 |
| 23 | 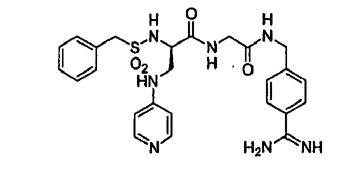 | 0,022 | 2,8 | 127 |
| 24 | 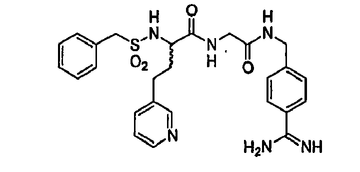 | 0,005 | 2,0 | 400 |
| 25 | 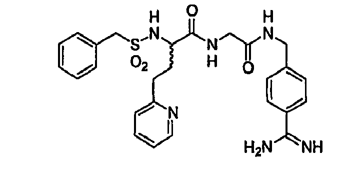 | 0,0021 | 2,0 | 952 |
| 26 | 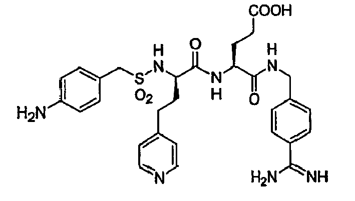 | 0,0017 | 25 | 14705 |
| 27 | 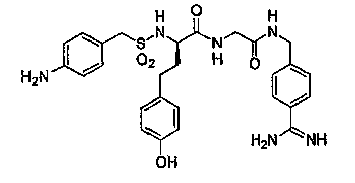 | 0,0019 | 0,56 | 295 |
| 28 | 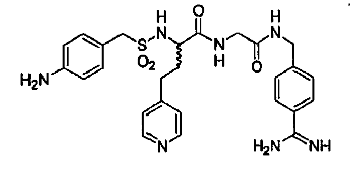 | 0,0022 | 1 | 454 |
| 29 | 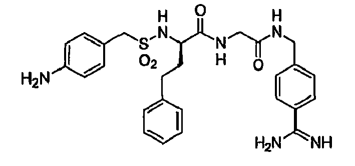 | 0,0026 | 0,26 | 100 |
| 30 | 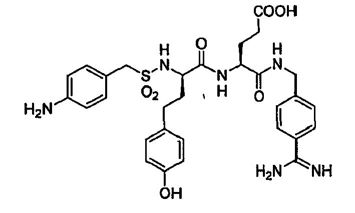 | 0,0034 | 78 | 22940 |
| 31 | 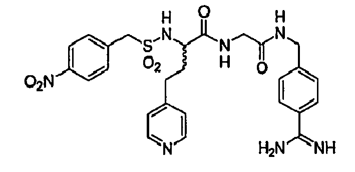 | 0,0035 | 1,9 | 543 |
| 32 | 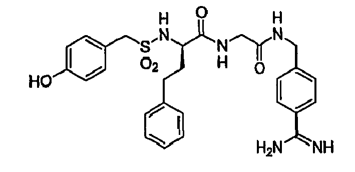 | 0,0036 | 0,38 | 105 |
| 33 | 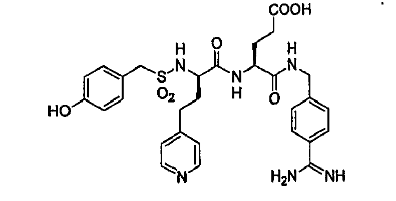 | 0,0036 | 100 | 27778 |
| 34 | 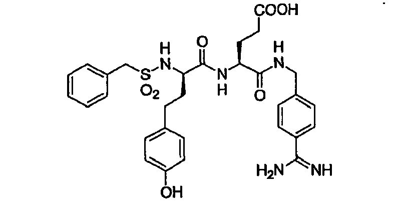 | 0,0037 | 19 | 5135 |
| 35 | 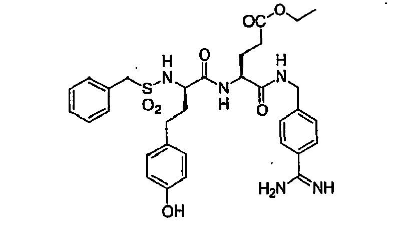 | 0,005 | 1 | 200 |
| 36 | 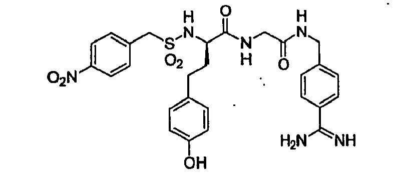 | 0,0052 | 0,86 | 165 |
| 37 | 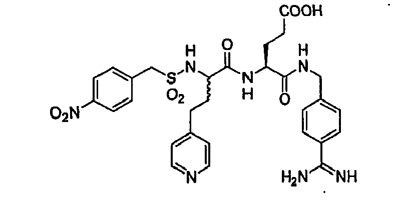 | 0,0056 | 35 | 6250 |
| 38 | 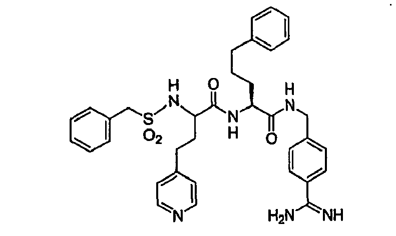 | 0,006 | 0,18 | 30 |
| 39 | 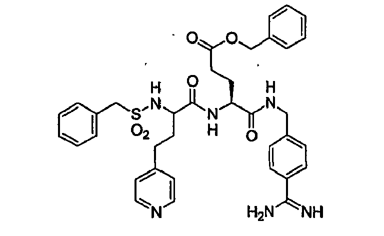 | 0,0064 | 0,17 | 26 |
| 40 | 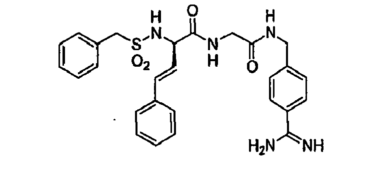 | 0,0065 | 1,1 | 170 |
| 41 | 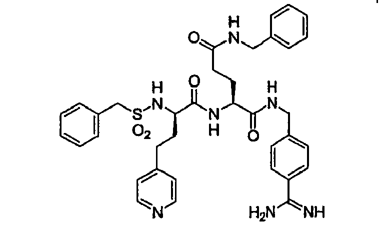 | 0,0068 | 1,7 | 250 |
| 42 | 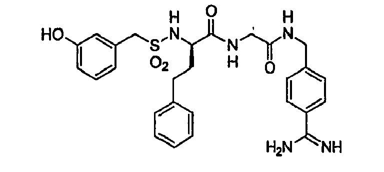 | 0,0072 | 1,5 | 288 |
| 43 | 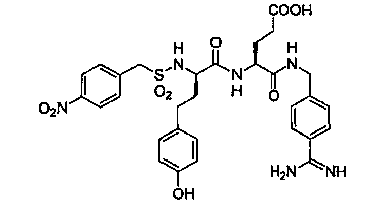 | 0,0075 | 15 | 2000 |
| 44 | 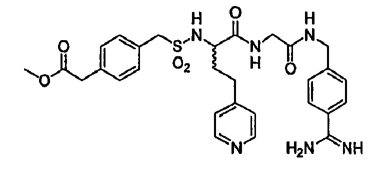 | 0,0082 | 3,8 | 463 |
| 45 | 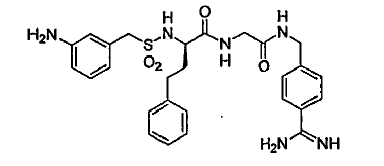 | 0,0093 | 1,4 | 150 |
| 46 | 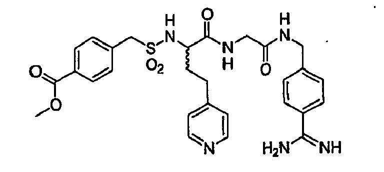 | 0,0098 | 7,6 | 775 |
| 47 | 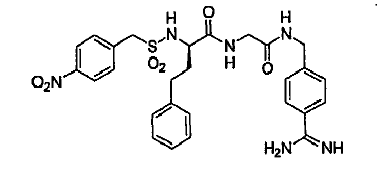 | 0,01 | 0,71 | 71 |
| 48 | 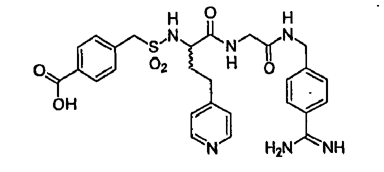 | 0,01 | n.b.^{*} | - |
| 49 | 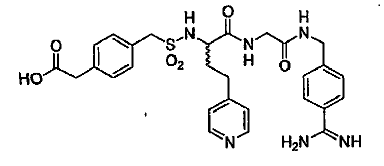 | 0,013 | 38 | 2923 |
| 50 | 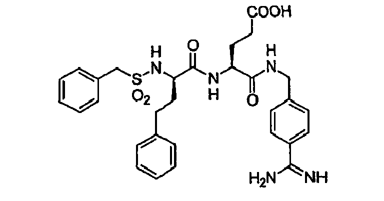 | 0,013 | 15 | 1153 |
| 51 | 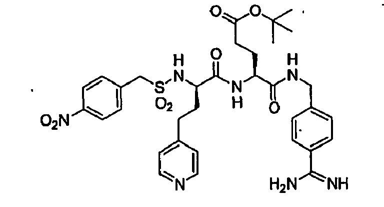 | 0,016 | 1,4 | 87 |
| 52 | 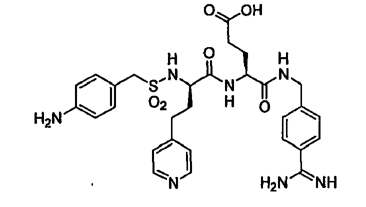 | 0,016 | 84 | 5250 |
| 53 | 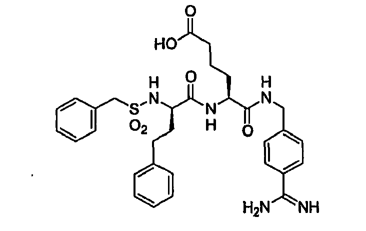 | 0,03 | 0,8 | 27 |
| 54 | 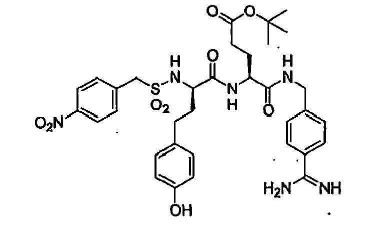 | 0,039 | 0,69 | 18 |
| 55 | 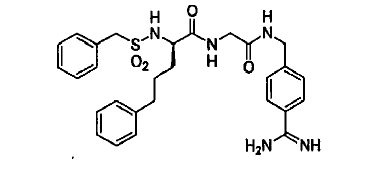 | 0,067 | 0,21 | 3 |
| 56 | 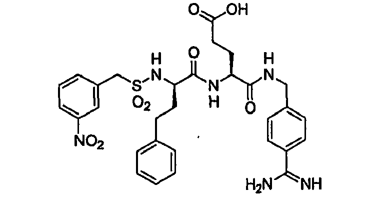 | 0,083 | 13 | 156 |
| 57 | 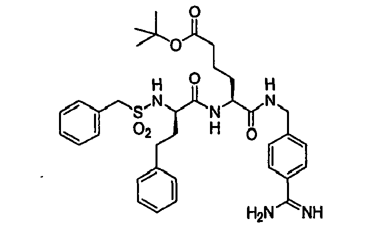 | 0,13 | 0,46 | 3,5 |
| 58 | 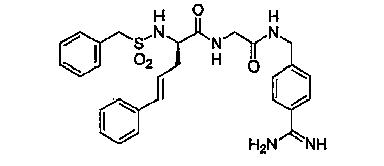 | 0,58 | 1,8 | 3,1 |
| 59 | 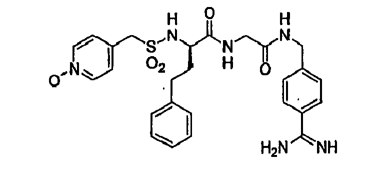 | 0,97 | 16 | 16 |
| 60 | 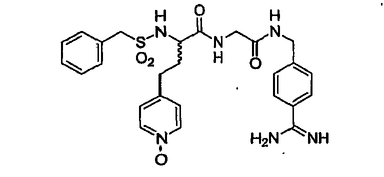 | 0,0048 | 3,5 | 730 |
| | | | | |
| 61 | 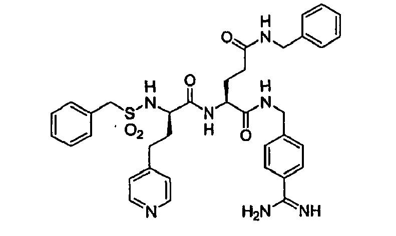 | 0,0068 | 1,7 | 250 |
| 62 | 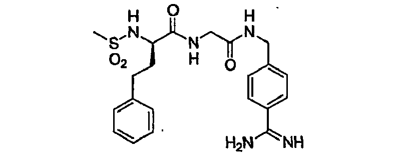 | 1,06 | 1,3 | 1,2 |
| 63 | 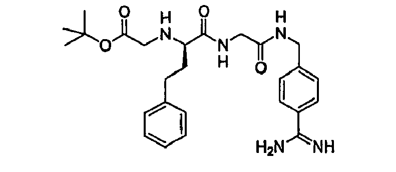 | 0,62 | 1,5 | 2,4 |
| 64 | 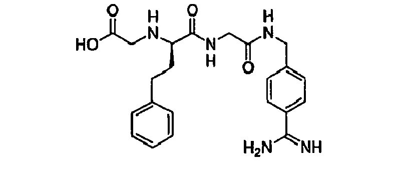 | 0,87 | 28 | 32 |
| 65 | 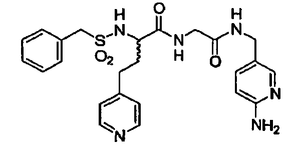 | 0,12 | 100 | 833 |

| | | | | |
|---|---|---|---|---|
| ^{*}n.b. = nicht bestimmt | | | | |

### Bestimmung der Hemmwirkung

Zur Bestimmung der Hemmwirkung wurden 200 µl Tris-Puffer (0,05 M, 0,154 M NaCl, 5% Ethanol, pH 8,0; enthält den Inhibitor), 25 µl Substrat (Moc-D-Nle-Gly-Arg-pNA in H₂O; Pentapharm Ltd., Basel, Schweiz) und 50 µl Faktor Xa (vom Rind, Diagnostic Reagents Ltd, Thame, GB) bei 25 °C inkubiert. Nach 3 min wurde die Reaktion durch Zugabe von 25 µl Essigsäure (50%) unterbrochen und die Absorption bei 405 nm mittels Microplate Reader (MR 5000, Dynatech, Denkendorf, Deutschland) bestimmt. Die Kᵢ-Werte wurden nach Dixon (Biochem. J. 55, 170-171, 1953) durch lineare Regression mittels eines Computerprogramms ermittelt. Die Kᵢ-Werte sind das Mittel aus mindestens drei Bestimmungen. Die Bestimmung der Thrombinhemmung erfolgte analog einer früher beschriebenen Methode (Stürzebecher et al., J. Med. Chem. 40, 3091-3099, 1997).

Die folgenden Seiten 47 bis 100 beinhalten spezielle Ausführungsbeispiele.
1. Verbindung der allgemeinen Formel I 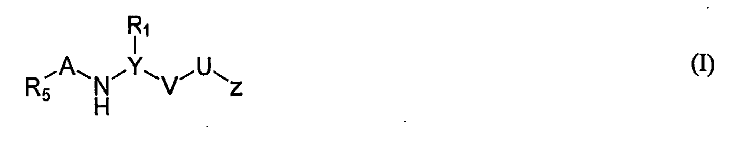 wobei
   A P₂ - P₁ ist, mit 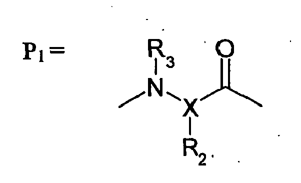 und 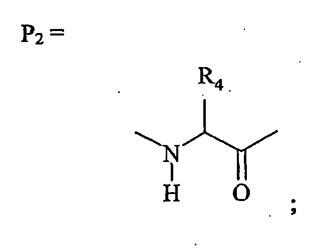
   R₁ ein H oder -(CH₂)ₐCOOR₆ mit a = 0, 1, 2, 3, 4 oder 5, vorzugsweise mit a= 0, 1 oder 2, ist, wobei R₆ ein verzweigter oder unverzweigter Alkylrest mit vorzugsweise 1 bis 6 C-Atomen, insbesondere 1 bis 3 C-Atomen, vor allem Ethyl, insbesondere ist R₁ ein H;
   R₂ ein H, -CH₂-OR₇ oder -CH₂-OCOOR₇ ist, wobei R₇ ein H oder ein verzweigter oder unverzweigter Alkylrest mit 1-5, insbesondere 1-3 C-Atomen, ist, oder R₂ ist ein -CH₂-CH₂-COOR_{7*}, wobei R_{7*} ein H oder ein verzweigter oder unverzweigter Alkylrest mit 1-5 C-Atomen, vorzugsweise Ethyl, ist;
   R₃ ein H ist;
   R₄ -(CH₂)_{f}-R₈ mit f = 0 oder 2, vorzugsweise mit f = 2, -CH₂NHR₈, -(CH₂)₂NHR₈ oder -CH=CH-R₈ ist, wobei R₈ ein einfach oder mehrfach substituierter oder unsubstituierter Cycloalkyl-, Aryl- oder Heteroarylrest ist, wobei der Cycloalkyl-, Aryl- oder Heteroarylrest vorzugsweise 5 bis 14, insbesondere 5 bis 6 C-Atome im Ring und im Falle des Heteroarylrestes vorzugsweise 1 bis 3 N als Heteroatome besitzt, oder wenn R₄ gleich -(CH₂)_{f}-R₈ mit R₈ gleich ein Hydroxycycloalkylrest mit 4 bis 14, insbesondere 6 bis 10, vor allem 6 C-Atomen ist, dann ist f = 1, und wobei P₂ in der Struktur A der allgemeinen Formel I in der D- oder L-Konfiguration, vorzugsweise in der D-Konfiguration vorliegt;
   R₅ -(CH₂)ᵢ-COOR₉ mit i = 1, 2 oder 3, vorzugsweise mit i = 1, und R₉ gleich ein verzweigter oder unverzweigter Alkylrest mit 1-5 C-Atomen, vorzugsweise Ethyl, ist, oder R₅ ist -SO₂R_{9*}, -SO₂-NH-R_{9*}, wobei R_{9*} ein H, ein verzweigtes oder unverzweigtes Alkyl mit 1-10, vorzugsweise 1 bis 6, insbesondere 1 bis 4, vor allem 1 bis 2 C-Atomen, ein einfach oder mehrfach substituierter oder unsubstituierter Aryl-, Heteroaryl-, Aralkyl-, vorzugsweise Benzyl-, Heteroaralkylrest oder ein Cyclohexylalkylrest, vorzugsweise ein Cyclohexylmethylrest ist, wobei der Substituent eine -OH, -O-COOR₇, -CH₂-OCOOR₇, mit R₇ wie oben definiert, -NH₂, -NO₂, -COOR₁₀, -CH₂-COOR₁₀-Gruppe oder ein Cl-, F- oder Br-Atom sein kann und wobei R₁₀ ein H oder ein Alkylrest mit 1 bis 6, insbesondere mit 1 bis 4 C-Atomen, vor allem Ethyl, ist;
   U ein Phenyl- oder Cyclohexylrest ist; ein aromatischer oder nichtaromatischer heterocyclischer Rest mit 1-10, vorzugsweise 6 Ringatomen mit mindestens einem N, S oder O als Heteroatom, insbesondere Pyridin, Piperidin oder Pyrimidin, ist, oder ein Thienylrest ist;
   V (CH₂)ₙ mit n = 0 oder 1, vorzugsweise 0, ist;
   X N oder CH, vorzugsweise CH, ist;
   Y N oder CH, vorzugsweise CH, ist;
   Z in 2, 3- oder 4-Position, vorzugsweise in 4-Position, vorkommt und eine Aminomethyl-, eine Guanidinofunktion oder eine Amidinogruppe 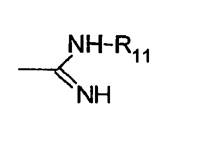
   ist, wobei R₁₁ H, OH, NH₂, -COR₁₂ oder -COOR₁₂ ist, wobei R₁₂ ein verzweigter oder unverzweigter Alkylrest mit 1 bis 8, vorzugsweise 1 bis 6 C-Atomen oder ein einfach oder mehrfach substituierter oder unsubstituierter Aryl- oder Heteroaryl-, Aralkyl- oder Heteroaralkylrest ist, wobei der Alkylrest vorzugsweise 1 bis 16, insbesondere 1 bis 8, vor allem 1 bis 4 und besonders bevorzugt 1 bis 2 C-Atome und der Aryl- oder Heteroarylrest vorzugsweise 4 bis 14, insbesondere 6 bis 10, vor allem 6 C-Atome und vorzugsweise 1 bis 3 N als Heteroatome besitzt;
   oder eine Verbindung der allgemeinen Formel I in Form eines Prodrugs oder in Form ihres Salzes.
2. Verbindung nach 1, wobei U an 1, 2 oder 3 Positionen mit einem Halogen, insbesondere Fluor oder Chlor, oder einem Methyl-, Ethyl-, Propyl-, Methoxy-, Ethoxy-, oder Propoxyrest substituiert ist.
3. Verbindung nach 1 oder 2, wobei mindestens eine Carboxylgruppe als Ester, bevorzugt als Ethylester, geschützt vorliegt.
4. Verbindung nach mindestens einem der 1 bis 3 in Form eines Prodrugs, bei dem ein oder mehrere Carboxylgruppen in Form ihrer Alkylester mit einem verzweigten oder unverzweigten Alkyl mit 1-5 C-Atomen, vorzugsweise Ethyl, und/oder bei dem ein oder mehrere Hydroxylgruppen als Carbonate, bei denen der endständige Rest gleich R₇, wie oben definiert vorliegen, und/oder bei dem der Amidino- bzw. Guanidinobenzylaminrest als Hydroxyamidin bzw. Hydroxyguanidin oder als Alkyloxycarbonylderivat vorliegt.
5. Verbindung nach mindestens einem der 1 bis 4, **dadurch**
   **gekennzeichnet, dass** das Strukturelement 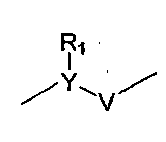 der Formel I eine -CH₂- oder -NH-Gruppe, vorzugsweise eine -CH₂-Gruppe bedeutet.
6. Verbindung nach mindestens einem der 1 bis 5, **dadurch gekennzeichnet, dass**
   R₁ ein H ist;
   R₂ ein H, -CH₂-CH₂-COOH, -CH₂-CH₂-COOCH₂CH₃ oder -CH₂OH ist;
   R₃ ein H ist;
   R₄ ein -(CH₂)₂-R₈, -CH₂NHR₈, -(CH₂)₂NHR₈ oder ein -CH₂-4-Hydroxycyclohexylrest ist, wobei R₈ ein einfach oder mehrfach substituierter oder unsubstituierter Cycloalkyl-, Aryl- oder Heteroarylrest ist, wobei der Cycloalkyl-, Aryl- oder Heteroarylrest 5 oder 6 C-Atome und im Falle eines Heteroarylrestes 1 oder 2 N als Heteroatome besitzt, vorzugsweise ist R₈ ein Phenyl-, Hydroxyphenyl-, Pyridyl- oder Aminopyridylrest;
   R₅ ein Methylsulfonyl, Ethylsulfonyl, n-Propylsulfonyl, iso-Propylsulfonyl, Butylsulfonyl, n-Butylsulfonyl, Benzylsulfonyl, Aminobenzylsulfonyl, Hydroxybenzylsulfonyl, Chlorobenzylsulfonyl, Fluorobenzylsulfonyl, Carboxybenzylsulfonyl, Ethyloxycarbonylbenzylsulfonyl, Carboxymethylbenzylsulfonyl, Ethyloxycarbonylmethylbenzylsulfonyl, Pyridylmethylsulfonyl, N-(oxid)-pyridylmethylsulfonyl, -CH₂-COOH oder ein -CH₂COOCH₂CH₃ Rest ist;
   U ein Phenylrest ist;
   V (CH₂)ₙ mit n = 0 ist;
   X CH ist;
   Y CH ist;
   Z in 4-Position vorkommt und eine Amidinogruppe 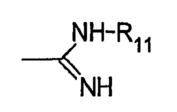 ist,
      wobei R₁₁ H, OH oder -COOR₁₂ mit R₁₂ ein verzweigter oder unverzweigter Alkylrest mit 2, 4 oder 6 C-Atomen ist.
7. Verbindung nach mindestens einem der 1 bis 6, **dadurch gekennzeichnet, dass** R₄ ein -CH₂-CH₂-R₈ Rest ist, wobei R₈ ein Aryl- oder Heteroarylrest mit 4-6 Ringatomen ist, der 1 oder 2 Heteroatome, vorzugsweise N, besitzt und der mit einer oder mehreren NH₂ und/oder -OH Gruppen substituiert sein kann, vorzugsweise ist P₂ in der Struktur A der allgemeinen Formel I von einem Homophenylalanin, Homotyrosin, Indanylglycin oder 4-Pyridylhomoalanin abgeleitet, und die P₂-Aminosäure liegt insbesondere in der D-Konfiguration vor.
8. Verbindung nach mindestens einem der 1 bis 7, **dadurch gekennzeichnet, dass** der Substituent -OH, -NH₂, -NO₂, -COOH, -COOCH₂CH₃, ein Halogen, vorzugsweise Fluor, Chlor oder Brom, insbesondere Fluor oder Chlor, ist.
9. Verbindung nach mindestens einem der 1 bis 8, **dadurch**
   **gekennzeichnet, dass** die Verbindung ausgewählt ist aus folgenden Strukturen: 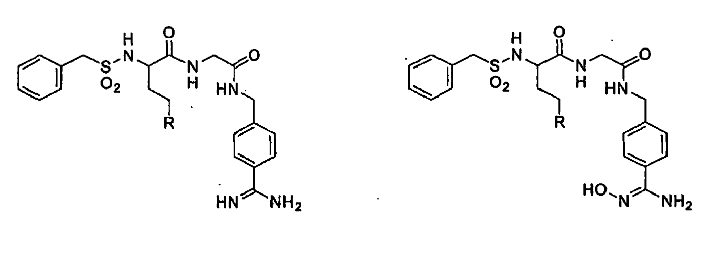 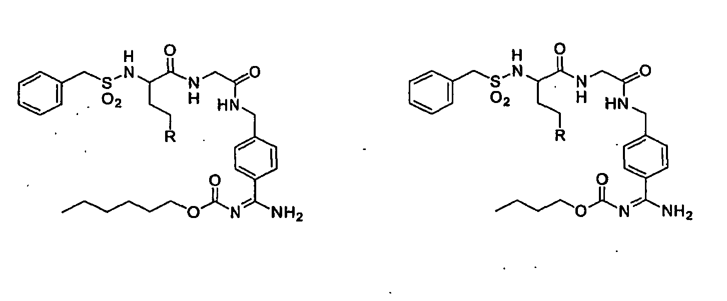 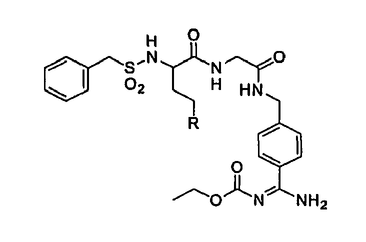 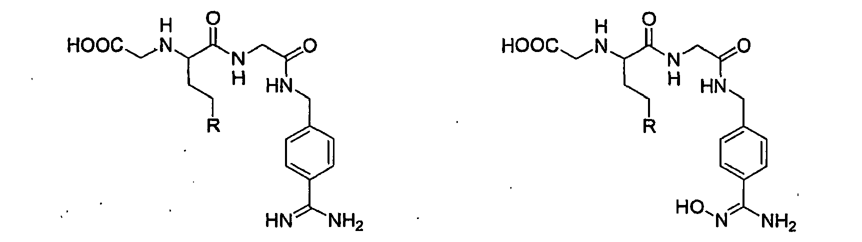 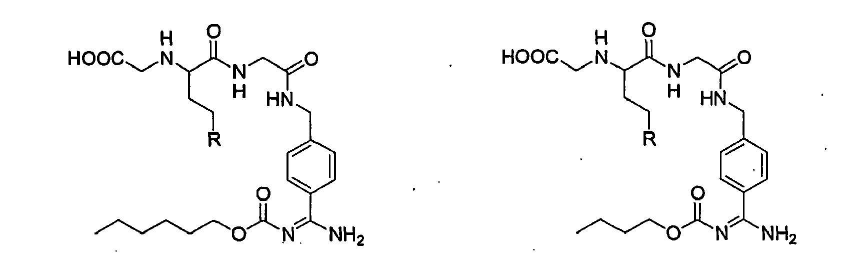 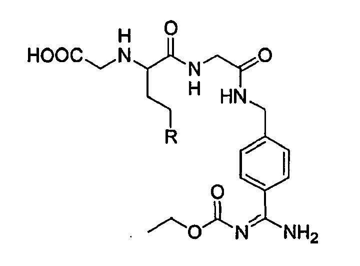 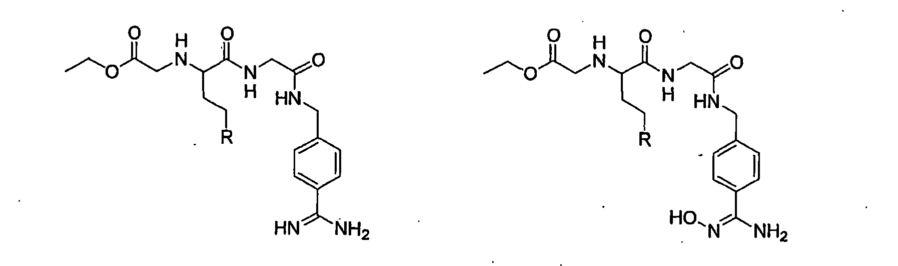 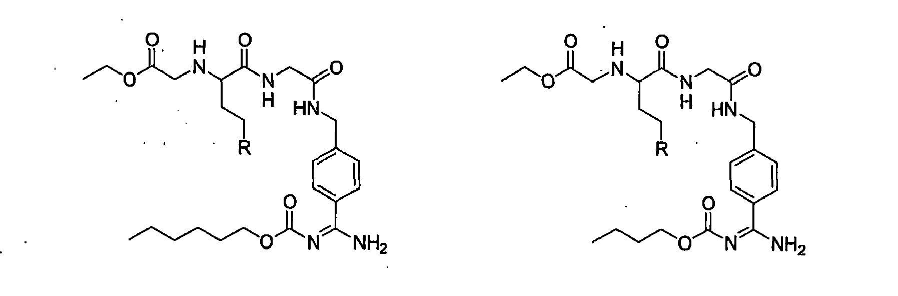 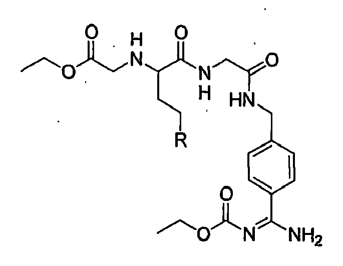 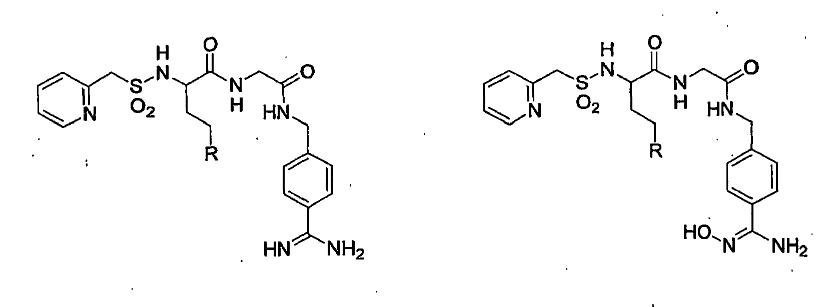 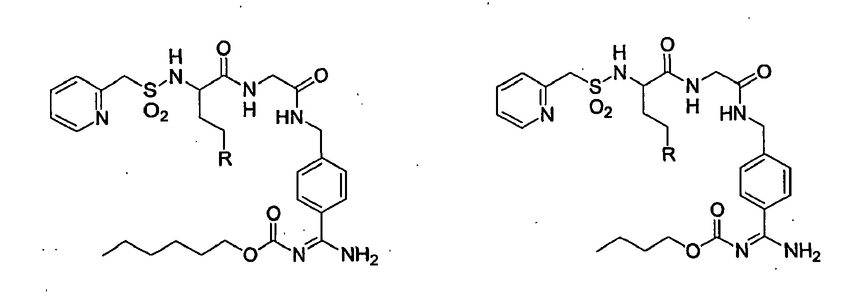 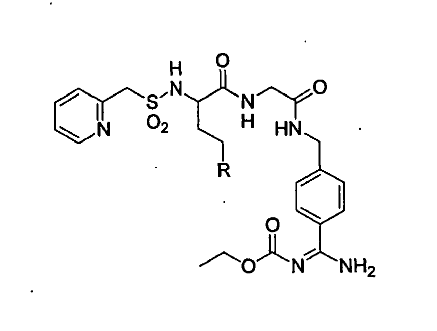 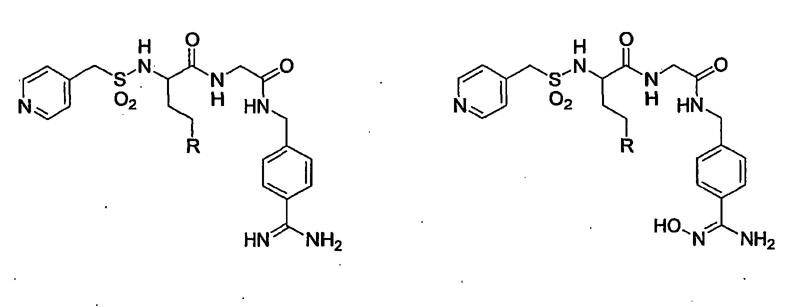 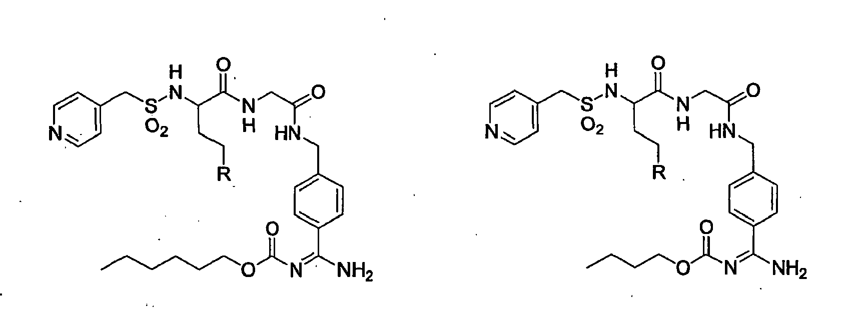 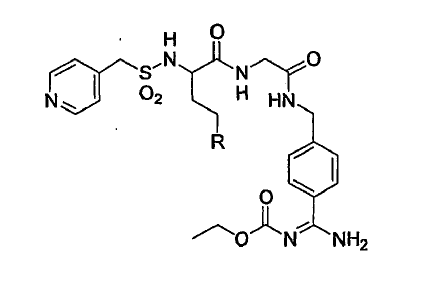 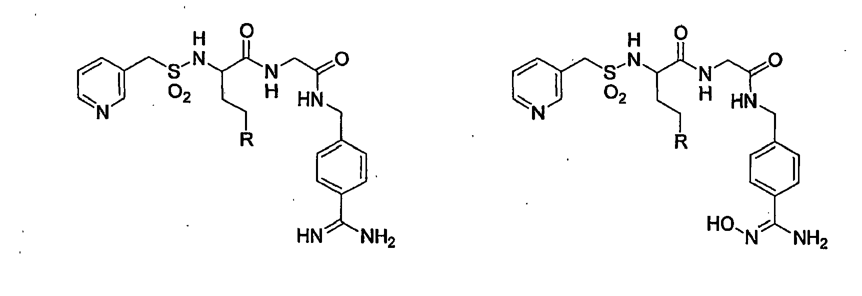 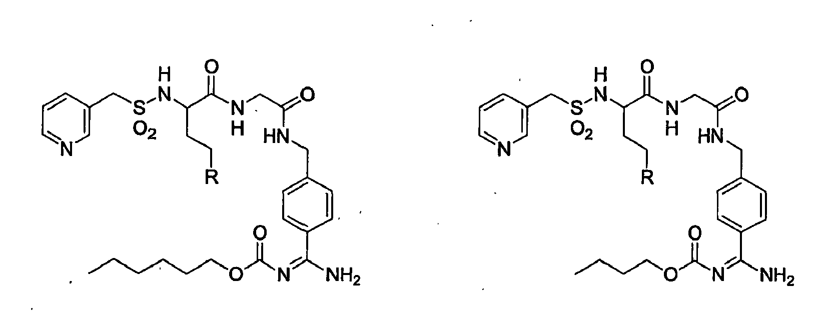 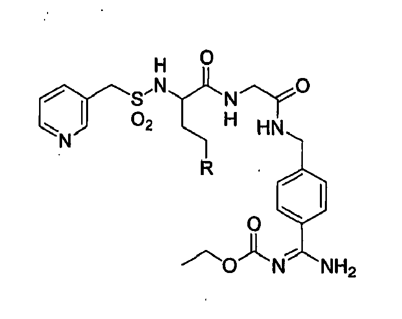 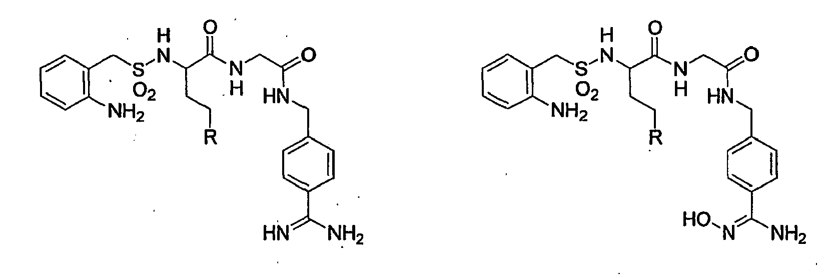 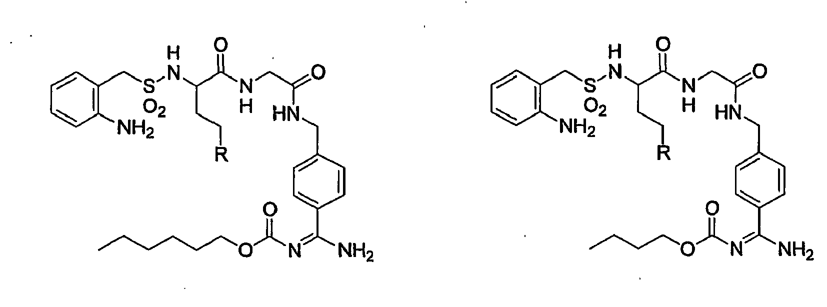 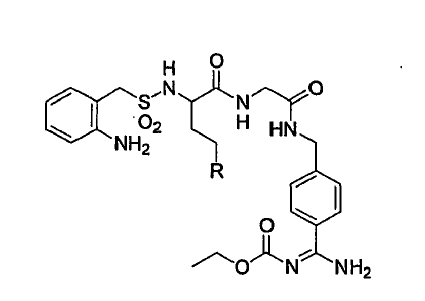 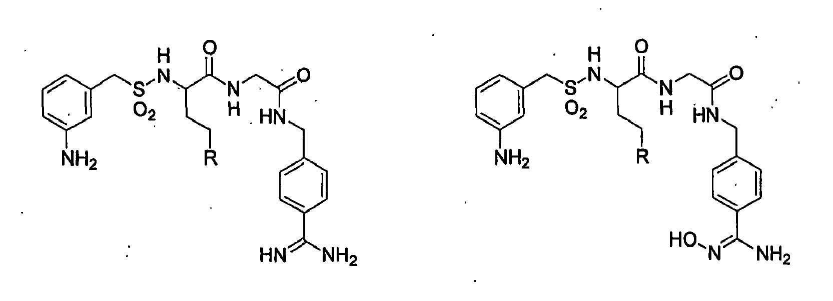 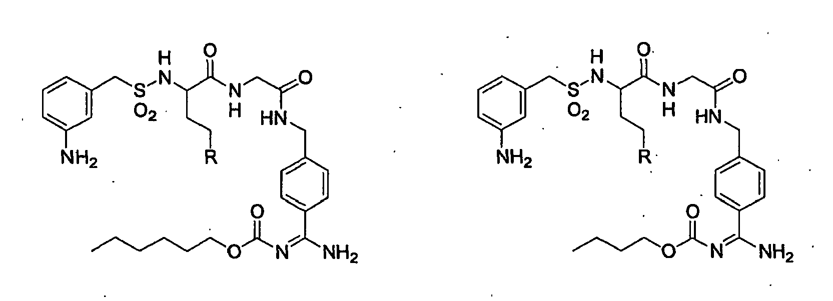 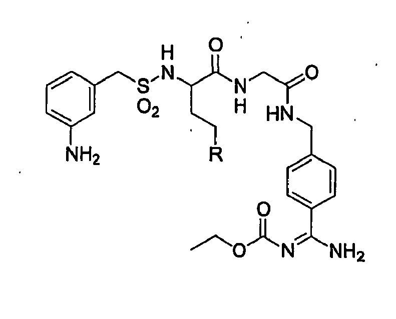 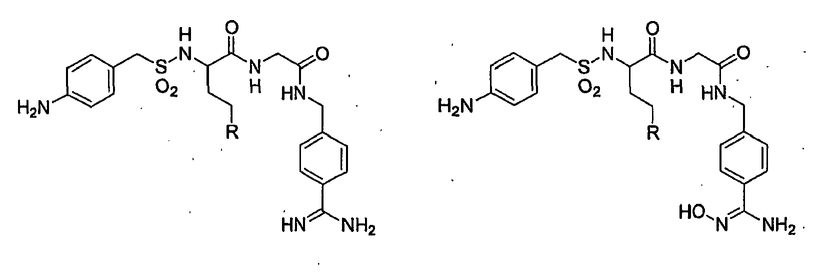 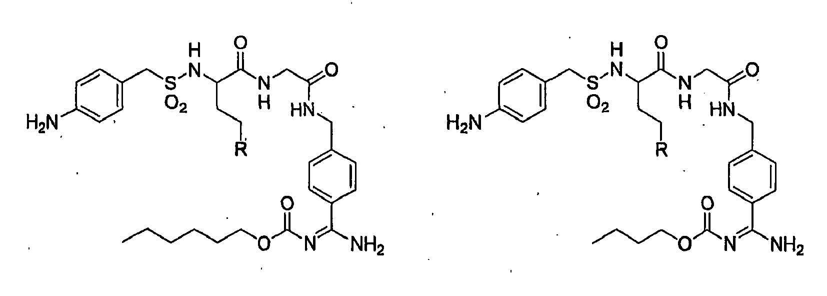 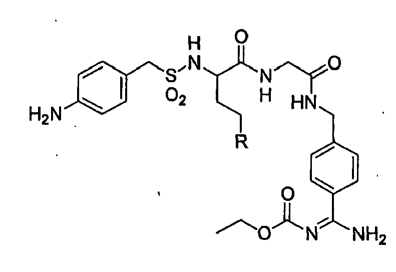 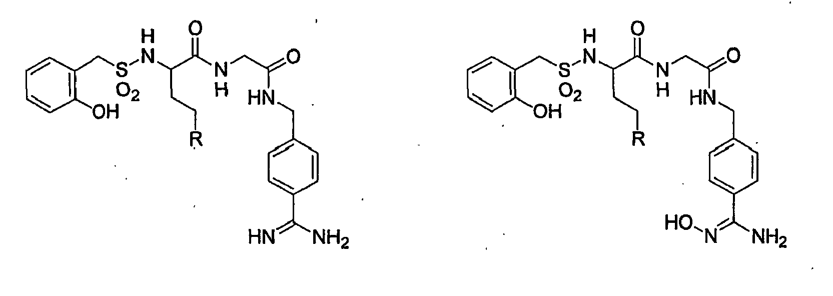 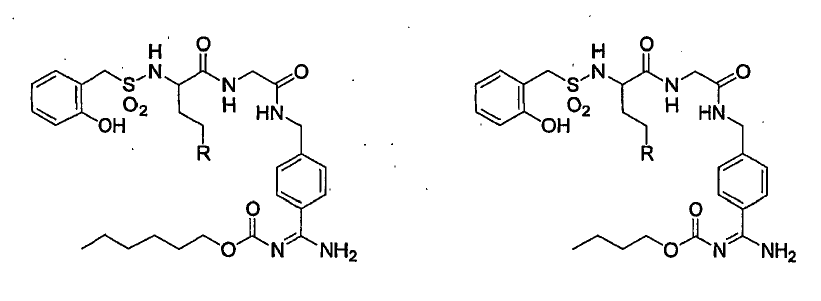 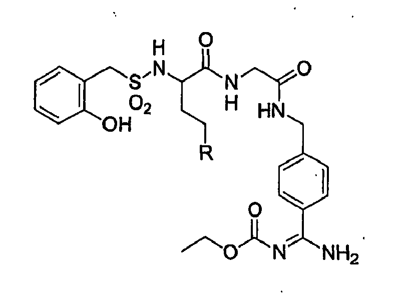 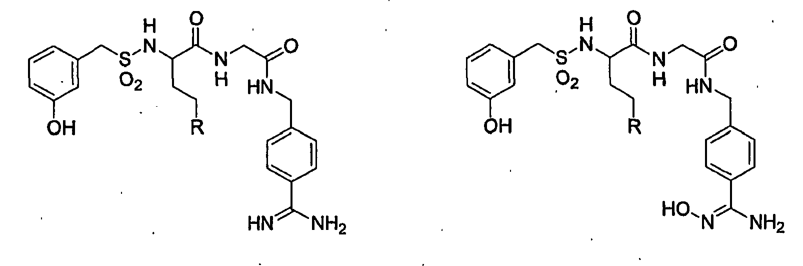 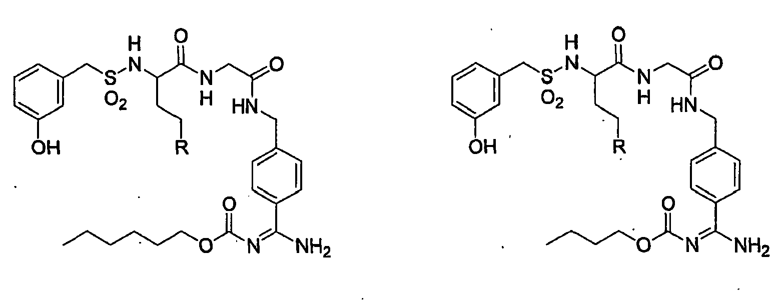 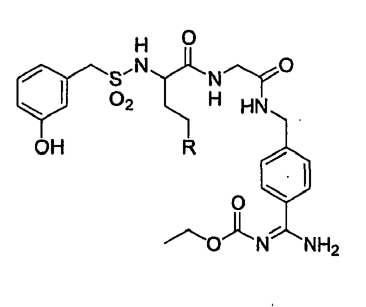 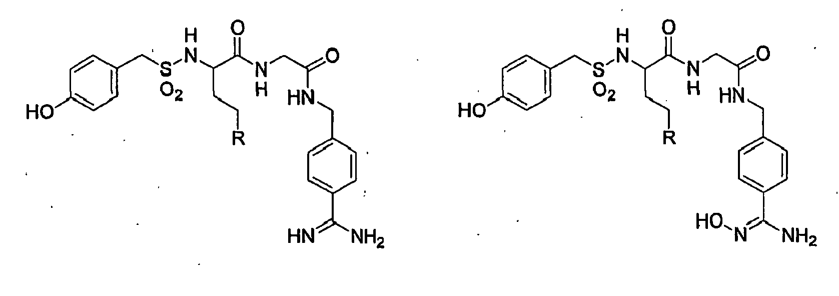 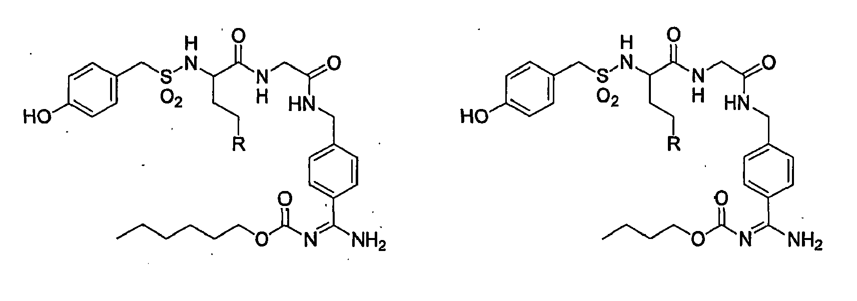 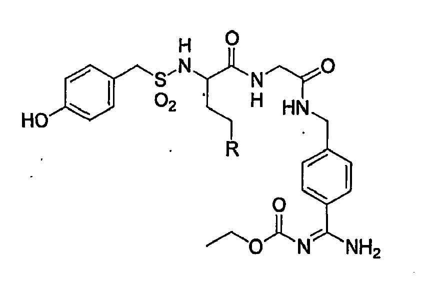 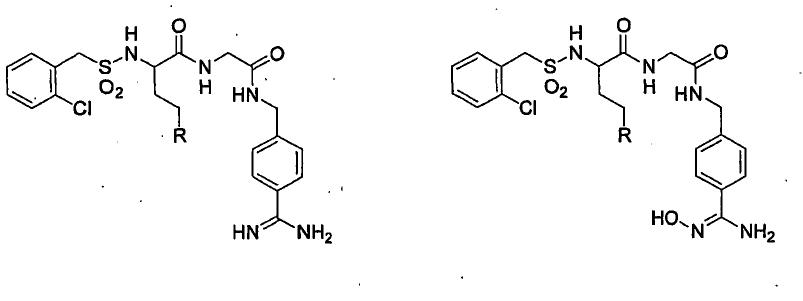 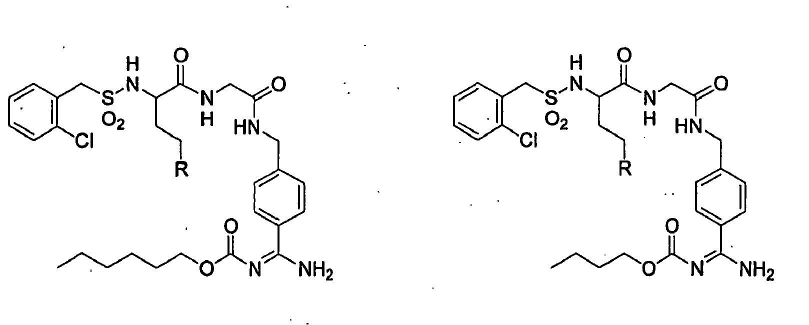 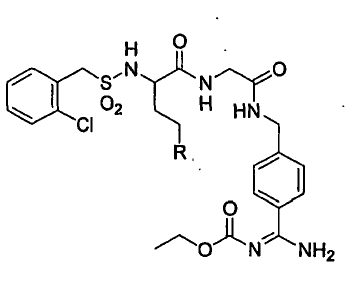 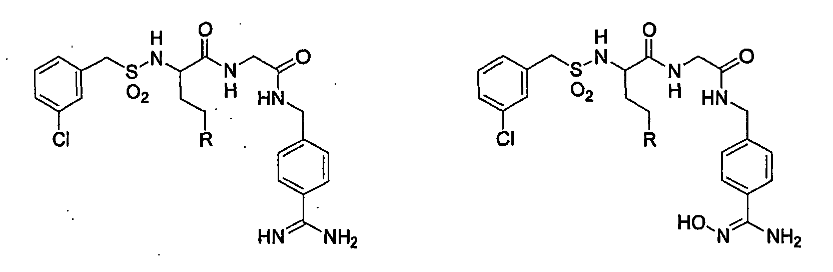 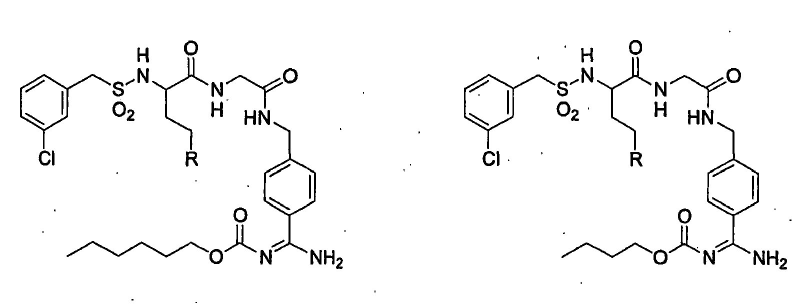 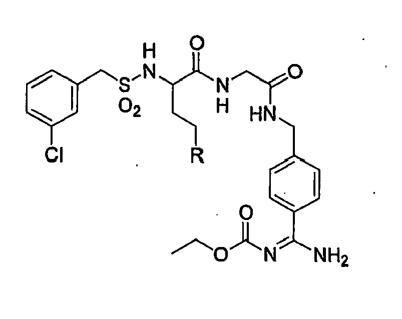 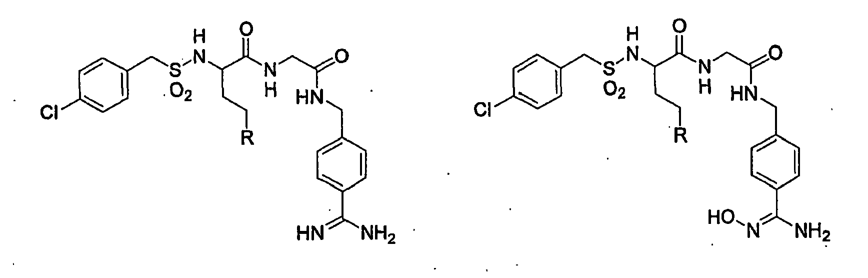 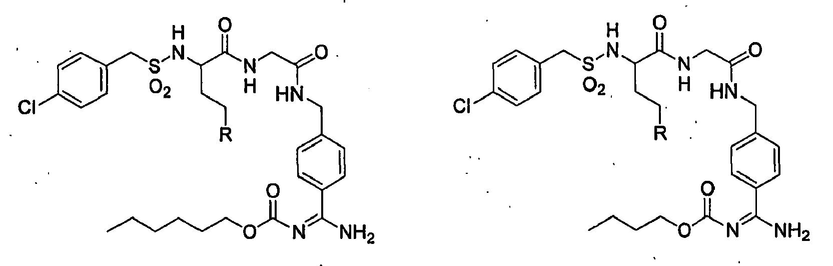 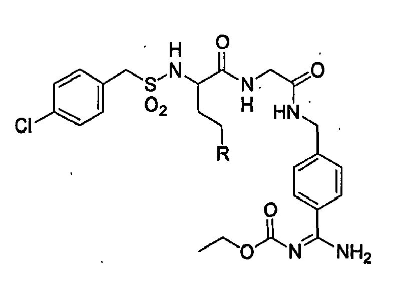 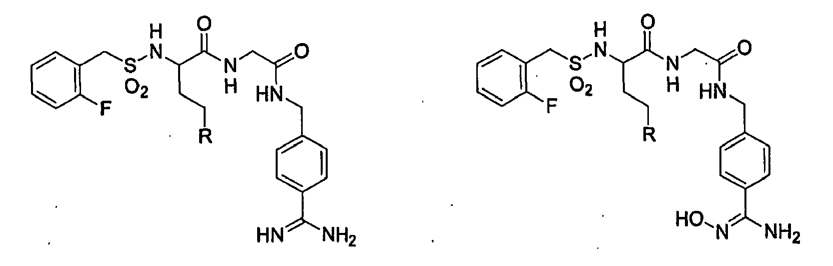 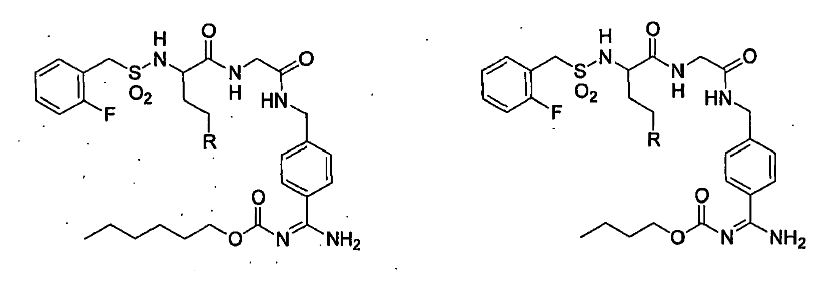 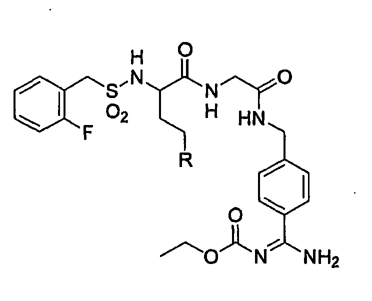 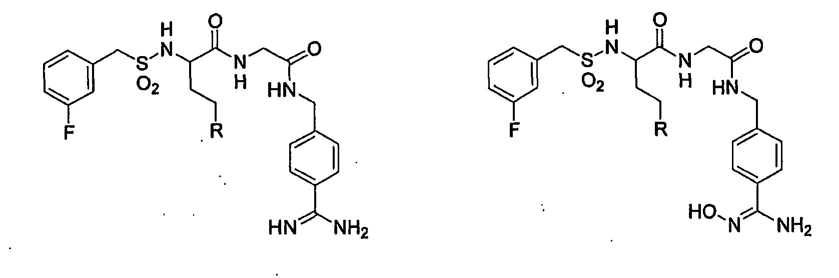 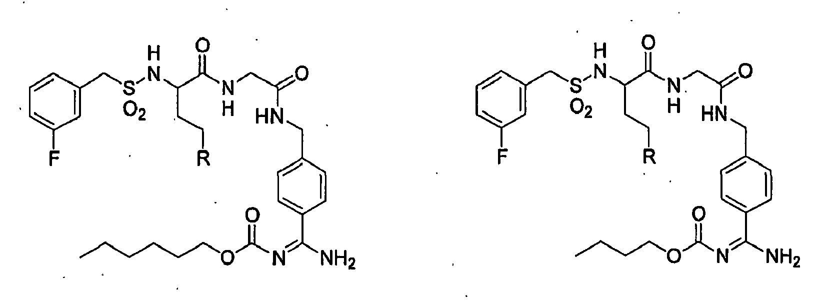 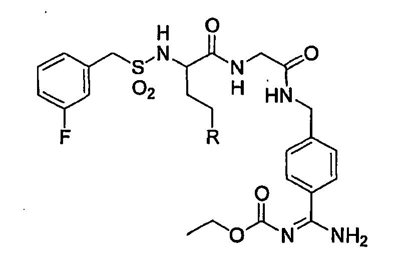 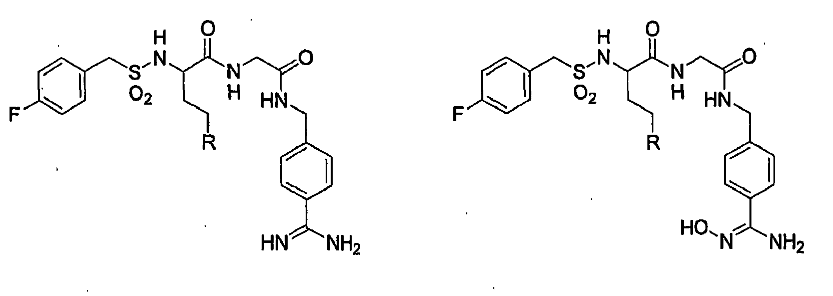 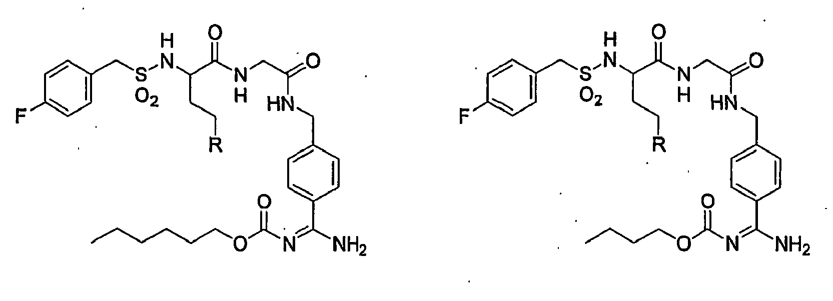 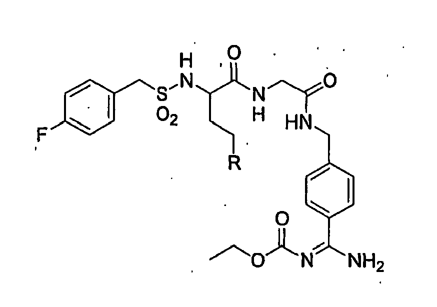 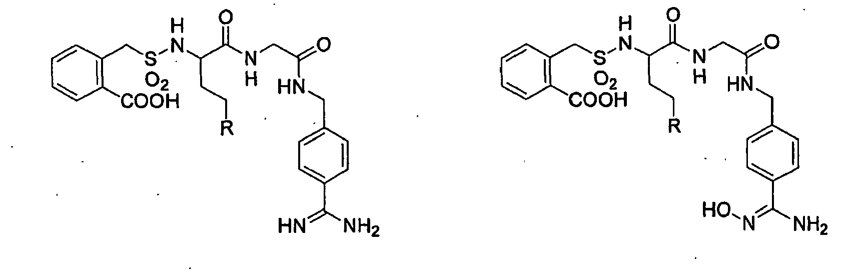 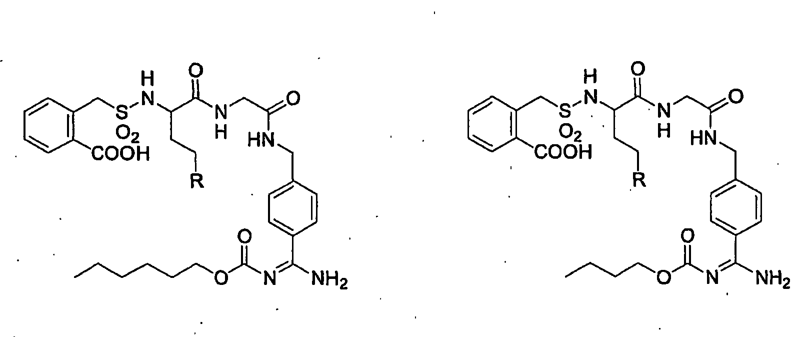 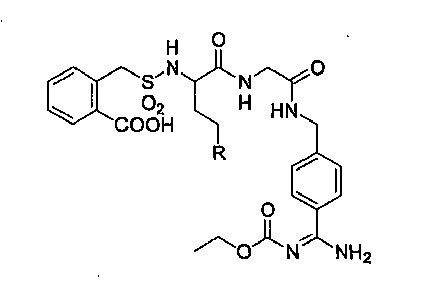 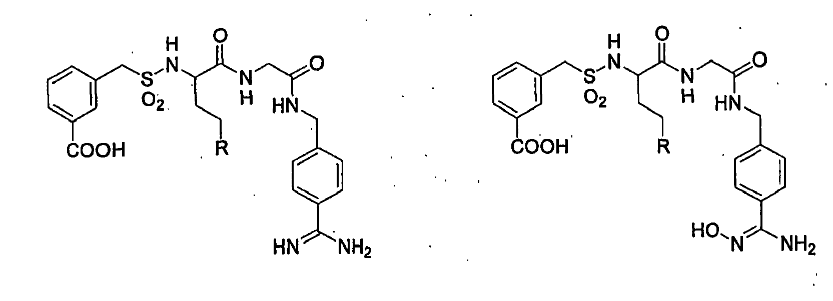 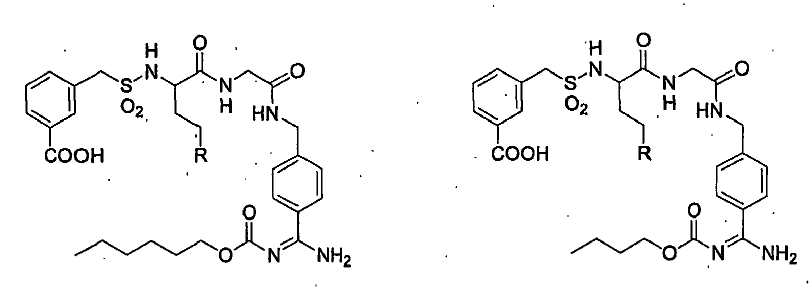 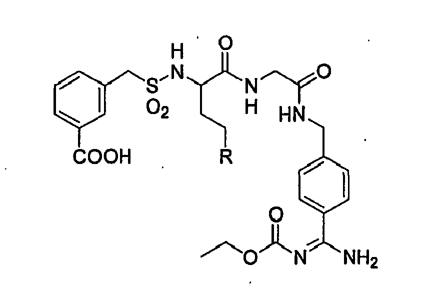 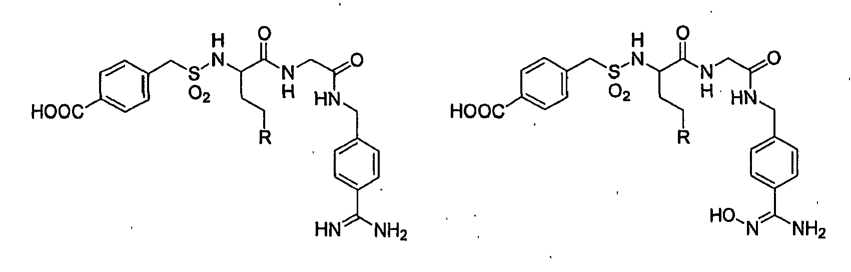 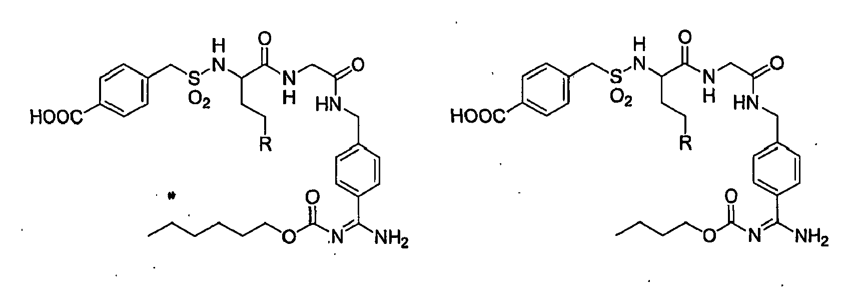 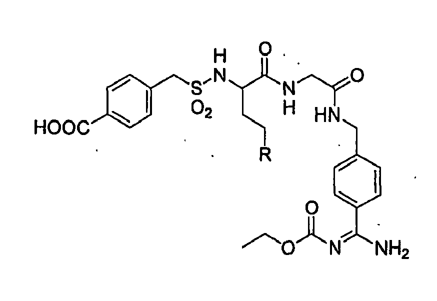 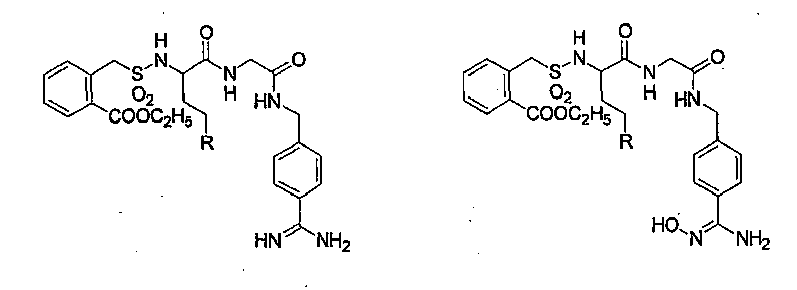 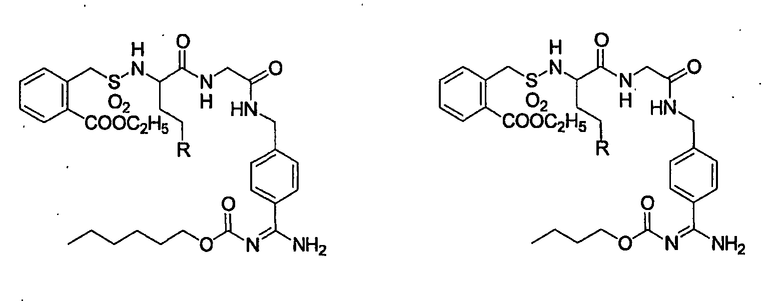 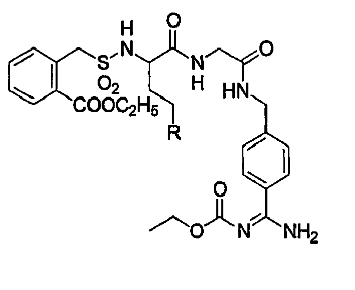 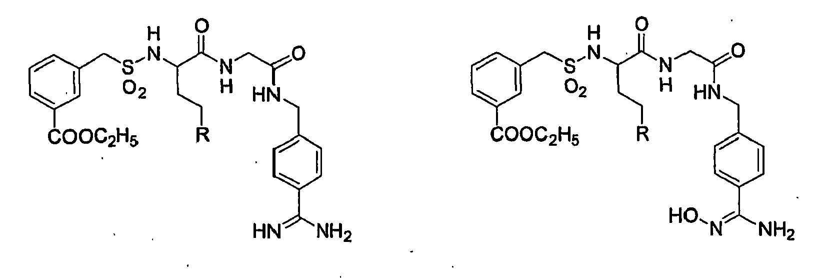 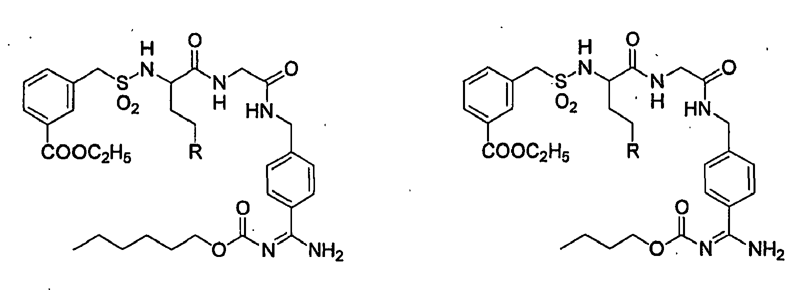 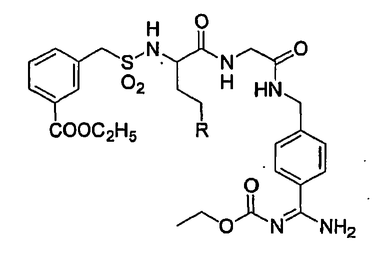 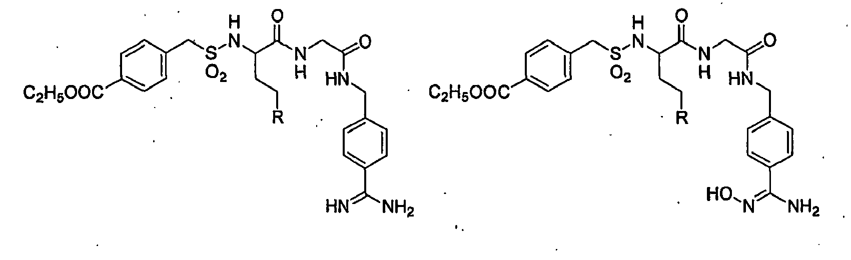 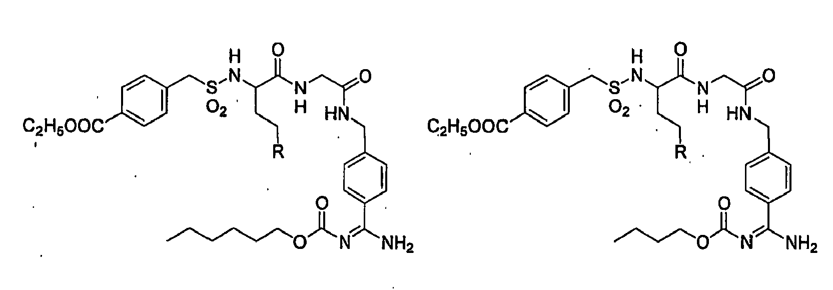 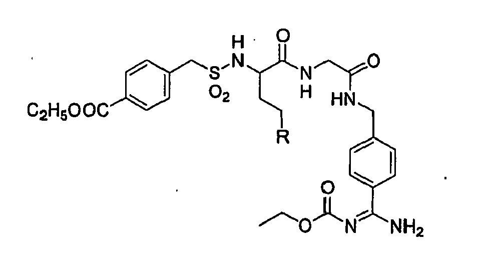 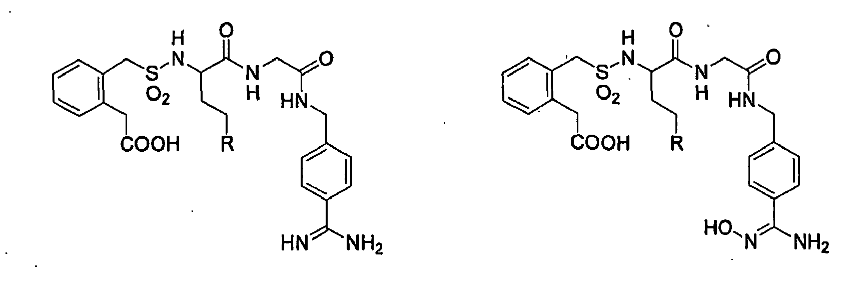 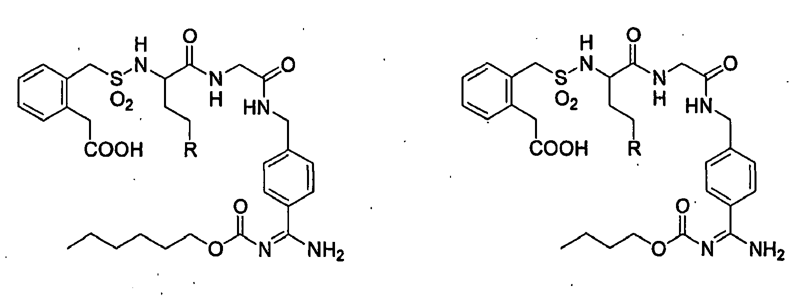 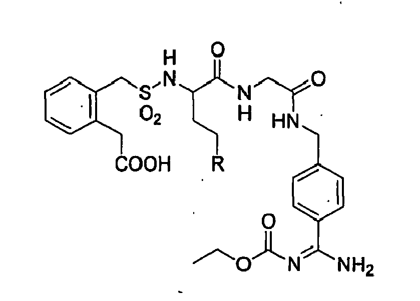 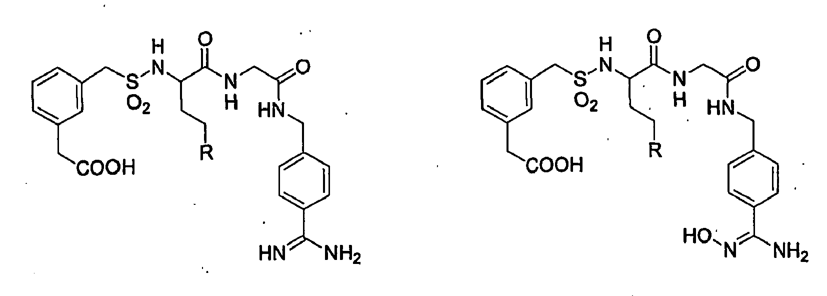 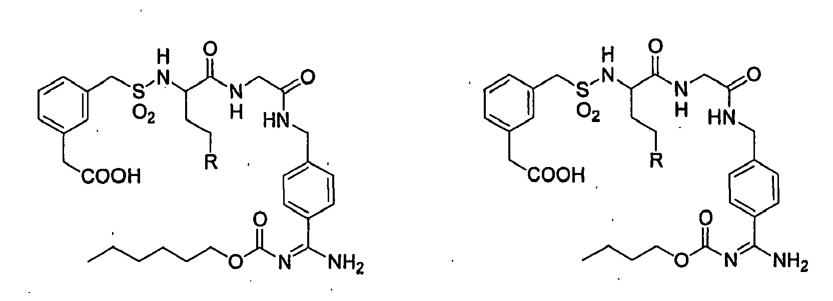 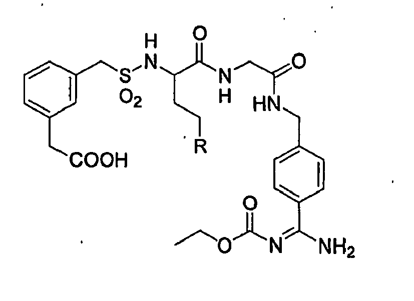 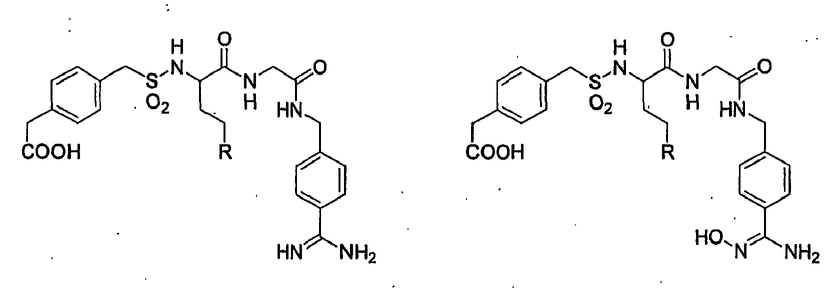 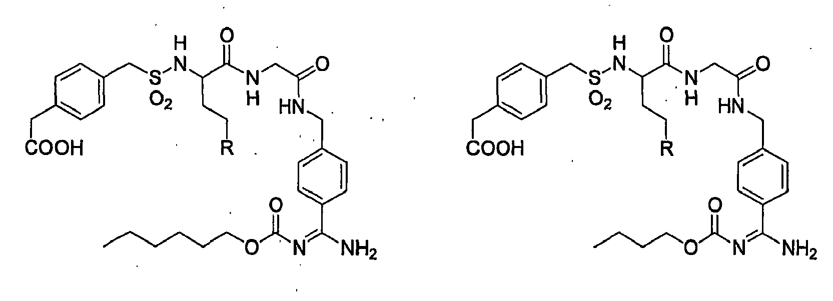 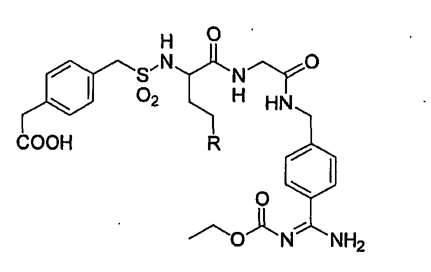 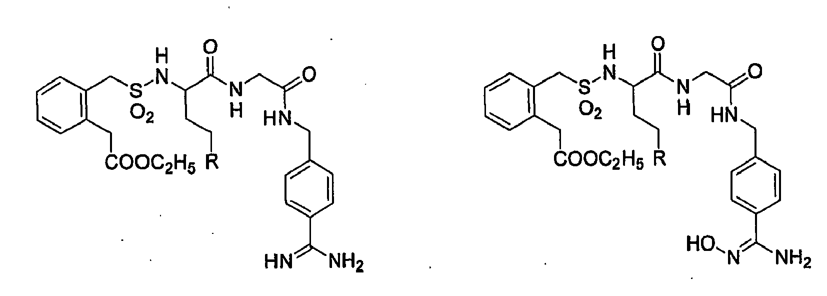 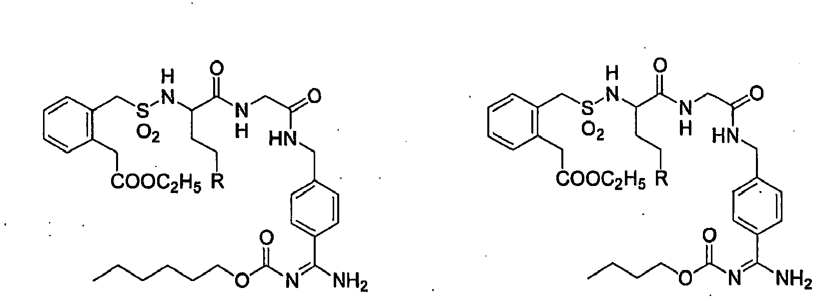 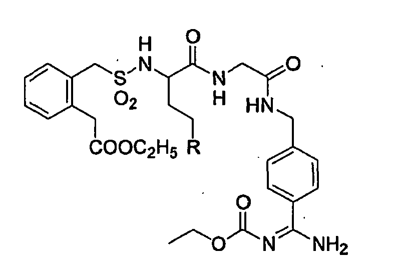 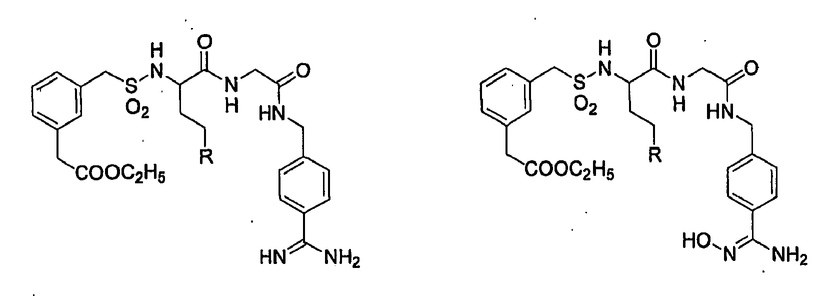 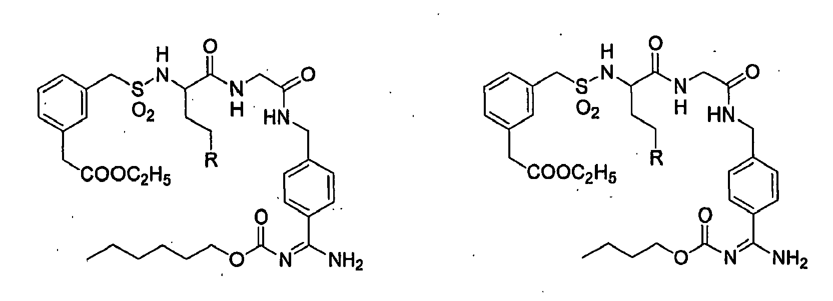 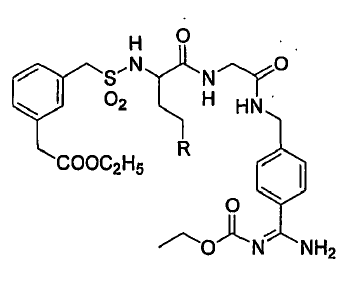 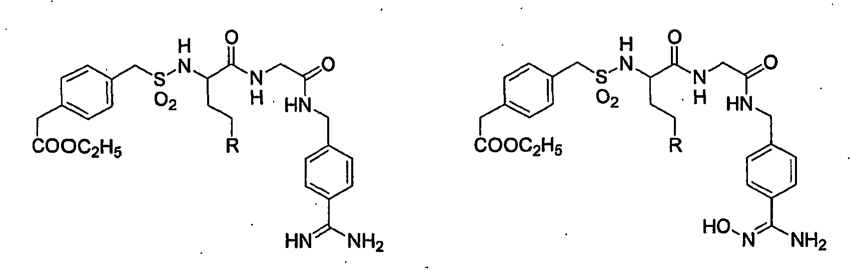 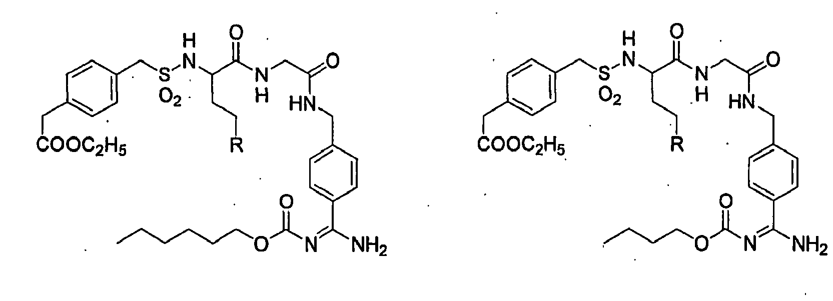 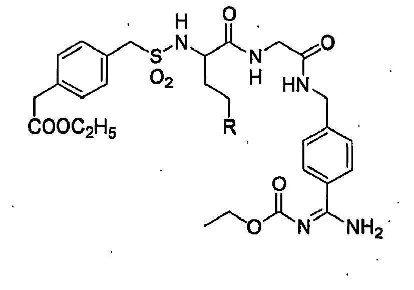 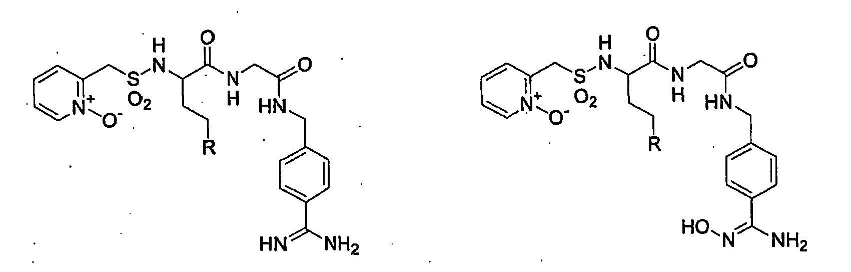 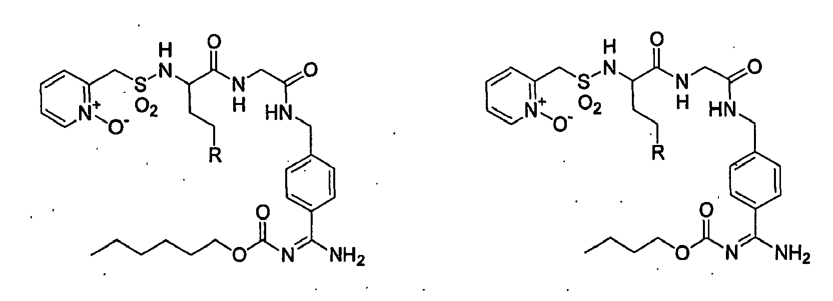 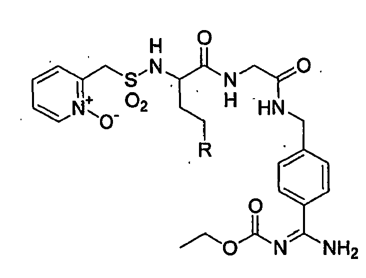 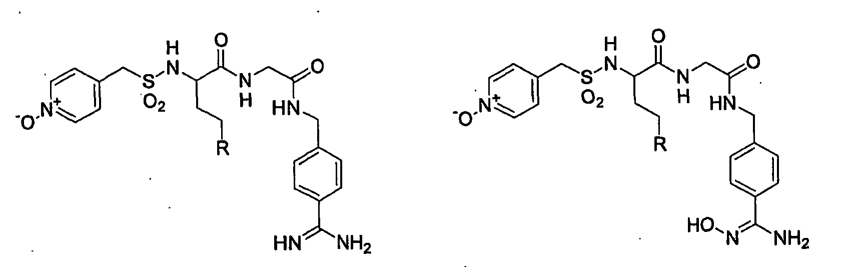 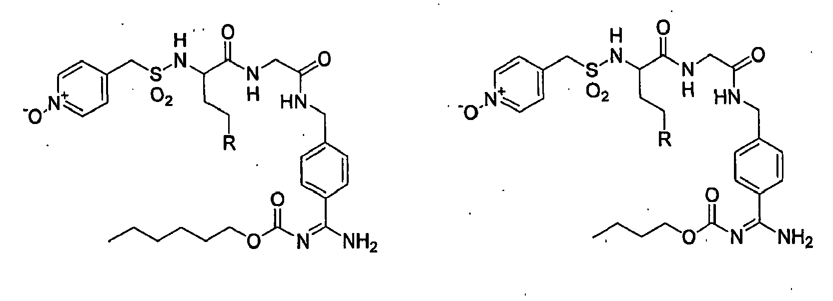 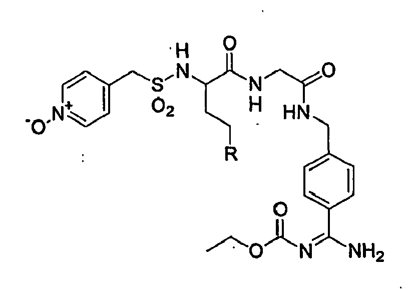 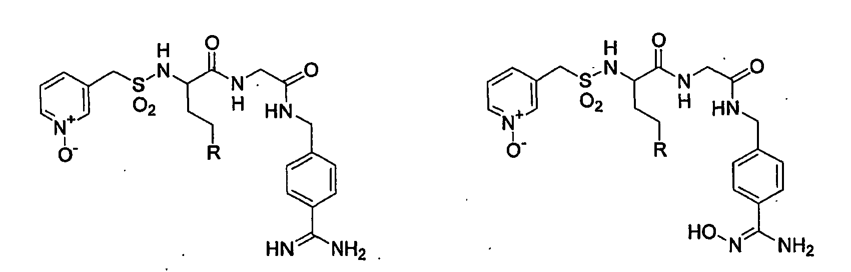 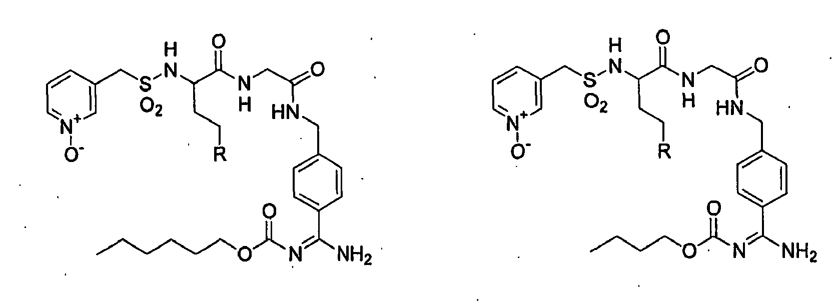 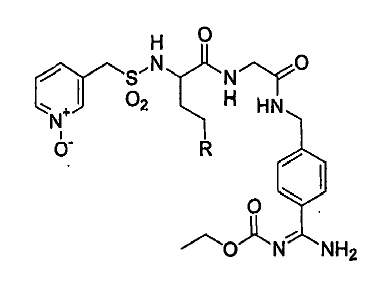 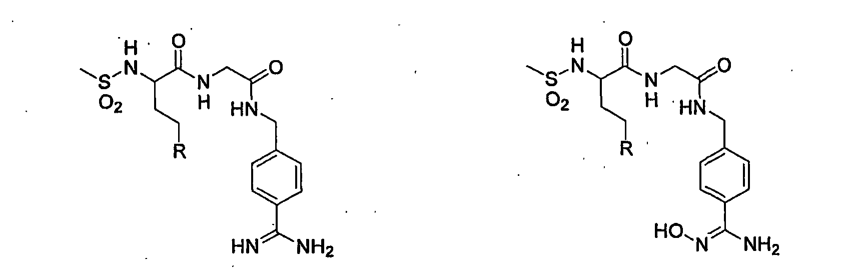 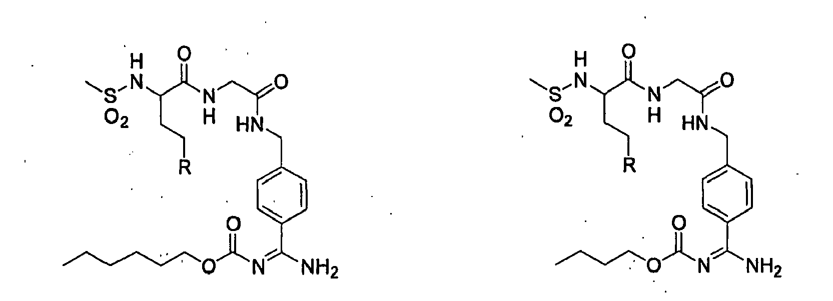 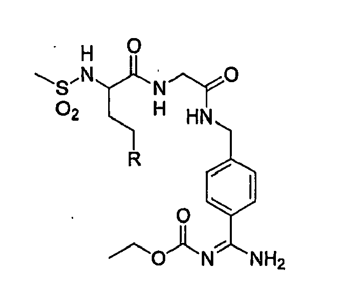 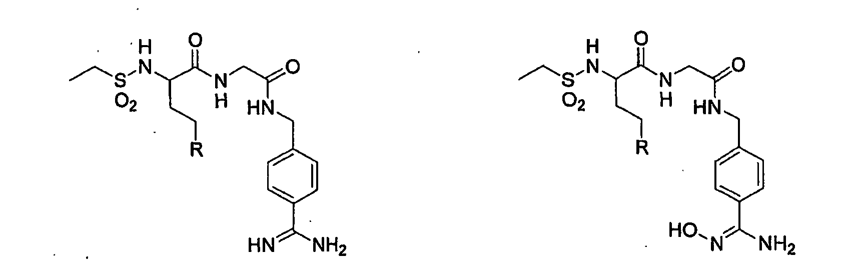 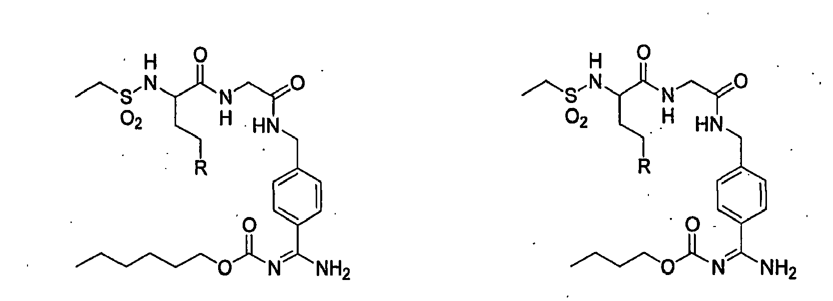 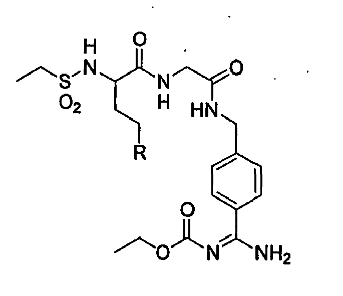 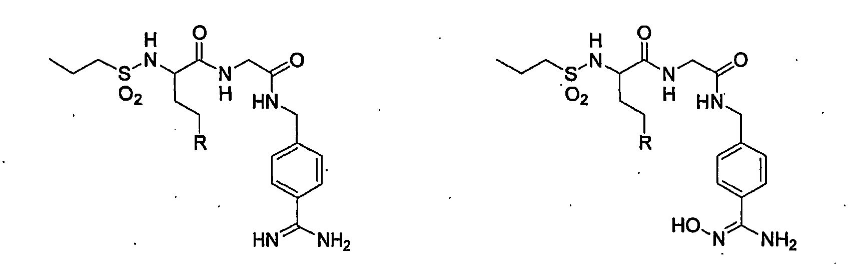 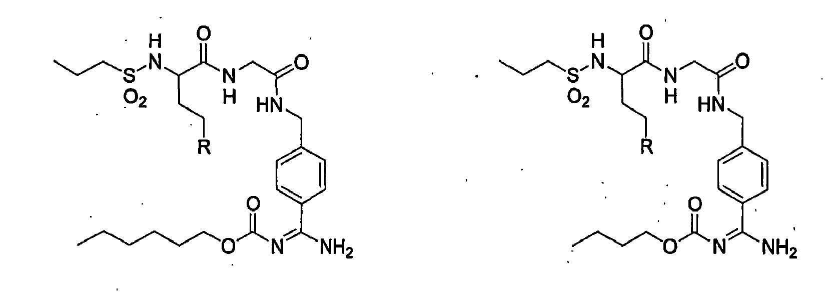 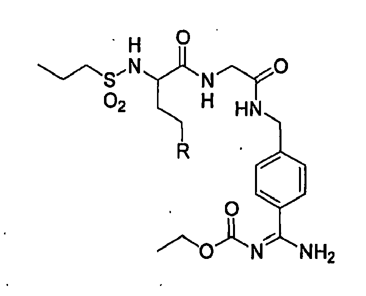 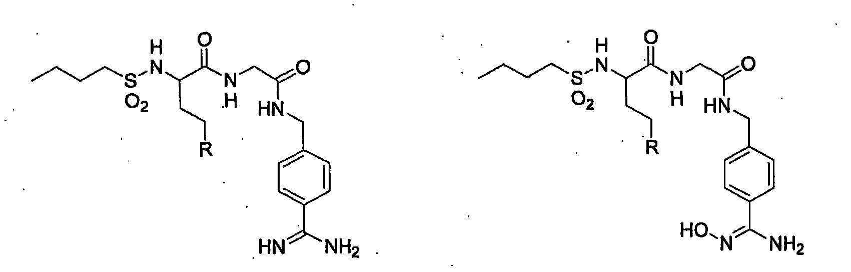 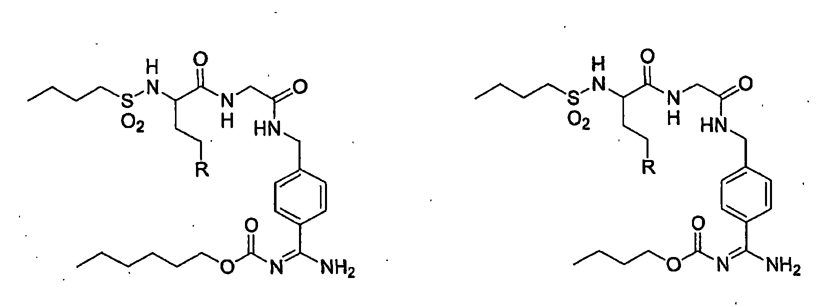 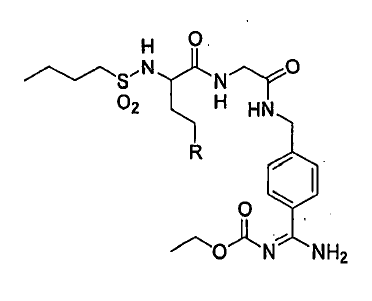 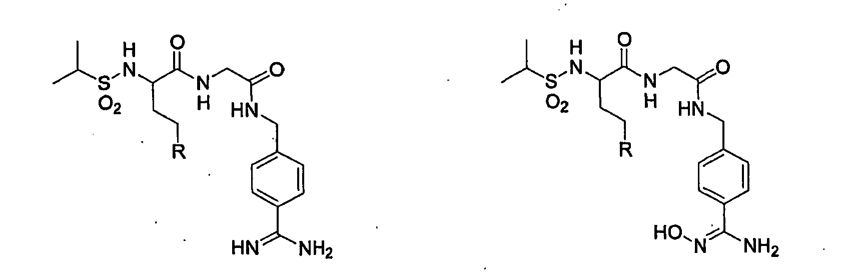 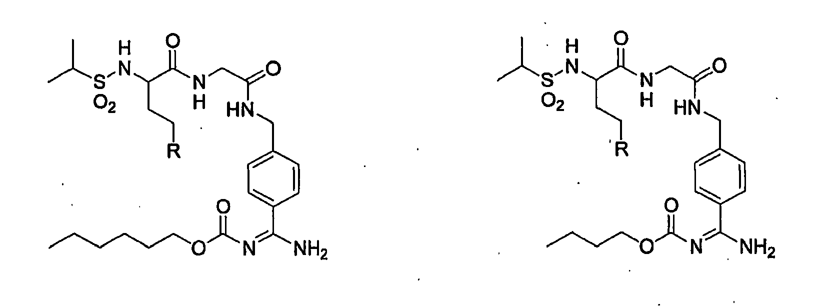 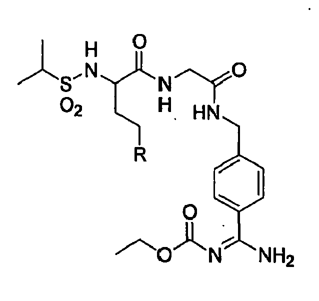 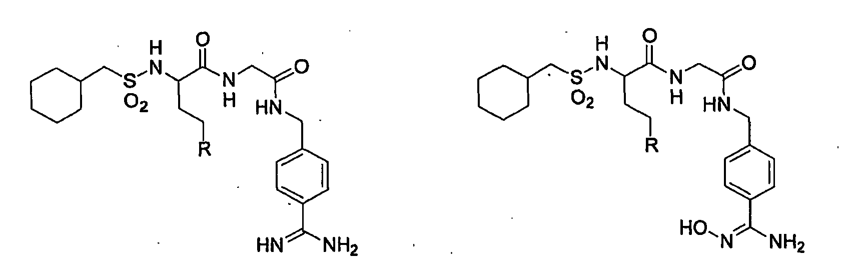 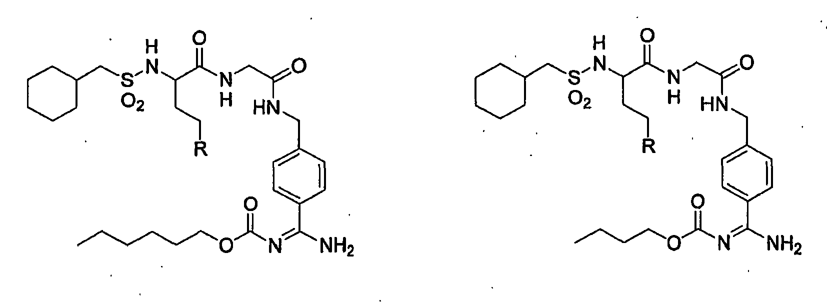 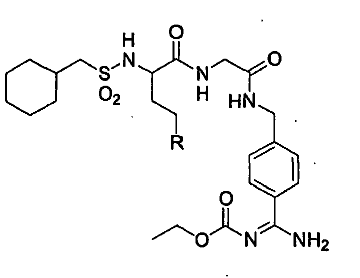 wobei der Rest R ausgewählt ist aus den Resten 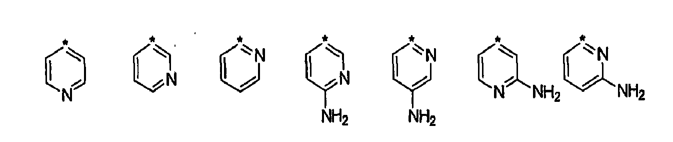 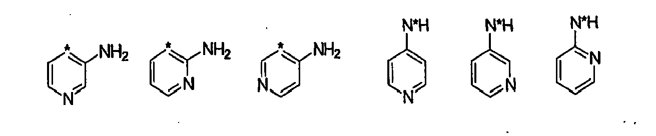 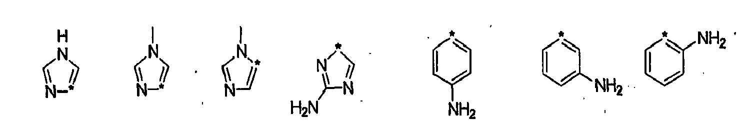 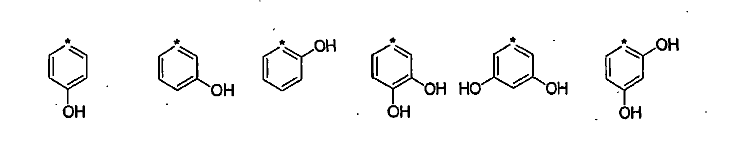 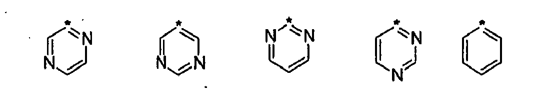 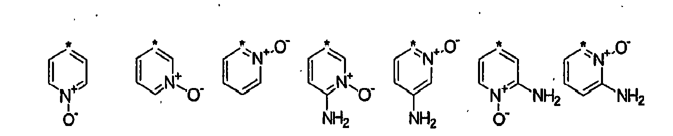 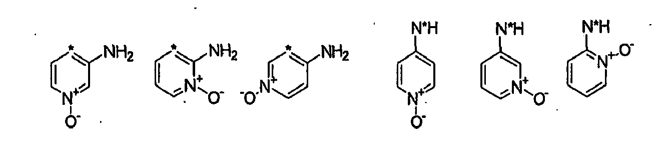 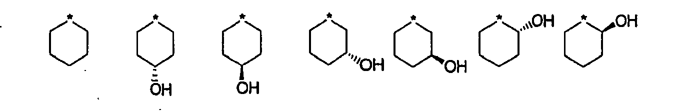 wobei die Bindung der Gruppe R zu dem Cγ-Kohlenstoffatom des Restes P₂ von dem mit Stern markierten Atom ausgeht und die Aminosäure, an der sich der Rest R befindet, sowohl in der D- als auch in der L-Konfiguration vorliegen kann, besonders bevorzugt jedoch in der D-Konfiguration.
10. Verbindung nach mindestens einem der 1 bis 8, **dadurch gekennzeichnet, dass** die Verbindung ausgewählt ist aus einer Struktur gemäß 9, wobei in den genannten Strukturen der Glycinrest mit dem Strukturelement 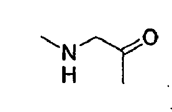 jeweils ersetzt ist durch einen Serinrest mit dem Strukturelement 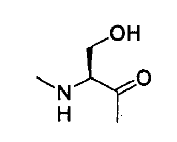 oder durch einen Glutaminsäurerest mit dem Strukturelement 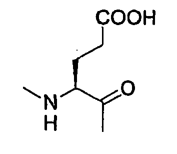 oder durch einen Glutamin-γ-ethylester mit dem Strukturelement 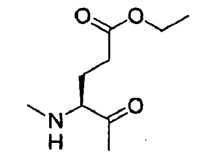
11. Verbindung nach mindestens einem der 1 bis 10, **dadurch gekennzeichnet, dass** die Verbindungen als Salze vorliegen, vorzugsweise mit Mineralsäuren oder mit geeigneten organischen Säuren, vorzugsweise mit Salzsäure, Schwefelsäure, Essigsäure, Ameisensäure, Methylsulfonsäure, Bernsteinsäure, Äpfelsäure oder Trifluoressigsäure, vor allem in Form ihrer Hydrochloride, Sulfate oder Acetate.
12. Verfahren zur Herstellung einer Verbindung gemäß mindestens einem der 1 bis 11, **dadurch gekennzeichnet, dass** sequentiell die entsprechenden Aminosäuren an ein an der Amidinogruppe geschütztes Amidinobenzylamin angekoppelt werden, wobei die N-terminale Aminosäure entweder den R₅-Rest bereits trägt oder dieser anschließend daran gebunden wird.
13. Arzneimittel enthaltend eine Verbindung gemäß mindestens einem der 1 bis 11 sowie pharmazeutisch geeignete Hilfs- und/oder Zusatzstoffe.
14. Arzneimittel nach 13, wobei das Arzneimittel in Form einer Tablette, eines Dragees, einer Kapsel, eines Pellets, Suppositoriums, einer Lösung, insbesondere einer Injektions- oder Infusionslösung, von Augen-, Nasen und Ohrentropfen, eines Safts, einer Kapsel, einer Emulsion oder Suspension, eines Globuli, Styli, Aerosols, Puders, einer Paste, Creme oder Salbe eingesetzt wird.
15. Verwendung einer Verbindung gemäß mindestens einem der 1 1 bis 11 oder eines Arzneimittels gemäß 13 oder 14 als Faktor Xa-Inhibitor zur Therapie oder Prophylaxe einer kardiovaskulären Erkrankung oder eines thromboembolischen Ereignisses, insbesondere in oraler, subkutaner, intravenöser oder transdermaler Form.
16. Verbindung nach einem der 1 bis 7, wobei Z eine Aminogruppe ist.
17. Verbindung nach einem der 1 bis 7 oder 16, wobei R₂ eine -(CH₂)ₐCONHR_{7*} oder -(CH₂)ₐCONHR_{7**} Gruppe ist, wobei a = 1, 2 oder 3 und R_{7**} ein Arylrest oder ein Heteroarylrest mit 1 -2 Heteroatomen ausgewählt aus N, S oder O ist.
18. Verbindung nach einem der 1 bis 7 oder 16, wobei R₁ eine -(CH₂)ₐCONHR_{7*} oder -(CH₂)ₐCONHR_{7**} Gruppe ist, wobei a = 1, 2 oder 3 und R_{7**} ein Arylrest oder ein Heteroarylrest mit 1 -2 Heteroatomen ausgewählt aus N, S oder O ist und der Heteroarylrest substituiert ist mit mindestens einen der folgenden: Halogen, Methyl, Ethyl, Amino,-CH₂NH₂, Nitro, -OH, -COOH, -CH₂COOH.
19. Verbindung nach einem der 1 bis 7 oder 16, wobei R₂ eine -(CH₂)ₙ-NH₂-Gruppe mit n = 1, 2, 3, 4 oder 5, vorzugsweise 1 oder 4 ist.
20. Verbindung nach einem der 1 bis 7, 16 bis 19, wobei in R₅ R₉ = H ist.
21. Verbindung nach einem der 1 bis 7, 16 bis 20, wobei R₄ eine -CH₂-SR₈ oder -CH₂CH₂-SR₈ Gruppe ist.
22. Verbindung nach einem der 1 bis 7, 16 bis 21, wobei P₁ ein 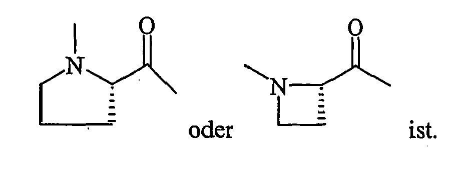
23. Verbindungen nach 10, **dadurch gekennzeichnet, dass** in den genannten Strukturen der Glycinrest mit dem Strukturelement 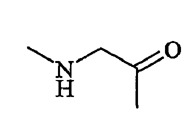 jeweils ersetzt ist durch einen Lysylrest mit dem Strukturelement 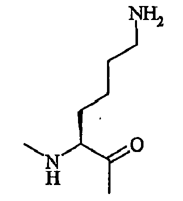 oder ersetzt ist durch einen α,β-Diaminopropionsäurerest mit dem Strukturelement 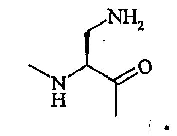
24. Verbindungen nach einem der 1 bis 7, **dadurch gekennzeichnet, dass** die Verbindung ausgewählt ist aus folgenden Strukturen: 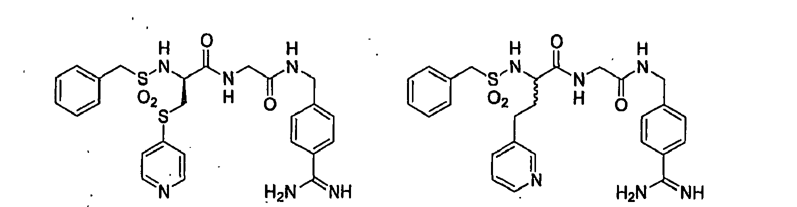 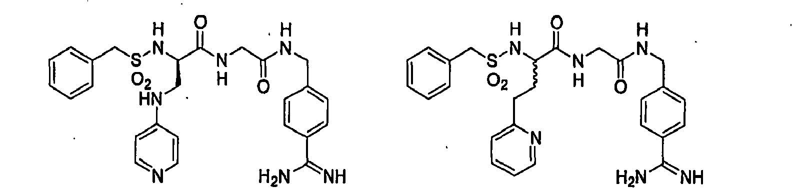 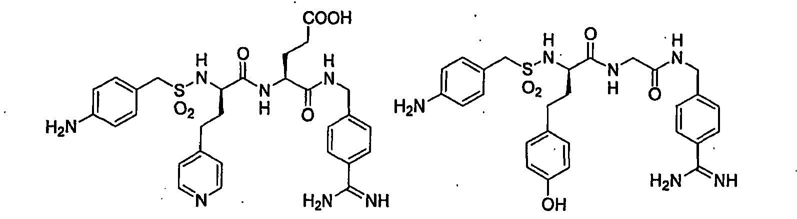 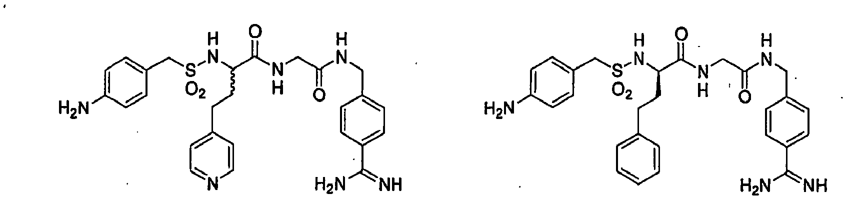 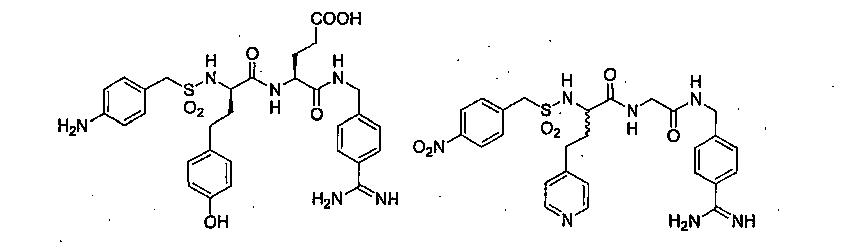 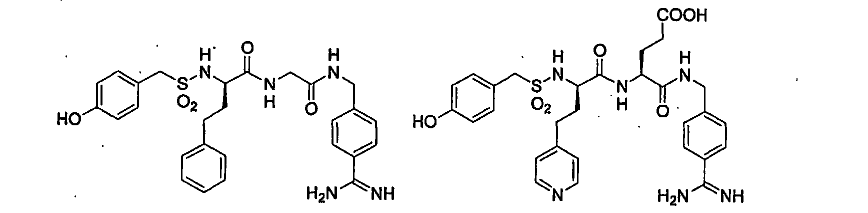 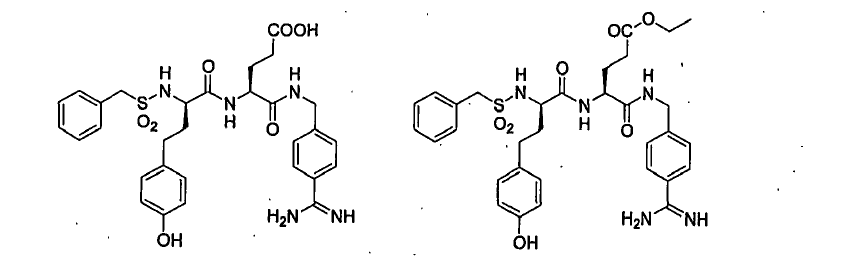 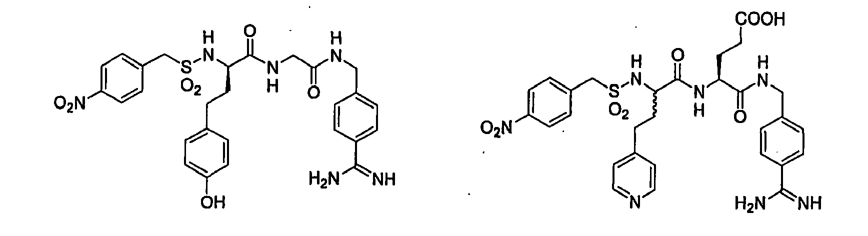 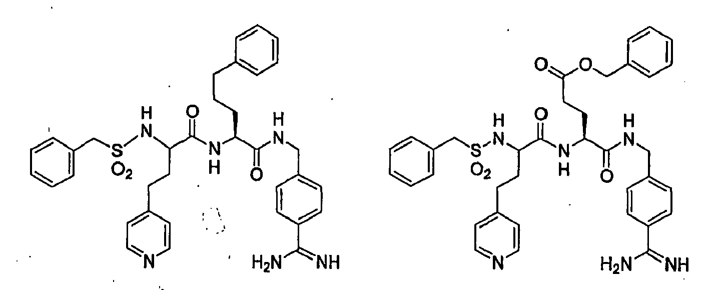 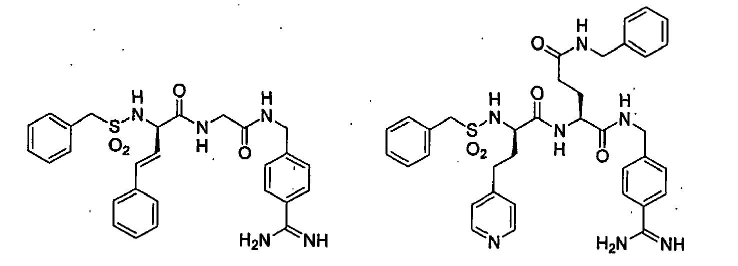 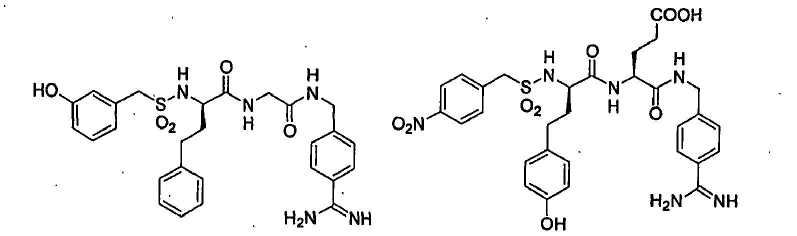 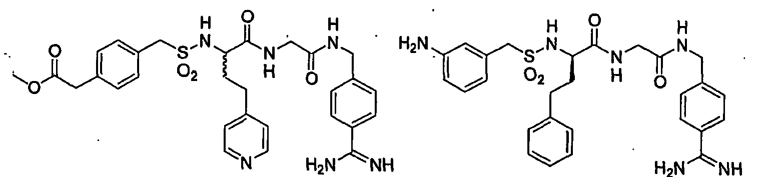 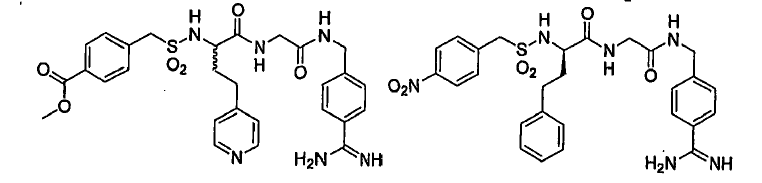 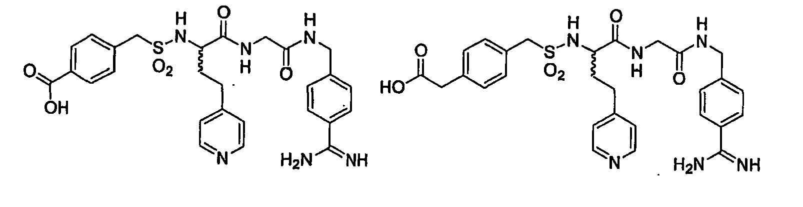 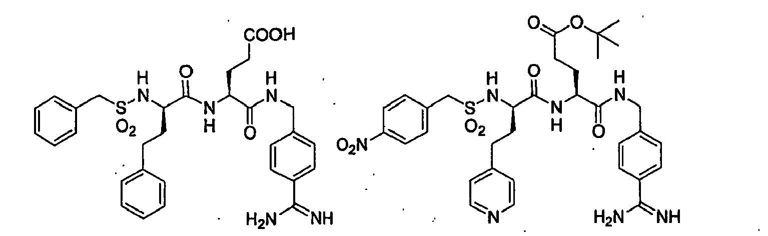 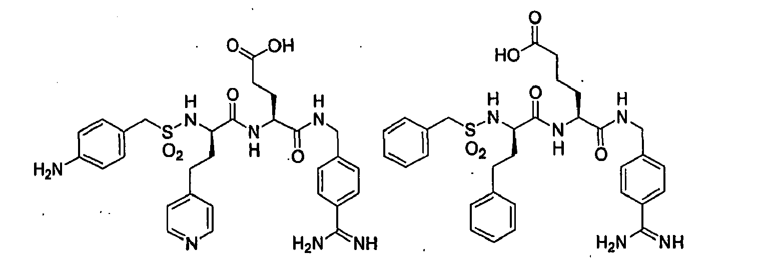 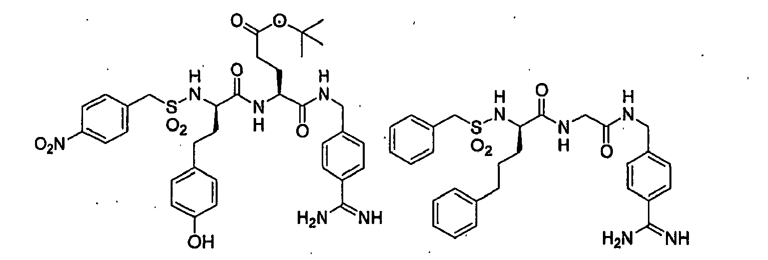 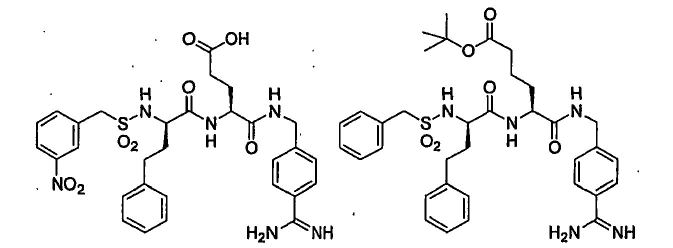 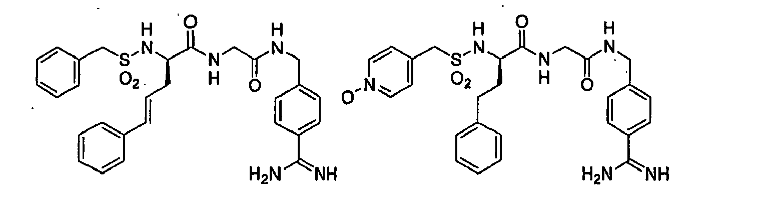 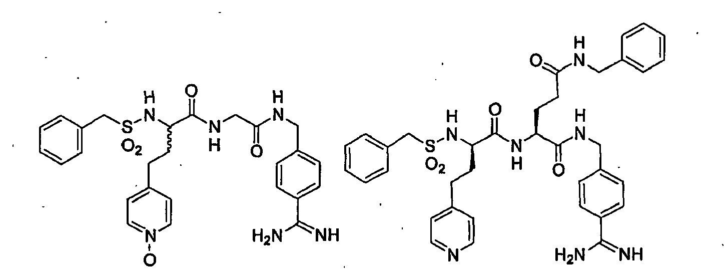 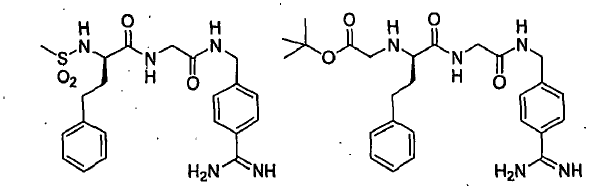 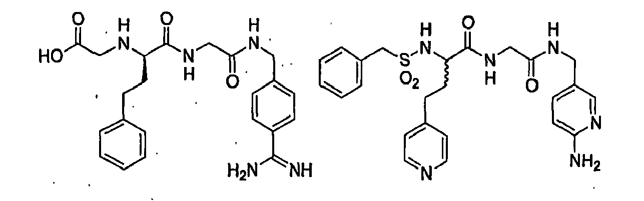
25. Verbindung nach mindestens einem der 16 bis 24, **dadurch gekennzeichnet, dass** die Verbindungen als Salze vorliegen, vorzugsweise mit Mineralsäuren oder mit geeigneten organischen Säuren, vorzugsweise mit Salzsäure, Schwefelsäure, Essigsäure, Ameisensäure, Methylsulfonsäure, Bernsteinsäure, Äpfelsäure oder Trifluoressigsäure, vor allem in Form ihrer Hydrochloride, Sulfate oder Acetate.
26. Verfahren zur Herstellung einer Verbindung gemäß mindestens einem der 16 bis 25, **dadurch gekennzeichnet, dass** sequentiell die entsprechenden Aminosäuren an ein an der Amidinogruppe geschütztes Amidinobenzylamin angekoppelt werden, wobei die N-terminale Aminosäure entweder den R₅-Rest bereits trägt oder dieser anschließend daran gebunden wird.
27. Arzneimittel enthaltend eine Verbindung gemäß mindestens einem der 16 bis 25 sowie pharmazeutisch geeignete Hilfs- und/oder Zusatzstoffe.
28. Arzneimittel nach 27, wobei das Arzneimittel in Form einer Tablette, eines Dragées, einer Kapsel, eines Pellets, Suppositoriums, einer Lösung, insbesondere einer Injektions- oder Infusionslösung, von Augen-, Nasen und Ohrentropfen, eines Safts, einer Kapsel, einer Emulsion oder Suspension, eines Globuli, Styli, Aerosols, Puders, einer Paste, Creme oder Salbe eingesetzt wird.
29. Verwendung einer Verbindung gemäß mindestens einem der 16 bis 26 oder eines Arzneimittels gemäß 27 oder 28 als Faktor Xa-Inhibitor zur Therapie oder Prophylaxe einer kardiovaskulären Erkrankung oder eines thromboembolischen Ereignisses, insbesondere in oraler, subkutaner, intravenöser oder transdermaler Form.

## Patentansprüche

1. Verbindung der allgemeinen Formel I 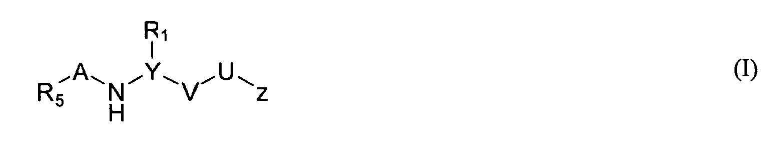 wobei
A P₂ - P₁ ist, mit 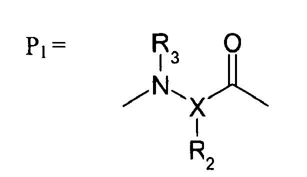 und 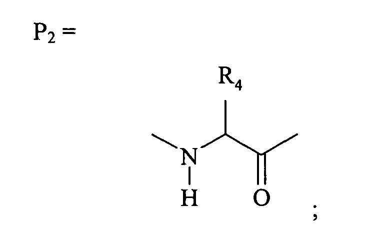
R₁ ein H oder -(CH₂)ₐCOOR₆ mit a = 0, 1, 2, 3, 4 oder 5, vorzugsweise mit a= 0, 1 oder 2, ist, wobei R₆ ein verzweigter oder unverzweigter Alkylrest mit vorzugsweise 1 bis 6 C-Atomen, insbesondere 1 bis 3 C-Atomen, vor allem Ethyl, insbesondere ist R₁ ein H;
R₂ ein H, -CH₂-OR₇ oder -CH₂-OCOOR₇ ist, wobei R₇ ein H oder ein verzweigter oder unverzweigter Alkylrest mit 1-5, insbesondere 1-3 C-Atomen, ist, oder R₂ ist ein -CH₂-CH₂-COOR_{7*}, wobei R_{7*} ein H oder ein verzweigter oder unverzweigter Alkylrest mit 1-5 C-Atomen, vorzugsweise Ethyl, ist;
R₃ ein H ist;
R₄ -(CH₂)_{f}-R₈ mit f = 0 oder 2, vorzugsweise mit f = 2, -CH₂NHR₈, -(CH₂)₂NHR₈ oder -CH=CH-R₈ ist, wobei R₈ ein einfach oder mehrfach substituierter oder unsubstituierter Cycloalkyl-, Aryl- oder Heteroarylrest ist, wobei der Cycloalkyl-, Aryl- oder Heteroarylrest vorzugsweise 5 bis 14, insbesondere 5 bis 6 C-Atome im Ring und im Falle des Heteroarylrestes vorzugsweise 1 bis 3 N als Heteroatome besitzt, oder wenn R₄ gleich -(CH₂)_{f}-R₈ mit R₈ gleich ein Hydroxycycloalkylrest mit 4 bis 14, insbesondere 6 bis 10, vor allem 6 C-Atomen ist, dann ist f = 1, und wobei P₂ in der Struktur A der allgemeinen Formel I in der D- oder L-Konfiguration, vorzugsweise in der D-Konfiguration vorliegt;
R₅ -(CH₂)ᵢ-COOR₉ mit i = 1, 2 oder 3, vorzugsweise mit i = 1, und R₉ gleich ein verzweigter oder unverzweigter Alkylrest mit 1-5 C-Atomen, vorzugsweise Ethyl, ist, oder R₅ ist -SO₂R_{9*}, -SO₂-NH-R_{9*}, wobei R_{9*} ein H, ein verzweigtes oder unverzweigtes Alkyl mit 1-10, vorzugsweise 1 bis 6, insbesondere 1 bis 4, vor allem 1 bis 2 C-Atomen, ein einfach oder mehrfach substituierter oder unsubstituierter Aryl-, Heteroaryl-, Aralkyl-, vorzugsweise Benzyl-, Heteroaralkylrest oder ein Cyclohexylalkylrest, vorzugsweise ein Cyclohexylmethylrest ist, wobei der Substituent eine -OH, -O-COOR₇, -CH₂-OCOOR₇, mit R₇ wie oben definiert, -NH₂, -NO₂, -COOR₁₀, -CH₂-COOR₁₀-Gruppe oder ein Cl-, F- oder Br-Atom sein kann und wobei R₁₀ ein H oder ein Alkylrest mit 1 bis 6, insbesondere mit 1 bis 4 C-Atomen, vor allem Ethyl, ist;
U ein Phenyl- oder Cyclohexylrest ist;
ein aromatischer oder nichtaromatischer heterocyclischer Rest mit 1-10, vorzugsweise 6 Ringatomen mit mindestens einem N, S oder O als Heteroatom, insbesondere Pyridin, Piperidin oder Pyrimidin, ist, oder
ein Thienylrest ist;
V (CH₂)ₙ mit n = 0 oder 1, vorzugsweise 0, ist;
X N oder CH, vorzugsweise CH, ist;
Y N oder CH, vorzugsweise CH, ist;
Z in 2, 3- oder 4-Position, vorzugsweise in 4-Position, vorkommt und eine Aminomethyl-, eine Guanidinofunktion oder eine Amidinogruppe 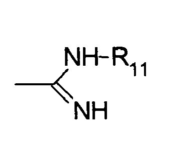 ist, wobei R₁₁ H, OH, NH₂, -COR₁₂ oder -COOR₁₂ ist, wobei R₁₂ ein verzweigter
oder unverzweigter Alkylrest mit 1 bis 8, vorzugsweise 1 bis 6 C-Atomen oder ein einfach oder mehrfach substituierter oder unsubstituierter Aryl- oder Heteroaryl-, Aralkyl- oder Heteroaralkylrest ist, wobei der Alkylrest vorzugsweise 1 bis 16, insbesondere 1 bis 8, vor allem 1 bis 4 und besonders bevorzugt 1 bis 2 C-Atome und der Aryl- oder Heteroarylrest vorzugsweise 4 bis 14, insbesondere 6 bis 10, vor allem 6 C-Atome und vorzugsweise 1 bis 3 N als Heteroatome besitzt;
oder eine Verbindung der allgemeinen Formel I in Form eines Prodrugs oder in Form ihres Salzes.

2. Verbindung nach Anspruch 1, wobei U an 1, 2 oder 3 Positionen mit einem Halogen, insbesondere Fluor oder Chlor, oder einem Methyl-, Ethyl-, Propyl-, Methoxy-, Ethoxy-, oder Propoxyrest substituiert ist.

3. Verbindung nach Anspruch 1 oder 2, wobei mindestens eine Carboxylgruppe als Ester, bevorzugt als Ethylester, geschützt vorliegt.

4. Verbindung nach mindestens einem der Ansprüche 1 bis 3 in Form eines Prodrugs, bei dem ein oder mehrere Carboxylgruppen in Form ihrer Alkylester mit einem verzweigten oder unverzweigten Alkyl mit 1-5 C-Atomen, vorzugsweise Ethyl, und/oder bei dem ein oder mehrere Hydroxylgruppen als Carbonate, bei denen der endständige Rest gleich R₇, wie oben definiert vorliegen, und/oder bei dem der Amidino- bzw. Guanidinobenzylaminrest als Hydroxyamidin bzw. Hydroxyguanidin oder als Alkyloxycarbonylderivat vorliegt.

5. Verbindung nach mindestens einem der Ansprüche 1 bis 4, **dadurch gekennzeichnet, dass**
R₁ ein H ist;
R₂ ein H, -CH₂-CH₂-COOH, -CH₂-CH₂-COOCH₂CH₃ oder -CH₂OH ist;
R₃ ein H ist;
R₄ ein -(CH₂)₂-R₈, -CH₂NHR₈, -(CH₂)₂NHR₈ oder ein -CH₂-4-Hydroxycyclohexylrest ist, wobei R₈ ein einfach oder mehrfach substituierter oder unsubstituierter Cycloalkyl-, Aryl- oder Heteroarylrest ist, wobei der Cycloalkyl-, Aryl- oder Heteroarylrest 5 oder 6 C-Atome und im Falle eines Heteroarylrestes 1 oder 2 N als Heteroatome besitzt, vorzugsweise ist R₈ ein Phenyl-, Hydroxyphenyl-, Pyridyl- oder Aminopyridylrest;
R₅ ein Methylsulfonyl, Ethylsulfonyl, n-Propylsulfonyl, iso-Propylsulfonyl, Butylsulfonyl, n-Butylsulfonyl, Benzylsulfonyl, Aminobenzylsulfonyl, Hydroxybenzylsulfonyl, Chlorobenzylsulfonyl, Fluorobenzylsulfonyl, Carboxybenzylsulfonyl, Ethyloxycarbonylbenzylsulfonyl, Carboxymethylbenzylsulfonyl, Ethyloxycarbonylmethylbenzylsulfonyl, Pyridylmethylsulfonyl, N-(oxid)-pyridylmethylsulfonyl, -CH₂-COOH oder ein -CH₂COOCH₂CH₃ Rest ist;
U ein Phenylrest ist;
V (CH₂)ₙ mit n = 0 ist;
X CH ist;
Y CH ist;
Z in 4-Position vorkommt und eine Amidinogruppe 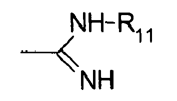 ist,
wobei R₁₁ H, OH oder -COOR₁₂ mit R₁₂ ein verzweigter oder unverzweigter Alkylrest mit 2, 4 oder 6 C-Atomen ist.

6. Verbindung nach mindestens einem der Ansprüche 1 bis 5, **dadurch gekennzeichnet, dass** R₄ ein -CH₂-CH₂-R₈ Rest ist, wobei Rg ein Aryl- oder Heteroarylrest mit 4-6 Ringatomen ist, der 1 oder 2 Heteroatome, vorzugsweise N, besitzt und der mit einer oder mehreren -NH₂ und/oder -OH Gruppen substituiert sein kann, vorzugsweise ist P₂ in der Struktur A der allgemeinen Formel I von einem Homophenylalanin, Homotyrosin, Indanylglycin oder 4-Pyridylhomoalanin abgeleitet, und die P₂-Aminosäure liegt insbesondere in der D-Konfiguration vor.

7. Verbindung nach einem der Ansprüche 1 bis 6, wobei Z eine Aminogruppe ist.

8. Verbindung nach einem der Ansprüche 1 bis 7, wobei R₂ eine -(CH₂)ₐCONHR_{7*} oder -(CH₂)ₐCONHR_{7**} Gruppe ist, wobei a = 1, 2 oder 3 und R_{7**} ein Arylrest oder ein Heteroarylrest mit 1 -2 Heteroatomen ausgewählt aus N, S oder O ist, mit der Maßgabe, dass die Verbindung nicht 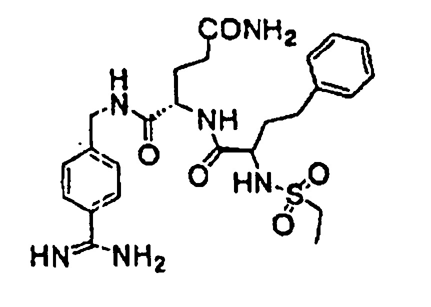 oder 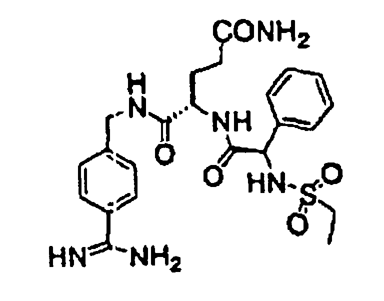 oder 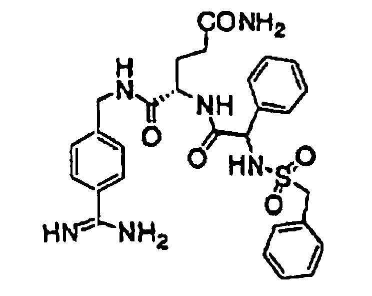 ist.

9. Verbindung nach einem der Ansprüche 1 bis 7, wobei R₁ eine -(CH₂)ₐCONHR_{7*} oder -(CH₂)ₐCONHR_{7**} Gruppe ist, wobei a = 1, 2 oder 3 und R_{7**} ein Arylrest oder ein Heteroarylrest mit 1 -2 Heteroatomen ausgewählt aus N, S oder O ist und der Heteroarylrest substituiert ist mit mindestens einen der folgenden: Halogen, Methyl, Ethyl, Amino,-CH₂NH₂, Nitro, -OH, -COOH, -CH₂COOH.

10. Verbindung nach einem der Ansprüche 1 bis 7, wobei R₂ eine -(CH₂)ₙ-NH₂-Gruppe mit n = 1, 2, 3, 4 oder 5, vorzugsweise 1 oder 4 ist.

11. Verbindung nach einem der Ansprüche 1 bis 10, wobei in R₅ R₉ = H ist.

12. Verbindung nach einem der Ansprüche 1 bis 11, wobei R₄ eine -CH₂-SR₈ oder -CH₂CH₂-SR₈ Gruppe ist.

13. Verbindung nach einem der Ansprüche 1 bis 12, wobei P₁ ein 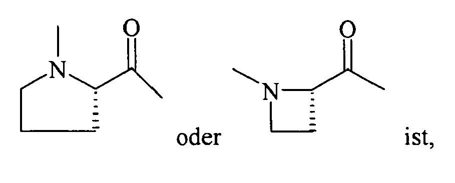 und wobei der Rest R₄ ein -CH₂-CH₂-R₈ Rest ist, wobei R₈ ein Heteroarylrest mit 4-6 Ringatomen ist, der 1 oder 2 Heteroatome, vorzugsweise N, besitzt und der mit einer oder mehreren -NH₂ und/oder -OH Gruppen substituiert sein kann, oder P₂ in der Struktur A der allgemeinen Formel I von einem Homotyrosin, Indanylglycin oder 4-Pyridylhomoalanin abgeleitet ist, oder R₈ ist ein Pyridyl-N-oxid-Rest.

14. Arzneimittel enthaltend eine Verbindung gemäß mindestens einem der Ansprüche 7 bis 13 sowie pharmazeutisch geeignete Hilfs- und/oder Zusatzstoffe, insbesondere wobei das Arzneimittel in Form einer Tablette, eines Dragees, einer Kapsel, eines Pellets, Suppositoriums, einer Lösung, insbesondere einer Injektions- oder Infusionslösung, von Augen-, Nasen und Ohrentropfen, eines Safts, einer Kapsel, einer Emulsion oder Suspension, eines Globuli, Styli, Aerosols, Puders, einer Paste, Creme oder Salbe eingesetzt wird.

15. Arzneimittel gemäß Anspruch 14 zur Verwendung als Faktor Xa-Inhibitor zur Therapie oder Prophylaxe einer kardiovaskulären Erkrankung oder eines thromboembolischen Ereignisses, insbesondere in oraler, subkutaner, intravenöser oder transdermaler Form.
